# EUROPEAN PATENT SPECIFICATION

(11) **EP 2 668 284 B1**
(45) Date of publication and mention of the grant of the patent: **15.10.2014**
(21) Application number: 12705186.0
(22) Date of filing: 27.01.2012
(51) Int. Cl.: C12Q 1/02, G01N 33/50, C12P 5/00

(54) **GEL-ENCAPSULATED MICROCOLONY SCREENING**
SCREENING VON IN EINEM GEL VERKAPSELTEN MIKROKOLONIEN
CRIBLAGE DE MICRO-COLONIES ENCAPSULÉES DANS DU GEL

(30) Priority: 28.01.2011 US 201161437214 P; 13.05.2011 US 201161486211 P
(43) Date of publication of application: 04.12.2013
(73) Proprietor: Amyris, Inc., Emeryville, CA 94608 (US)
(72) Inventor: AGRESTI, Jeremy, Emeryville, CA 94608 (US)
(74) Representative: Brasnett, Adrian Hugh
(86) International application number: PCT/US2012/023024
(87) International publication number: WO 2012/103516

(56) References cited:
- WO-A1-98/41869
- GB-A- 2 159 171
- US-A- 4 801 529
- SHAH ET AL: "Designer emulsions using microfluidics", MATERIALS TODAY, ELSEVIER SCIENCE, KIDLINGTON, GB, vol. 11, no. 4, 15 March 2008 (2008-03-15) , pages 18-27, XP022535514, ISSN: 1369-7021, DOI: 10.1016/S1369-7021(08)70053-1
- CHANG-HYOUNG CHOI ET AL: "Encapsulation of cell into monodispersed hydrogels on microfluidic device", PROCEEDINGS OF SPIE, vol. 6416, 1 January 2006 (2006-01-01), pages 641613-641613-4, XP55025704, ISSN: 0277-786X, DOI: 10.1117/12.708548
- MARTIN VINCENT J J ET AL: "Engineering a mevalonate pathway in Escherichia coli for production of terpenoids", NATURE BIOTECHNOLOGY, NATURE PUBLISHING GROUP, NEW YORK, NY, US, vol. 21, no. 7, 1 July 2003 (2003-07-01), pages 796-802, XP002420804, ISSN: 1087-0156, DOI: 10.1038/NBT833

## Description

### 1. FIELD OF THE INVENTION

The compositions and methods provided herein generally relate to the industrial use of microorganisms. In particular, provided herein are methods and compositions useful for detecting the production of industrially useful compounds (*e.g.,* isoprenoids, polyketides, and fatty acids) in a cell, for example, a microbial cell genetically modified to produce one or more such compounds.

### 2. BACKGROUND

The advent of synthetic biology has brought about the promise of microbially-produced biofuels, chemicals and biomaterials from renewable sources at production scale and quality. However, the commercial success of industrial synthetic biology will depend largely on whether the production cost of renewable products can be made to compete with, or out-compete, the production costs of their respective non-renewable counterparts. Towards this goal, strain engineering and screening efforts are ideally aimed towards the identification of desirable strains (*e.g*., strains capable of high yield (grams of compound per gram of substrate), high production (grams per liter) and/or high productivity (grams per liter per hour)) as early in the production process as possible.

Host cell microbes can be engineered to comprise biosynthetic genes or pathways, and metabolic flux through these pathways can be optimized to achieve high product titers. For example, microbes have been engineered to overexpress a portion, or all, of the mevalonate (MEV) metabolic pathway for industrial production of precursors to the anti-malarial drug artemisinin. *See, e.g.,* Martin et al., Nat. Biotechnol. 21:796-802 (2003); Ro et al., Nature 440:940943 (2006); and U.S. Patent Nos. 7,172,886 and 7,192,751. Engineering of a strain to a desired phenotype is often carried out as an iterative process involving several rounds of engineering, analysis and modeling of metabolic fluxes. During this process, several approaches to controlling metabolic flux can be tested and optimized, including but not limited to, the incorporation of heterologous promoters to fine tune expression of individual pathway components, rational design of optimized networks and pathways, and directed evolutionary strategies involving random mutagenesis.

Screening methods for strains having improved performance are ideally: (1) sensitive enough to discern incremental improvements in performance from one modified population to the next; (2) specific enough to distinguish endogenous molecules from recombinantly produced heterologous products, whether intracellularly contained or secreted into surrounding media; (3) robust enough to screen many libraries of modified strains at once; and (4) informative as to the metabolic impact of production on the host. With regard to the latter, it can be the case that the importation and expression of heterologous genes in the host will lead to metabolic imbalance and/or the accumulation of toxic metabolites. In such scenarios, it is useful to know whether production comes at the cost of reduced viability of the host. Furthermore, in view of the possibilities for widely divergent biomass from one producing population to the next, the ability to detect recombinant product specifically and without influence or input from cell biomass can provide a more accurate depiction of the yield, production, and/or productivity of a given strain. Moreover, where the industrial compound is secreted by the host cell, a further requirement is imposed on the screening method, that is, the ability to maintain a physical linkage between the amount of compound produced by the cell and the underlying genotype giving rise to this phenotype.

Various methods in the art that utilize cell encapsulation technology in combination with flow cytometry have generally aimed to identify the presence or absence of macromolecules produced by cells in a cell library. Flow cytometry has powerful analytic functions, enabling evaluation of cells or particles at an extremely rapid rate, up to 40,000 events per second, making this technology ideal for the reliable and accurate quantitative evaluation of cell populations and for selection of specific cells.

Nevertheless, given the challenge in synthetic biology of identifying subtle improvements in metabolic flux from one strain to the next, there remains a need for screening systems and methodologies that are simultaneously more sensitive, reliable, robust and efficient than current technologies. Particularly needed are methods for detecting and quantifying the production of recombinant compounds in a manner that maintains a linkage between phenotype and genotype without comprising the viability of the host cell after identification. The present invention addresses these needs and provides related advantages as well.

WO 98/41869 A1 descibes a screening method using encapsulated cells. GB 2159171 describes a method of encapsulating cells within membranes permeable to a substance of interest.

### 3. SUMMARY OF THE INVENTION

The present invention provides a method of detecting recombinantly produced water-immiscible compound in a cell, the method comprising: (a) encapsulating the cell in a hydrogel particle; (b) culturing the cell within the hydrogel particle whereby a cell population is formed within the particle; and (c) detecting the recombinantly produced water-immiscible compound within the hydrogel particle, wherein said detecting comprises contacting the hydrogel particle with a fluorescent solvatochromic dye and normalizing the amount of water-immiscible compound within the hydrogel particle to the amount of cell biomass within the hydrogel particle.

The invention aslo provides method of screening a library of cells for a cell recombinantly producing a water-immiscible compound, the method comprising: (a) encapsulating each cell of the library in a hydrogel particle; (b) culturing the cell within the hydrogel particle whereby a cell population is formed within the particle; (c) detecting recombinantly produced water-immiscible compound within each hydrogel particle, wherein said detecting comprises contacting the hydrogel particle with a fluorescent solvatochromic dye and normalizing the amount of water-immiscible compound within the hydrogel particle to the amount of cell biomass within the hydrogel particle; and (d) selecting a cell producing said recombinantly produced water-immiscible compound.

The invention also provides a method of enriching a population of cells for cells recombinantly producing a water-immiscible compound, the method comprising: (a) providing a population of hydrogel particles, wherein the population comprises hydrogel particles that encapsulate a cell or a clonal population of cells genetically modified to produce a water-immiscible compound; (b) culturing the cell or clonal population of cells within each hydrogel particle; (c) detecting a hydrogel particle comprising recombinantly produced water-immiscible compound, wherein said detecting comprises contacting the hydrogel particle with a fluorescent solvatochromic dye and normalizing the amount of water-immiscible compound within the hydrogel particle to the amount of cell biomass within the hydrogel particle; (d) recovering the cell or clonal population of cells from the hydrogel particle of step (b); (e) re-encapsulating the cell or clonal population of cells from step (c); and (f) repeating steps (a) - (d).

The invention also provides a hydrogel-encapsulated cell or clonal cell population comprising recombinantly produced water-immiscible compound and a fluorescent solvatochromic dye that enables normalization of the amount of water-immiscible compound within the hydrogel to the amount of cell biomass within the hydrogel. The invention further provides a hydrogel particle comprising a cell or clonal cell population, recombinantly produced water-immiscible compound and a fluorescent solvatochromic dye that enables normalization of the amount of water-immiscible compound within the hydrogel to the amount of cell biomass within the hydrogel.

Described herein are methods and compositions useful for detecting a recombinantly produced water-immiscible compound in a cell, for example, a microbial cell genetically modified to produce one or more water-immiscible compounds at greater yield and/or with increased persistence compared to a parent microbial cell that is not genetically modified. In particular, the methods provided herein, alternately referred to as "picoscreening," provide for high-throughput, sensitive and quantitative means for screening microbial strains that are engineered, for example, to produce industrially useful water-immiscible compounds, including but not limited to isoprenoids, polyketides, fatty acids, and derivatives thereof. The methods provided herein also provide for the identification and selection of, and enrichment for, a cell, *e.g.,* a recombinant cell, and clonal cell populations thereof, which produce and/or comprise increased levels of such recombinantly produced water-immiscible compounds.

In some embodiments, the method comprises encapsulating a cell in a hydrogel particle and detecting recombinantly produced water-immiscible compound within the hydrogel particle. In some embodiments, the cell is selected from the group consisting of a yeast cell, a bacterial cell, a mammalian cell, a fungal cell, an insect cell, and a plant cell. In some embodiments, the cell is a yeast cell. In some embodiments, the yeast is *Saccharomyces cerevisiae.*

In some embodiments, the methods of detecting comprise contacting the hydrogel particle with a fluorescent dye that directly binds to the recombinantly produced water-immiscible compound and detecting the fluorescent dye within the hydrogel particle. In some embodiments, the fluorescent dye is Nile Red. In some embodiments, the detecting comprises normalizing the amount of water-immiscible compound within the hydrogel particle to the amount of cell biomass within the hydrogel particle.
In some embodiments, the hydrogel particle is capable of retaining a water-immiscible compound, *e.g*., a water-immiscible compound produced by the cell. In some embodiments, the hydrogel comprises agarose. In some embodiments, the hydrogel particle is less than about 1 millimeter in diameter. In some embodiments, the hydrogel particle is less than about 500 micrometers in diameter. In some embodiments, the hydrogel particle is less than about 100 micrometers in diameter. In some embodiments, the hydrogel particle is less than 50 micrometers in diameter. In some embodiments, the hydrogel particle is about 50, 45, 40, 35, 30 or 25 micrometers in diameter.

In some embodiments of the methods of detecting provided herein, the encapsulating comprises contacting the cell with an aqueous hydrogel suspension under conditions sufficient to form a hydrogel particle comprising the cell. In some embodiments, the conditions comprise contacting the aqueous hydrogel suspension comprising the cell with a fluorocarbon oil. In some embodiments, the fluorocarbon oil comprises a flurosurfactant. In some embodiments, the contacting with a fluorocarbon oil comprises loading the aqueous hydrogel suspension comprising the cell onto a microfluidic device comprising the fluorocarbon oil, wherein the hydrogel suspension contacts the fluorocarbon oil at a T-junction of the microfluidic device, wherein the contacting with the fluorocarbon oil results in formation of a non-aqueous hydrogel particle comprising the cell. In some embodiments, the methods of detection further comprise the step of separating the hydrogel particle from the fluorocarbon oil.

In some embodiments, the methods of detecting further comprise culturing the cell within the hydrogel particle prior to the detecting step. In some embodiments, the culturing is for a period of 12 to 24 hours.

In some embodiments, the recombinantly produced water-immiscible compound is a terpene, C₅ isoprenoid, C₁₀ isoprenoid or C₁₅ isoprenoid. In some embodiments, the recombinantly produced water-immiscible compound is farnesene. In some embodiments, the cell is a recombinant yeast cell comprising one or more heterologous nucleotide sequences encoding one or more enzymes of the mevalonate (MEV) pathway. In some embodiments, the recombinantly produced water-immiscible compound is farnesene. In some embodiments, the cell is a recombinant yeast cell comprising one or more heterologous nucleotide sequences encoding one or more enzymes of the 1-deoxy-D-xylulose 5-diphosphate (DXP) pathway. In some embodiments, the recombinant yeast cell comprises a heterologous nucleotide sequence encoding an enzyme that can convert isopentenyl pyrophosphate (IPP) into dimethylallyl pyrophosphate (DMAPP). In some embodiments, the recombinant yeast cell further comprises a heterologous nucleotide sequence encoding an enzyme that can modify IPP or a polyprenyl to form an isoprenoid compound.

In some embodiments, the cell is a recombinant yeast cell comprising one or more heterologous nucleotide sequences encoding one or more enzymes of the polyketide biosynthesis pathway. In some embodiments, the recombinant yeast cell comprises one or more nucleic acids encoding a polyketide synthase system (PKS). In some embodiments, the PKS is a modular PKS. In some embodiments, the PKS is an aromatic PKS.

In some embodiments, the cell is a recombinant yeast cell comprising one or more heterologous nucleotide sequences encoding one or more enzymes of the fatty acid biosynthesis pathway. In some embodiments, the recombinant yeast cell comprises a nucleic acid encoding acetyl-CoA synthase. In some embodiments, the recombinant yeast cell comprises a heterologous nucleotide sequence that encodes an enzyme that can convert acetyl-CoA into malonyl-CoA.

In some embodiments, the method of detecting a recombinantly produced water-immiscible compound in a cell comprises: (a) contacting the cell with an aqueous hydrogel suspension; (b) loading the aqueous hydrogel suspension comprising the cell onto a microfluidic device comprising a fluorocarbon oil, wherein said hydrogel suspension contacts the fluorocarbon oil at a T-junction of the microfluidic device, wherein said contacting with the fluorocarbon oil results in formation of a non-aqueous hydrogel particle comprising the cell; (c) separating, *e.g*., washing the fluorocarbon oil from the hydrogel particle; (d) culturing the cell within the hydrogel particle; (e) contacting the hydrogel particle with a fluorescent dye that directly binds to the recombinantly produced water-immiscible compound; and (f) detecting the fluorescent dye within the hydrogel particle.

In another aspect, provided herein is a method of screening a library of cells for a cell recombinantly producing a water-immiscible compound, comprising encapsulating each cell of the library in a hydrogel particle; detecting the recombinantly produced water-immiscible compound within each hydrogel particle, and selecting a cell producing said recombinantly produced water-immiscible compound.

In another aspect, provided herein is a method of enriching a population of cells for cells recombinantly producing a water-immiscible compound, the method comprising: (a) providing a population of hydrogel particles, wherein the population comprises hydrogel particles that encapsulate a cell or a clonal population of cells genetically modified to produce a water-immiscible compound; (b) detecting a hydrogel particle comprising recombinantly produced water-immiscible compound; (c) recovering the cell or clonal population of cells from the hydrogel particle of step (b); (d) re-encapsulating the cell or clonal population of cells from step (c); and (e) repeating steps (a) - (c).

In another aspect, provided herein is a method of encapsulating a cell within a hydrogel particle, the method comprising: (a) contacting the cell with an aqueous hydrogel suspension; and (b) loading the aqueous hydrogel suspension comprising the cell onto a microfluidic device comprising a fluorocarbon oil, wherein said hydrogel suspension contacts the fluorocarbon oil at a T-junction of the microfluidic device, wherein said contacting with the fluorocarbon oil results in formation of a non-aqueous hydrogel particle comprising the cell.

In another aspect, provided herein is a hydrogel-encapsulated cell or clonal cell population comprising recombinantly produced water-immiscible compound. In some embodiments, the cell is selected from the group consisting of a fungal cell, *e.g.,* a yeast cell, a bacterial cell, a mammalian cell, an insect cell, and a plant cell. In some embodiments, the cell is a yeast cell. In some embodiments, the yeast is *Saccharomyces cerevisiae.*

In another aspect, provided herein is a hydrogel particle comprising a cell or clonal cell population, and further comprising recombinantly produced water-immiscible compound. In some embodiments, the cell is selected from the group consisting of a fungal cell, *e.g.,* a yeast cell, a bacterial cell, a mammalian cell, a fungal cell, an insect cell, and a plant cell. In some embodiments, the cell is a yeast cell. In some embodiments, the yeast is *Saccharomyces cerevisiae.* In some embodiments, the hydrogel comprises agarose. In some embodiments, the hydrogel particle is less than about 1 millimeter in diameter. In some embodiments, the hydrogel particle is less than about 500 micrometers in diameter. In some embodiments, the hydrogel particle is less than about 100 micrometers in diameter. In some embodiments, the hydrogel particle is less than 50 micrometers in diameter. In some embodiments, the hydrogel particle is about 50, 45, 40, 35, 30 or 25 micrometers in diameter.

### 5. BRIEF DESCRIPTION OF THE FIGURES

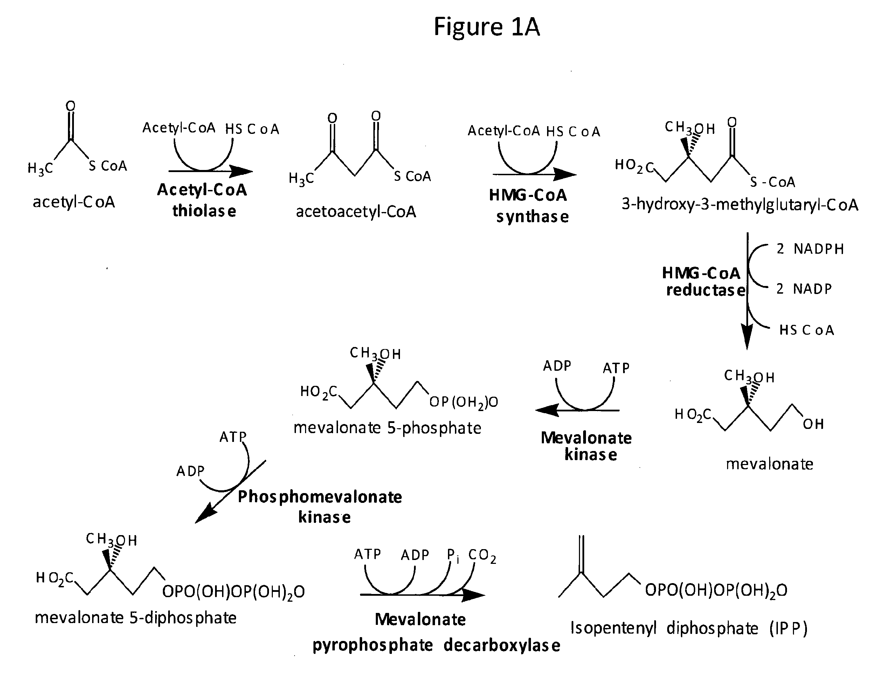

**FIG. 1A** provides a schematic representation of the mevalonate ("MEV") pathway for the production of isopentenyl diphosphate ("IPP").

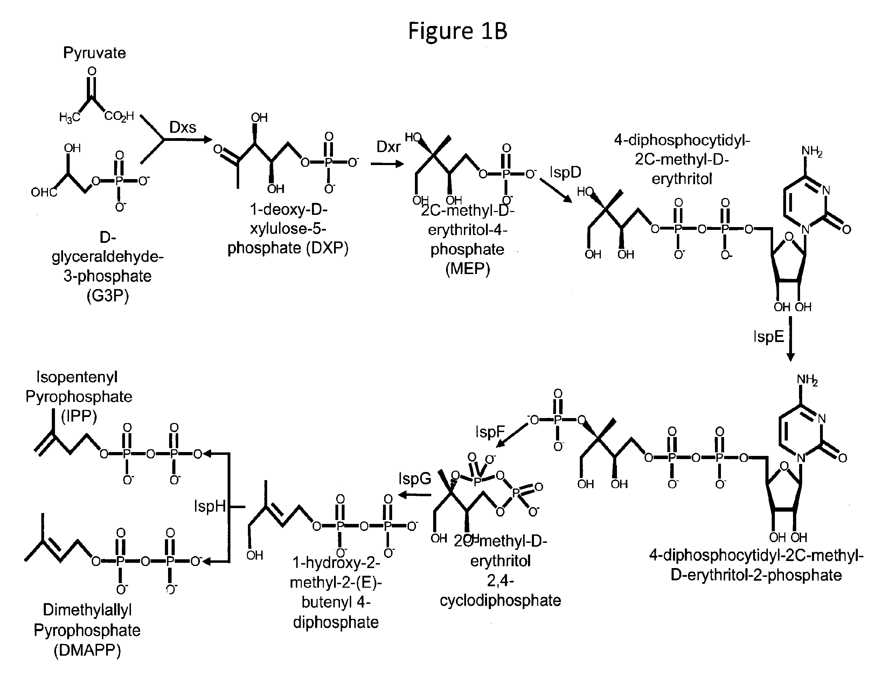

**FIG. 1B** provides a schematic representation of the 1-deoxy-D-xylulose 5-diphosphate ("DXP") pathway for the production of isopentenyl pyrophosphate ("IPP") and dimethylallyl pyrophosphate ("DMAPP"). Dxs is 1-deoxy-D-xylulose-5-phosphate synthase; Dxr is 1-deoxy-D-xylulose-5-phosphate reductoisomerase (also known as IspC); IspD is 4-diphosphocytidyl-2C-methyl-D-erythritol synthase; IspE is 4-diphosphocytidyl-2C-methyl-D-erythritol synthase; IspF is 2C-methyl-D-erythritol 2,4-cyclodiphosphate synthase; IspG is 1-hydroxy-2-methyl-2-(E)-butenyl 4-diphosphate synthase (IspG); and ispH is isopentenyl/dimethylallyl diphosphate synthase.

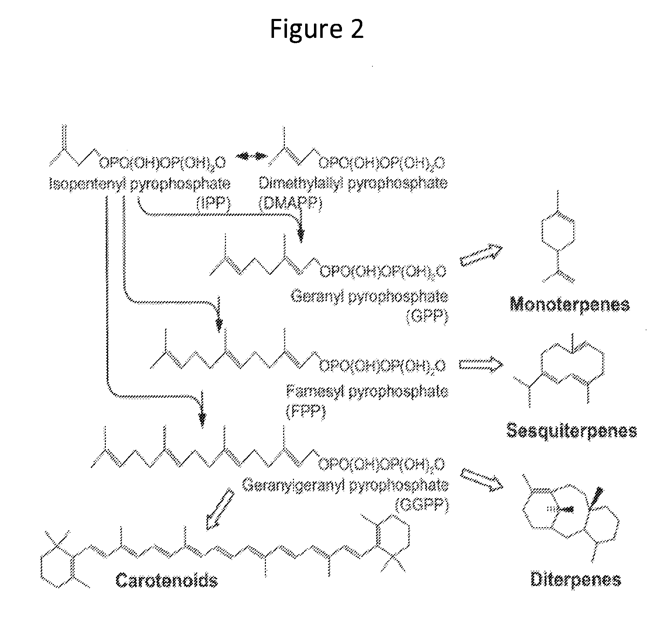

**FIG. 2** provides a schematic representation of the conversion of IPP and dimethylallyl pyrophosphate ("DMAPP") to geranyl pyrophosphate ("GPP"), farnesyl pyrophosphate ("FPP"), and geranylgeranyl pyrophosphate ("GGPP").

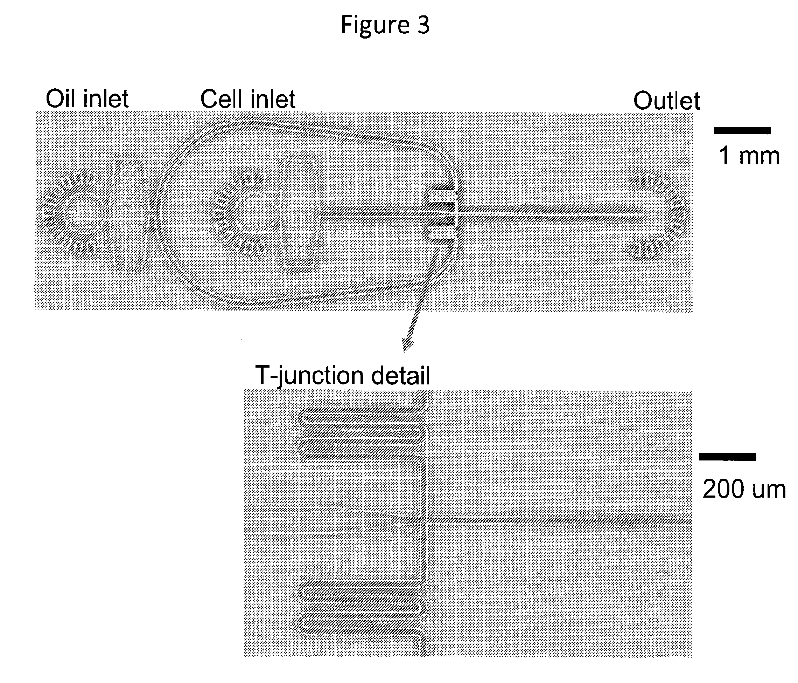

**FIG. 3** provides an exemplary microfluidic device useful for forming hydrogel particles as described herein. The device is fabricated in a planar substrate and comprises a cell inlet which is fluidly connected to a cell solution flow channel, which is fluidly connected to a particle flow channel, followed by a particle collection outlet at its terminus. The device further comprises an oil inlet which is fluidly connected to an oil flow channel, which is transversely positioned to the cell solution flow channel and intersects the cell solution flow channel at a T-junction.

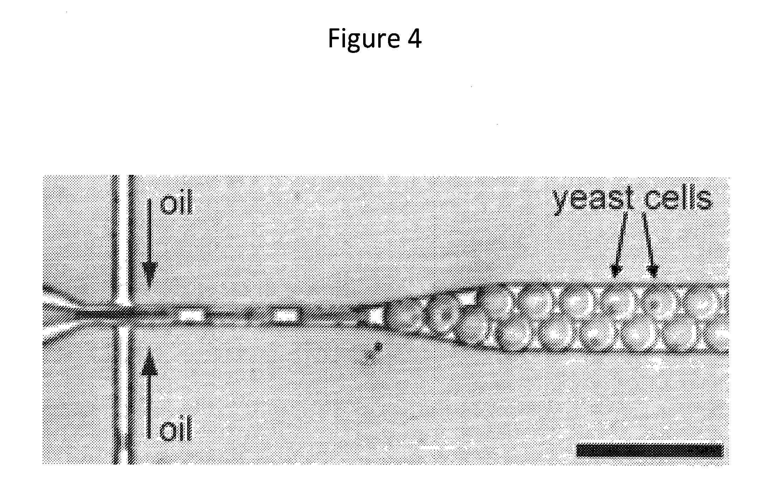

**FIG. 4** provides a depiction of an exemplary process for forming a hydrogel encapsulated cell. Cells enter from the left in the aqueous stream, which is focused through the nozzle by the two oil streams to break off the particles. At the exit of the device *(i.e.,* the particle collection outlet), the particles enter a length of tubing and flow into a container.

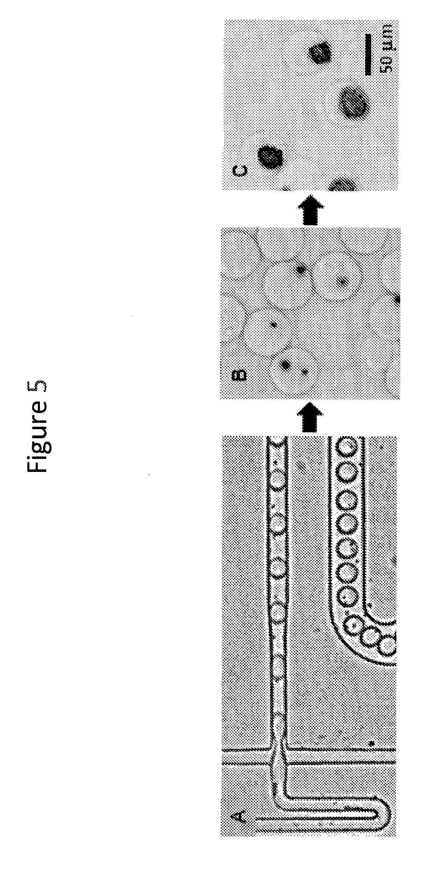

**FIG. 5** provides an exemplary embodiment of a picoscreen process. (A) A suspension of yeast cells in molten agarose is loaded into a microfluidic device. The agarose suspension meets an oil/surfactant mixture at the T-junction. The flowing oil shears off uniformly-sized agarose drops suspended in oil. (B) After collecting the drops, the oil is washed away from the particles and the particles are re-suspended in growth medium. (C) After 24h of growth, microcolonies of between 40 and 80 cells form. The particles are then stained with a fluorescent dye which can then be screened using a fluorescence-based assay.

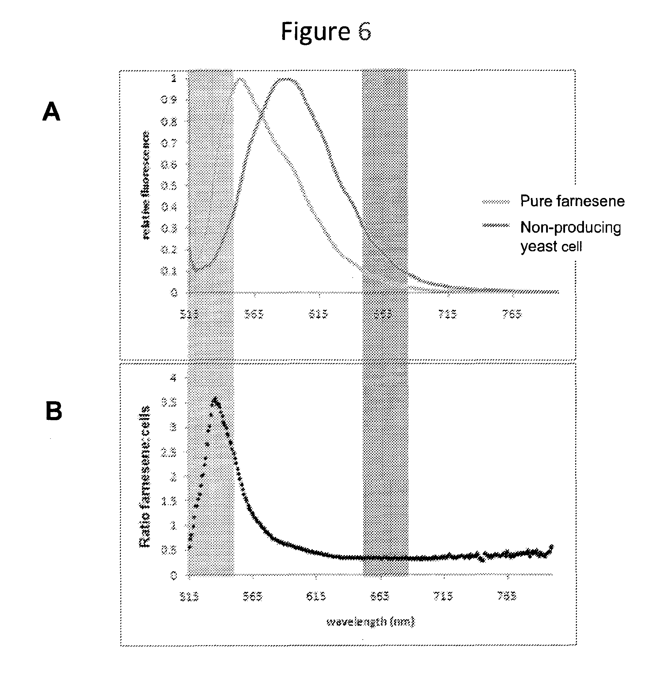

**FIG. 6** provides the emission spectra of Nile Red. (A) The emission spectra of Nile Red shifts from a max of ∼590 nm in the largely phospholipid environment of non-producing yeast (right peak), to a max of ~ 550 nm in pure farnesene (left peak). (B) The ratio of Nile Red in farnesene to a non-producing cell as a function of wavelength. The green (left) and red (right) vertical bars represent the emission filters used for one embodiment of a picoscreen FACS assay with spectra chosen to maximize the green/red fluorescence ratio in the ratiometric analysis.

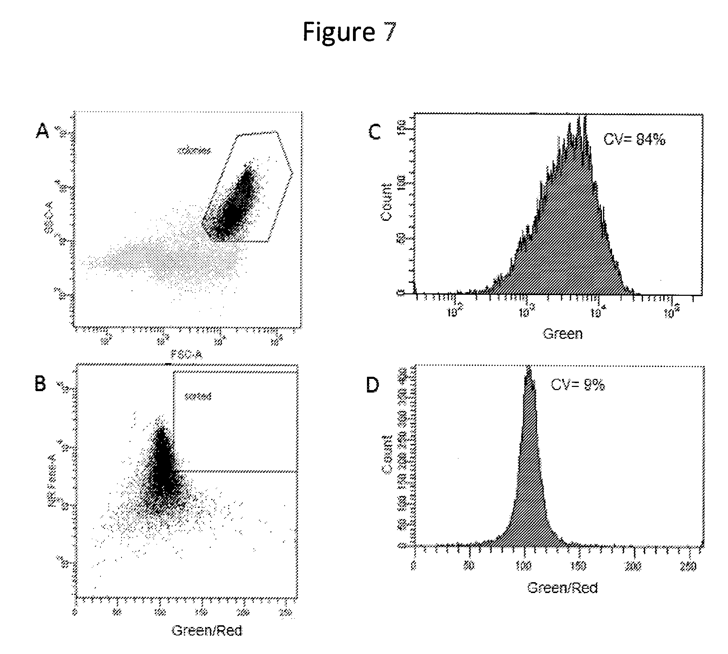

**FIG. 7** provides an exemplary FACS analysis of a farnesene producing strain encapsulated in hydrogel particles and stained with Nile Red.

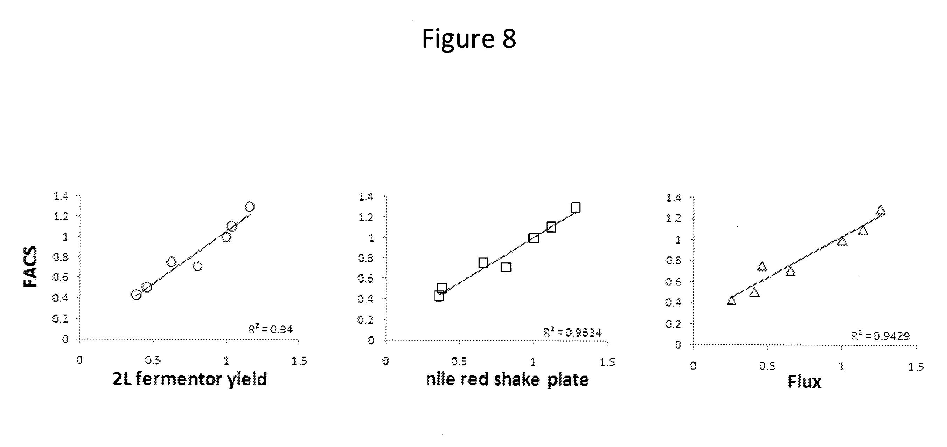

**FIG. 8** provides a correlation of farnesene production as determined by picoscreen (x-axis, "FACS") plotted against farnesene production determined by other assays (y-axis; from left to right: 2L fermentor yield, nile red shake plate assay, and farnesene flux assay). Seven strains engineered to produce varying amounts of farnesene were assayed for farnesene production.

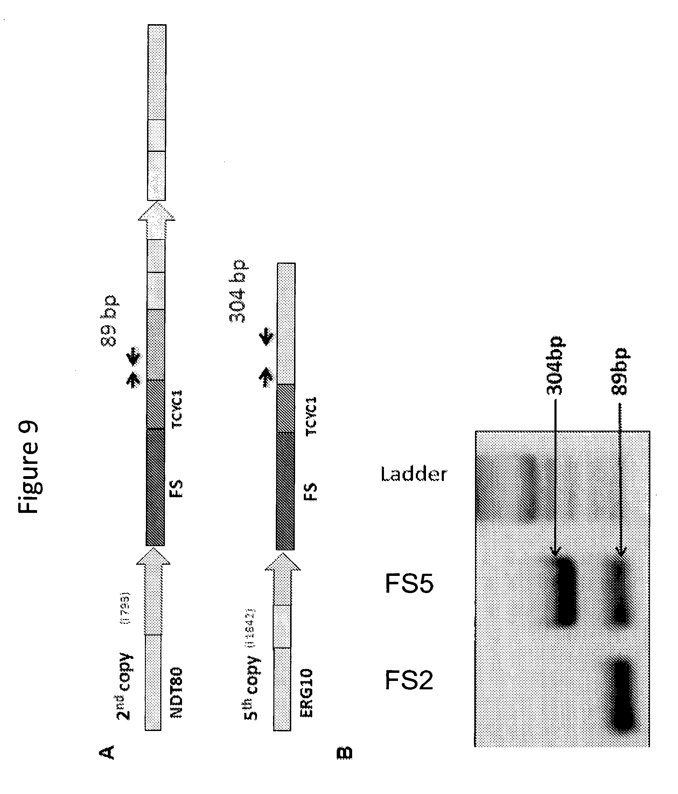

**FIG. 9** provides a PCR enrichment assay. (A) Three primers were designed to amplify two bands to distinguish strain FS2 (comprising 2 copies of farnesene synthase (FS)) from strain FS5 (comprising 5 copies of FS). An 89 bp fragment is produced from the second FS integration site in both strain FS2 and strain FS5, while a 304 bp fragment is produced from the fifth FS integration site, present only in strain FS5. (B) The presence of the 304 bp band is used as an unambiguous marker that a colony is derived from strain FS5.

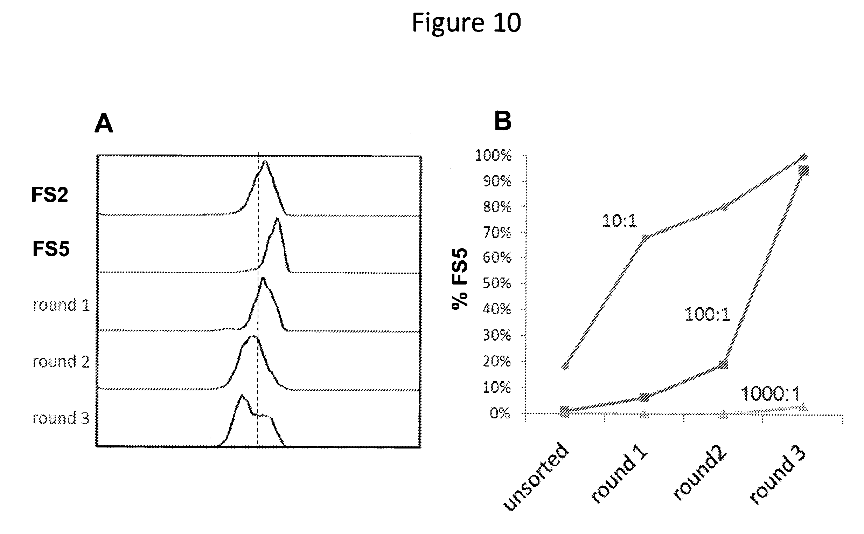

**FIG. 10** provides an enrichment of a high famesene-producing yeast strain (FS5) from a model library. The strains FS2 (low farnesene producing strain) and FS5 were mixed in ratios of 10:1, 100:1 and 1000:1, and picoscreening was performed to enrich the higher producer (FS5). (A) FACS histrogram data of the pure strains are presented in rows 1 and 2. Histograms of the mixed populations after rounds 1, 2 and 3 (rows 3 to 5) of encapsulation and sorting show that substantial enrichment of strain FS5 is achieved by round three. (B) Quantification of enrichment between rounds of sorting for each of the mixing ratios. For libraries comprising starting ratios of 10:1 and 100:1, respectively, enrichment for FS5 by round 3 is such that nearly 100% of the resulting population consists of FS5 derived colonies.

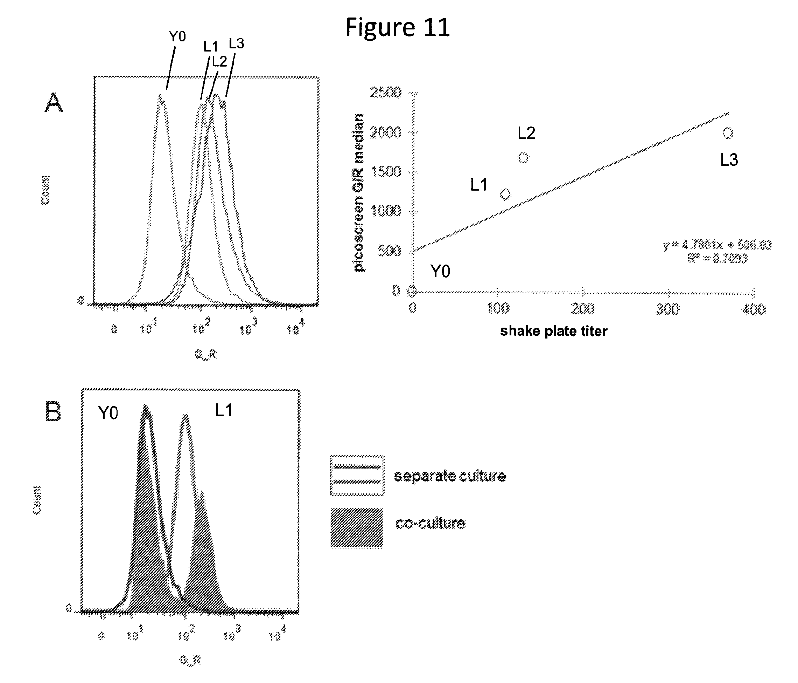

**FIG. 11** provides results of a picoscreen for the detection of limonene recombinantly produced from encapsulated yeast cells. (A) G/R fluorescence peaks of a range of limonene-producing strains subjected to the picoscreen process. Strain Y0 is a non-producer, and strains L1, L2 and L3 span a range of limonene production levels. The left panel shows that the medians of the different populations increase in fluorescence. The right panel shows the FACS median plotted as a function of 96-well shake plate titers, and demonstrates that the picoscreen values are proportional to the shake plate values. (B) Fluorescence peaks of encapsulated producing cells (L1) and non-producing cells (Y0) either co-cultured together (solid peaks) or cultured separately (hollow peaks). The separate fluorescence peaks for Y0 and L1 are maintained under co-culture conditions, indicating that product remains encapsulated in particles containing a producing strain, and does not bleed out or into particles containing a non-producing strain. Differences in the median value can be attributed to tube-to-tube variation.

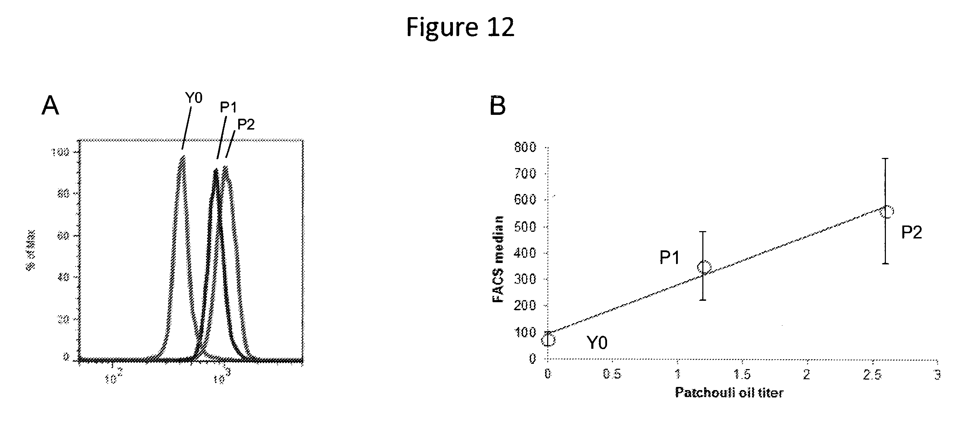

**FIG. 12** provides results of a picoscreen for the detection of patchouli recombinantly produced from encapsulated yeast cells. (A) G/R fluorescence peaks of a non-producing strain (Y0) and two different patchoulol producing strains (P1 and P2) encapsulated and subjected to picoscreen. (B) A correlation of patchoulol production as determined by picoscreen (x-axis, "FACS") plotted against standard shake plate titers (y-axis) as measured by gas chromatography (GC).

### 6. DETAILED DESCRIPTION OF THE EMBODIMENTS

### 6.1 Definitions

As used herein, the term "mevalonate pathway" or "MEV pathway" is used herein to refer to the biosynthetic pathway that converts acetyl-CoA to IPP. The MEV pathway is illustrated schematically in FIG. 1A.

As used herein, the term "deoxyxylulose 5-phosphate pathway" or "DXP pathway" is used herein to refer to the pathway that converts glyceraldehyde-3-phosphate and pyruvate to IPP and DMAPP. The DXP pathway is illustrated schematically in FIG. 1B.

As used herein, the term "encapsulated" refers to one or more cells, for example, a clonal cell population, residing primarily within the interior of a hydrogel particle as opposed to merely residing upon or attaching to the surface of the hydrogel particle. In some embodiments, the concentration of cells may be as low as a single cell within the hydrogel particle. In this embodiment, cell division from the single cell within the hydrogel particle produces an encapsulated clonal cell population.

As used herein, the term "hydrogel" refers to a cross-linked polymeric material which exhibits the ability to swell in water or aqueous solution without dissolution, and to retain a significant portion of water or aqueous solution within its structure. In some embodiments, a "hydrogel particle" as used herein has the ability to retain water-immiscible compounds, e.g., recombinantly produced water-immiscible compounds produced by a cell encapsulated within the hydrogel particle.

As used herein, the phrase "heterologous nucleotide sequence" refers to a nucleotide sequence which may be: (a) foreign to its host cell (*i*.*e*., is "exogenous" to the cell); (b) naturally found in the host cell (*i.e.,* "endogenous") but present at an unnatural quantity in the cell (*i.e.,* greater or lesser quantity than naturally found in the host cell); or (c) be naturally found in the host cell but positioned outside of its natural locus.

As used herein, the term "persistent" in the context of production of an isoprenoid by a genetically modified microbial cell refers to the ability of the genetically modified microbial cell to produce an isoprenoid compound over longer time spans in an industrial fermentation, compared to a non-genetically modified parent microbial cell.

As used herein, the term "parent" refers to a cell that serves as a starting point for introduction of genetic modifications that leads to the generation of a genetically modified microbial cell as described herein, *e.g.,* genetically modified to effect increased production and/or increased levels of a water-immiscible compound, *e.g.,* an isoprenoid, a polyketide or a fatty acid, within the cell, but does not comprise all of the genetic modifications of the genetically modified cell.

As used herein, the phrases "recombinantly produced water-immiscible compound" and "heterologous water-immiscible compound" refer to a compound produced from a genetically modified cell or microorganism having at least four carbon atoms wherein the compound is immiscible with water. The compound having at least four carbon atoms may be branched; linear or cyclic and optionally can include one or more heteroatoms (*e.g.,* nitrogen, oxygen and sulfur) as well as one or more substituents or functional moieties (*e.g.,* -OH, -NH2, -COOH, -C(H)=O, -NO3, -NH-, -C(=O)-, and the like). In some embodiments, the compound is an oil. In other embodiments, the compound is hydrophobic. Exemplary recombinantly produced, *i.e.* heterologous water-immiscible compounds of the methods and compositions provided herein include, but are not limited to, isoprenoids, polyketides, and fatty acids. In some embodiments, the recombinantly produced, *i.e.* heterologous water-immiscible compound comprises a carbon chain ranging in length from 4 carbon atoms to 40 carbon atoms. In some embodiments, the recombinantly produced, *i.e.* heterologous water-immiscible compound comprises a carbon chain of 5 to 30, 10 to 25, or 15 to 20 carbon atoms. In some embodiments, the recombinantly produced, *i.e.* heterologous water-immiscible compound comprises a carbon chain of greater than 5, 10, 15 or 20 carbon atoms. In some embodiments, the recombinantly produced, *i.e.* heterologous water-immiscible compound comprises a carbon chain of less than 40 carbon atoms.

As used herein, the phrase "directly binds" refers to a physical interaction between a first molecule *(e.g.,* a fluorescent dye) and a second molecule *(e.g.,* a water-immiscible compound) that does not require a secondary binding reagent such as an antibody.

### 6.2 Methods for Detecting Recombinantly Produced Water-immiscible Compound in a Cell

### 6.2.1 Methods of Encapsulating a Cell

In certain embodiments, the method of detecting recombinantly produced water-immiscible compound (WIC) in a cell comprises encapsulating the cell in a hydrogel particle and detecting recombinantly produced water-immiscible compound levels within the hydrogel particle. The hydrogel particle functions as a reaction vessel, or miniature fermenter, that traps the recombinantly produced water-immiscible compound that may otherwise exit the cell before the cell can be screened and/or segregated for heterologous water-immiscible compound production. In preferred embodiments, the methods comprise encapsulating a single cell in a hydrogel particle, which under suitable cell culture conditions, grows and divides within the particle to produce a clonal microcolony of cells. The caging effect of the hydrogel allows for the staining of hydrogel particles, for example, with a water-immiscible compound-specific fluorescent dye, and quantitation of heterologous water-immiscible compound production, for example, by fluorescence activated cell sorting (FACS).

In some embodiments of the methods provided herein, the step of encapsulating the cell in a hydrogel particle comprises contacting the cell with an aqueous hydrogel suspension under conditions sufficient to encapsulate the cell in a hydrogel particle. Exemplary hydrogels useful for encapsulating a cell are described in Section 5.2.1.1, and an exemplary protocol for encapsulating a cell in an agarose particle is provided in Example 2 below.

In some embodiments, the cells to be screened are collected from culture and rinsed one or more times with a physiological buffer, *e.g.,* phosphate buffered saline (PBS), and resuspended, *e.g.,* in PBS or in culture media prior to contact with the aqueous hydrogel suspension.

In some embodiments, the resuspended cells are contacted with the aqueous hydrogel suspension at a particular volume to volume (v/v) ratio. In particular embodiments, the concentration of the cell suspension will depend on the desired particle size, the hydrogel to be used for encapsulation, and the method in which the recombinantly produced water-immiscible compound is detected in the particle (*e*.*g*., FACS). In some embodiments, the concentration of cells can be calculated as a function of the desired particle size as follows: *Concentration (cells*/*L) = desired* # *of cells*/*particle* / *(4*/*3 x Pi x r³)*, where r=radius of the desired particle. The desired # of cells/particle will preferably be one or less than one, to maintain the clonogenicity of the microcolony, that is, to ensure that no more than one cell per particle is encapsulated.

In some embodiments, the cell suspension may comprise cells at a concentration 2X of the final concentration appropriate for the desired particle size and/or type of hydrogel. In some embodiments, the aqueous hydrogel suspension may be similarly formulated at 2X of the final concentration, and said contacting comprises combining the solutions at a 1:1 ratio (v/v). The solutions are mixed gently so as to not significantly impair cell viability. In some embodiments, the desired particle size is about 25 microns in diameter, and the cells are resuspended at a 2X concentration of about 7.3x10⁷ cells/ml. In other embodiments, the desired particle size is about 32 microns in diameter, and the cells are resuspended at a 2X concentration of about 3.5x10⁷ cells/ml. In other embodiments, the desired particle size is about 50 microns in diameter, and the cells are resuspended at a 2X concentration of about 9x10⁶ cells/ml. In other embodiments, the desired particle size is about 100 microns in diameter, and the cells are resuspended at a 2X concentration of about 1x10⁶ cells/ml. In some embodiments, the cells are resuspended at a 2X concentration of about 1x10⁶, 2x10⁶, 3x10⁶, 4x10⁶, 5x10⁶, 6x10⁶, 7x10⁶, 8x10⁶, 9x10⁶, 1x10⁷, or more than 1x10⁷.

In a particular embodiment, a 2X concentration of cells comprising about 3.7x10⁷ cells/ml is contacted with a 2X aqueous agarose solution comprising 1.5% agarose. In another particular embodiment, a 2X concentration of cells comprising about 9x10⁶ cells/ml is contacted with a 2X aqueous agarose solution comprising 2% agarose. In another particular embodiment, a 2X concentration of cells comprising about 1x10⁶ cells/ml is contacted with a 2X aqueous agarose solution comprising 3% agarose.

Following contact of the cell with the aqueous hydrogel suspension, a hydrogel particle can be formed using any method known in the art. In some embodiments, spherical hydrogel particles are formed by dispersing the liquid hydrogel matrix (comprising cells) in a water-immiscible oil, *e.g.,* a fluorocarbon oil. In some embodiments, to ensure uniformly-sized particles of the desired size are produced, a microfluidic device comprising a junction, *e.g.,* a T-junction, formed by the intersection of an aqueous flow channel and a transversely positioned oil flow channel, is used to form the water-in-oil emulsion. The liquid hydrogel matrix (comprising cells) is loaded, *i.e.,* streamed into one channel, and the immiscible oil is loaded, *i.e.,* streamed into a channel transversely positioned to the channel comprising the aqueous hydrogel solution, and contact is effected at the T-junction, leading to formation of a hydrogel particle comprising a cell. Newly formed particles are then streamed through a collection channel and collected, *e.g.,* in a collection tube. The gel is then hardened, for example, by lowering the temperature of the solution or by the addition of crosslinker, and the particles are transferred from the oil into an aqueous solution, for example, a nutrient growth medium.

In some embodiments, the hydrogel particles useful in the methods provided herein can be formed without the use of a microfluidic device. For example, membrane emulsification can produce emulsions with relatively small size distributions. In situations where low size polydispersity is less important, other methods of forming emulsions, such as stirring, homogenization, or shaking are useful. Other useful methods include forming a jet of liquid in air, then breaking the jet into aerosol particles by mechanically cutting the jet with a spinning wire, shaking the jet with a high-frequency transducer, or shearing the jet by forcing it through an orifice with pressurized air.

### 6.2.1.1 Hydrogels

A hydrogel is a polymer that has been hardened into a solid matrix, which is swollen by, but not dissolved by water. In some embodiments, the mesh size of the hydrogel is small enough to encapsulate larger objects, such as cells and micron-scale water-immiscible compound drops, but large enough to allow small molecules and ions, *e.g.,* which support cell growth and proliferation, or which are required for detection of the recombinantly produced water-immiscible compound, to diffuse freely through the matrix.

In some embodiments, the mesh size of the hydrogel is between about 1 nm and 100 nm. In some embodiments, the mesh size of the hydrogel is between about 10 nm and 90 nm. In some embodiments, the mesh size of the hydrogel is between about 20 nm and 80 nm. In some embodiments, the mesh size of the hydrogel encapsulation is about 5, 10, 15, 20, 25, 30, 35, 40, 45, 50, 55, 60, 65, 70, 75, 80, 85, 90, 95, or 100 nm.

In some embodiments, the size of the hydrogel particle is large enough to trap a colony of tens of cells, but small enough to fit through the fluidic components of, *e.g.,* a commercial cell sorter. In some embodiments, the hydrogel particles are 20-50 microns in diameter. In some embodiments, the hydrogel particles are 30-40 microns in diameter. In some embodiments, the hydrogel particles are about 20, 21, 22, 23, 24, 25, 26, 27, 28, 29, 30, 31, 32, 33, 34, 35, 36, 37, 38, 39, 40 41, 42, 43, 44, 45, 46, 47, 48, 49, or 50 microns in diameter. In some embodiments, the hydrogel particles are between about 50 to 100 microns in diameter. In some embodiments, the hydrogel particles are about 50, 60, 70, 80, 90, 100 or greater than 100 microns in diameter.

In some embodiments, complete encapsulation of a single founder cell enables a small colony containing a clonal population cells to remain trapped immediately adjacent to the founder cell, and separate from other founder cells. Thus, the phenotype of one genotype can be averaged over many tens of cells, which greatly reduces assay noise. In a preferred embodiment, the hydrogel matrix localizes recombinantly produced water-immiscible compound by shielding embedded objects within the hydrogel from shear forces and convection. Therefore, small molecules, such as molecular hydrocarbons, can slowly migrate away from the producing cells by diffusion alone. This slow movement away from the origin of production leads to a high concentration, which initiates nucleation and growth of micron-scale water-immiscible compound drops. As the water-immiscible compound drops approach and exceed the mesh size of the gel, they are permanently caged in the matrix and remain associated with the cells that produced them. Because the drops are shielded from shear forces that would be present outside the gel matrix, they cannot be broken down into smaller drops that might be able to diffuse out of the particles.

In some embodiments, the cells are encapsulated in a hydrogel formed from any bio-polymer which supports cell growth. Bio-polymers are naturally-occurring polymers, such as proteins and carbohydrates. In preferred embodiments, the bio-polymer is biocompatible and non-cytotoxic to the encapsulated cell. Examples of suitable bio-polymers useful in the present methods include, but are not limited to, collagens (including Types I, II, III, IV and V), denatured collagens (or gelatins), recombinant collagens, fibrin-fibrinogen, elastin, glycoproteins, alginate, chitosan, hyaluronic acid, chondroitin sulphates and glycosaminoglycans (or proteoglycans). In some embodiments, the bio-polymer is in its naturally-occurring form. In other embodiments, the bio-polymer is derivatised to facilitate cross-linking with a synthetic polymer.

In particular embodiments, the bio-polymer is selected from the group consisting of thermally gelling polysaccharides, such as agarose, or thermally gelling proteins, such as gelatin.

Agarose is a natural polymer extracted from seaweed, and varies in its properties *(e.g.,* molecular weight, precise chemical composition, side chains, *etc*.). Agarose can be solidified by reducing the temperature of the solution, without the addition of any other components. This property is particularly useful for the formation of particles, especially when using microfluidics or other emulsion-based methods to form the hydrogel particles described herein. In preferable embodiments, the agarose has a gelling temperature such that living cells may be mixed into a solution of agarose at a temperature that does not significantly impair cell viability. In preferable embodiments, the agarose does not gel at a temperature higher than that which is compatible with cell viability. Thus, if the gelation temperature of the agarose is above the cell viability temperature, the agarose or gel should not gel more quickly than is necessary to mix the cells into the agarose solution (pre-gel state) at a temperature below the gelation temperature. In some embodiments, the agarose permits mixing (e.g., via gentle mechanical stirring or pipetting) at a temperature ranging from about 18° C to 60°C, 24° C to 50°C, 30° C to 40°C, or 32° C to 37°C.

In some embodiments, the agarose solution takes more than one minute to gel, so as to allow sufficient time to mix the cells into the solution. In some embodiments, the agarose solution gels in less than about four hours, more preferably less than one hour, and more preferably on the order of minutes (*e.g.,* about 1 to 20 minutes, or about 2 to 5 minutes). One of skill in the art will appreciate that the actual gel point temperature is not critical if the gelation is sufficiently slow and as long as the gel is stable at the temperature range which preserves cell viability.

Other thermally gelling proteins useful for the methods provided herein include, but are not limited to elastin-mimetic protein polymers and silk-elastin block copolymers. *See, e.g.,* Hurt and Gehrke, J. Phar. Sci. Vol. 96 No. 3 (March 2007); McMillan et al., (1999) Macromolecules 32: 3643-3648; Huang et al., (2000) Macromolecules 33: 2989-2997 and McMillan et al., (2000) Macromolecules 33: 4809-4821.

Other potentially useful thermally gelling polysaccharides include kappa-carrageenan and iota-carrageenan.

In some embodiments, the cells are encapsulated in any bio-synthetic matrix which supports cell growth. In some embodiments, the bio-synthetic matrix is a polymer comprising polyacrylamide, or one or more acrylamide derivatives. As used herein, an "acrylamide derivative" refers to a N-alkyl or N,N-dialkyl substituted acrylamide or methacrylamide. Examples of acrylamide derivatives suitable for use in encapsulating cells include, but are not limited to, N-methylacrylamide, N-ethylacrylamide, N-isopropylacrylamide (NiPAAm), N-octylacrylamide, N-cyclohexylacrylamide, N-methyl-N-ethylacrylamide, N-methylmethacrylamide, N-ethylmethacrylamide, N-isopropylmethacrylamide, N,N-dimethylacrylamide, N,N-diethylacrylamide, N,N-dimethylmethacrylamide, N,N-diethylmethacrylamide, N,N-dicyclohexylacrylamide, N-methyl-N-cyclohexylacrylamide, N-acryloylpyrrolidine, N-vinyl-2-pyrrollidinone, N-methacryloylpyrrolidine, and combinations thereof.

In some embodiments, the cells are encapsulated in a physically cross-linked polymer, a chemically cross-linked polymer, electrostatically cross-linked or an entangled polymer, which supports cell growth. In some embodiments, the cells are encapsulated in a hydrogel network comprising one or more of a physically cross-linked polymer, a chemically cross-linked polymer, electrostatically cross-linked or an entangled polymer. In some embodiments, the one or more co-polymer should be sufficiently soluble in aqueous solution to facilitate hydrogel formation.

In a particular embodiment, the entangled polymer is selected from the group consisting of thermally gelling polysaccharides such as agarose or thermally gelling proteins such as gelatin, and the physically cross-linked, chemically cross-linked, or electrostatically cross-linked copolymer is synthesized from a water-soluble vinyl monomer such as polyethylene glycol diacrylate ("PEG-DA") and 2-hydroxyethyl methacrylate ("HEMA"), either as a homopolymer or copolymer.

In some embodiments, the hydrogel network comprises a bio-polymer or a derivatised version thereof cross-linked to the synthetic polymer by means of the pendant cross-linking moieties in the synthetic polymer. Thus, in this embodiment, the bio-polymer contains one or more groups which are capable of reacting with the cross-linking moiety of the synthetic polymer (*e.g.* a primary amine or a thiol), or can be derivatised to contain such a group. Cells can be readily entrapped in the final matrix by addition of the cells to a solution of the synthetic polymer prior to admixture with the bio-polymer to form a cross-linked hydrogel. Alternatively, the cells can be added to a solution containing both the synthetic and bio-polymers prior to the cross-linking step. For the encapsulation of cells in the matrix, the various components (cells, synthetic polymer and bio-polymer) are dispersed in an aqueous medium, such as a cell culture medium or a diluted or modified version thereof. The cell suspension is mixed gently into the polymer solution until the cells are substantially uniformly dispersed in the solution, then the hydrogel is formed as described above.

In some embodiments, where the cells are to be encapsulated in a hydrogel comprised of one or more cross-linked polymers, *e.g.,* a physically cross-linked, chemically cross-linked, or electrostatically cross-linked polymer, the polymer is preferably polymerized and/or cross-linked over a period of time so that that a substantial portion of the living cells remain viable. In some embodiments,, the cells are cross-linked for less than about one hour, less than about 50, 40, 30, 20, 10, 9, 8, 7, 6, 5, 4, 3, 2, or 1 minutes.

### 6.2.1.2 Microfluidic Devices

An exemplary microfluidic device, useful for forming hydrogel particles as described above, comprises a substrate having at least one surface with a plurality of flow channels fabricated into the surface. A "channel" as used herein refers to a feature on or in a substrate that at least partially directs the flow of a fluid. In some cases, the channel may be formed, at least in part, by a single component, *e*.*g*., an etched substrate or molded unit. The channel can have any cross-sectional shape, for example, circular, oval, triangular, irregular, square or rectangular (having any aspect ratio), or the like, and can be covered or uncovered *(i.e.,* open to the external environment surrounding the channel). In embodiments where the channel is completely covered, at least one portion of the channel can have a cross-section that is completely enclosed, and/or the entire channel may be completely enclosed along its entire length with the exception of its inlet and outlet.

In some embodiments, the device comprises at least two flow channels fabricated into the surface, wherein at least one channel is fluidly connected to at least one transversely positioned channel at a first point in each of the channels, *i.e.,* at a T-junction. In some embodiments, the device comprises at least two inlets, *i.e.,* an area of the device that receives, *e.g.,* cells in aqueous solution, or fluorocarbon oil, respectively. Each inlet can comprise one or more inlet channels, wells or reservoirs, openings, and other features which facilitate the entry of *e.g.,* cells in aqueous solution, or fluorocarbon oil, into the substrate. An inlet generally comprises a junction between the sample inlet channel (*e*.*g*., the cell inlet channel or the oil inlet channel) and the main channel (*e*.*g*., the cell solution flow channel or the oil flow channel, respectively) such that a solution of a sample *(e.g.,* cells in aqueous solution, or fluorocarbon oil) is introduced to the main channel. In some embodiments, the device may contain more than one inlet, *e.g.,* more than one cell inlet or more than one oil inlet, if desired. In some embodiments, different sample inlet channels can communicate, *i.e.,* can be in fluid communication with, the main channel at different inlets. For example, multiple cell inlet channels can communicate with the cell solution flow channel, and multiple oil inlet channels can communicate with the oil flow channel. Alternately, different sample inlet channels can communicate with the main channel at the same inlet.

In some embodiments, the device comprises a sample solution reservoir, or well, or other apparatus for introducing a sample to the device, at an inlet, *e.g.,* the cell inlet or oil inlet, which is in fluid communication with an inlet channel. Reservoirs and wells used for loading one or more samples onto the microfluidic device include but are not limited to, syringes, cartridges, vials, eppendorf tubes and cell culture materials.

One embodiment of a microfluidic device useful for facilitating the formation of hydrogel particle formation is shown in FIG. 3. The overall device is fabricated in a planar substrate and comprises a cell inlet which is fluidly connected to a cell solution flow channel, which is fluidly connected to a particle flow channel, followed by a particle collection outlet at its terminus. The device further comprises an oil inlet which is fluidly connected to an oil flow channel, which is transversely positioned to the cell solution flow channel and intersects the cell solution flow channel at a T-junction. The oil flow channel can intersect the cell solution channel such that the oil is introduced into the cell solution flow channel at an angle perpendicular to a stream of fluid passing through the cell solution flow channel. In some embodiments, the oil flow channel and cell solution flow channel intercept at a T-junction, *i.e.*, such that the oil flow channel is perpendicular (90 degrees) to the cell solution flow channel. In other embodiments, the oil flow channel can intercept the cell solution flow channel at any angle, and need not introduce the oil to the cell solution flow channel at an angle that is perpendicular to that flow. In some embodiments, the angle between intersecting channels is in the range of from about 60 to about 120 degrees. In some embodiments, the angle between intersecting channels is about 45, 60, 90, or 120 degrees.

In some embodiments, the movement of a solution, *e.g.,* cells in aqueous solution, or fluorocarbon oil, through the microfluidic device is driven by pressure drive flow control, and can utilize valves and pumps to manipulate the flow of the solution in one or more directions and/or into one or more channels of the microfluidic device. In some embodiments, the pressure within a flow channel can be regulated by adjusting the pressure on the respective inlet channel, for example, with pressurized syringes feeding into the inlet channel. By controlling the pressure difference between the oil and cell solution sources at their respective inlet, the size and periodicity of the hydrogel particles generated may be regulated. The size and periodicity of the hydrogel particles may also depend on channel diameter, the viscosity of the fluids, and shear pressure.

**FIG. 4** provides a depiction of an exemplary process for forming a hydrogel encapsulated cell. Cells enter from the left from the cell inlet in a continuous aqueous stream through the cell solution flow channel. Upon contact at the T-junction with the fluorocarbon oil flowing through the oil flow channel, the cell solution becomes dispersed or discontinuous, and particles are formed which are surrounded by oil and channeled through the particle flow channel. At the exit of the device (*i.e.,* the particle collection outlet), the particles enter a length of tubing and flow into a container, such as a microcentrifuge tube. The gel particles are hardened, then separated from, *i.e.,* washed out of the fluorocarbon oil and into an aqueous buffer.

In some embodiments, the cross sectional dimensions of the particle flow channel of the microfluidic device will contribute to the size of the hydrogel particle that is formed, *i.e.,* after the hydrogel suspension contacts the fluorocarbon oil at the T-junction. In some embodiments, the particle flow channel will have a cross sectional dimension, *e.g.,* in the range of from about 0.1 µm to about 500, µm, 0.1 µm to about 200 µm, 0.1 µm to about 100 µm, 0.1 µm to about 50 µm, or less than 50 µm. Similarly, in some embodiments, the cross sectional dimensions of the oil flow channel will contribute to the size of the hydrogel particle that is formed after the hydrogel suspension contacts the fluorocarbon oil at the T-junction. In some embodiments, the oil flow channel will have a cross sectional dimension, *e.g.,* in the range of from about 0.1 µm to about 500, µm, 0.1 µm to about 200 µm, 0.1 µm to about 100 µm, 0.1 µm to about 50 µm, or less than 50 µm.

Suitable substrate materials for producing the microfluidic device, useful in facilitating the formation of a hydrogel particle, are generally selected based upon their compatibility with the nature of the particles to be formed. Such conditions can include extremes of pH, temperature, the fluorocarbon oil/fluorosurfactant applied thereto, the polymers applied thereto, and the like. Examples of useful substrate materials include, *e.g.,* glass, quartz and silicon as well as polymeric substrates, *e.g.* plastics. In some embodiments, the substrate materials are rigid, semi-rigid, or non-rigid, opaque, semi-opaque or transparent. For example, devices which include an optical or visual element to allow for the monitoring of particle formation, will generally be fabricated, at least in part, from transparent materials to allow, or at least, facilitate that monitoring. Alternatively, transparent windows of, *e.g.,* glass or quartz, are optionally incorporated into the device for these types of monitoring elements. Additionally, the polymeric materials may have linear or branched backbones, and are optionally crosslinked or non-crosslinked. Examples of particularly suitable polymeric materials include, *e.g.,* polydimethylsiloxanes (PDMS), polyurethane, polyvinylchloride (PVC) polystyrene, polysulfone, polycarbonate and the like.

Manufacturing of microscale elements, *e.g.,* flow channels, into the surface of the substrates may generally be carried out by any number of microfabrication techniques that are well known in the art. For example, lithographic techniques are optionally employed in fabricating, *e.g.,* glass, quartz or silicon substrates, using methods well known in the semiconductor manufacturing industries such as photolithographic etching, plasma etching or wet chemical etching. Alternatively, micromachining methods such as laser drilling, micromilling and the like are optionally employed. Similarly, for polymeric substrates, well known manufacturing techniques may also be used. These techniques include injection molding or stamp molding methods where large numbers of substrates are optionally produced using, *e.g.,* rolling stamps to produce large sheets of microscale substrates or polymer microcasting techniques where the substrate is polymerized within a micromachined mold.

The devices will typically include an additional planar element which overlays the channeled substrate enclosing and fluidly sealing the various flow channels to form conduits. Attaching the planar cover element is achieved by a variety of means, including, *e.g.,* thermal bonding, adhesives or, in the case of certain substrates, *e.g.,* glass, or semi-rigid and non-rigid polymeric substrates, a natural adhesion between the two components. The planar cover element may additionally be provided with access ports and/or reservoirs for introducing the various fluid elements needed for hydrogel particle formation.

Surface modification of polymeric substrates may take on a variety of different forms. For example, to prevent material (*e.g.,* cells and/or hydrogel particles) from adhering to the sides of the channels, the channels (and coverslip, if used) may have a coating which minimizes adhesion. The surface of the channels of the microfluidic device can be coated with any anti-wetting or blocking agent for the dispersed phase. The channel can also be coated with any protein to prevent adhesion of the biological/chemical sample. Channels can be coated by any means known in the art. For example, the channels can be coated with a hydrophobic coating of the type sold by PPG Industries, Inc. under the trademark Aquapel (*e.g.,* perfluoroalkylalkylsilane surface treatment of plastic and coated plastic substrate surfaces in conjunction with the use of a silica primer layer) and disclosed in U.S. Pat. No. 5,523,162. By fluorinating the surfaces of the channels, the continuous phase preferentially wets the channels and allows for the stable generation and movement of droplets through the device. The low surface tension of the channel walls thereby minimizes the accumulation of channel clogging particulates. By fluorinating the surfaces of the channels, the continuous phase preferentially wets the channels and allows for the stable generation and movement of material (e.g., cells and/or hydrogel particles) through the device. The low surface tension of the channel walls thereby minimizes the accumulation of channel clogging particulates.

In one embodiment, preparation of a charged surface on the substrate involves the exposure of the surface to be modified, *e.g.,* the flow channels, to an appropriate solvent which partially dissolves or softens the surface of the polymeric substrate. Selection of appropriate solvents will generally depend upon the polymeric material that is used for the substrate. For example, chlorinated hydrocarbon solvents, *i.e.,* trichloroethane (TCE), dichloroethane and the like, are particularly useful as solvents for use with PMMA and polycarbonate polymers. A detergent is then contacted with the partially dissolved surface. The hydrophobic portion of the detergent molecules will associate with the partially dissolve polymer. A wide variety of detergents may be used, for example, SDS (sodium dodecyl sulfate), DTAB (dodecyltrimethylammonium bromide), or CTAB (cetyltrimethylammoniumbromide). The solvent is then washed from the surface, *e.g.,* using water, whereupon the polymer surface hardens with the detergent embedded into the surface, presenting the charged head group to the fluid interface.

In alternative aspects, polymeric materials, such as polydimethylsiloxane, may be modified by plasma irradiation. In particular, plasma irradiation of PDMS oxidizes the methyl groups, liberating the carbons and leaving hydroxyl groups in their place, effectively creating a glass-like surface on the polymeric material, with its associated hydroxyl functional groups.

The polymeric substrate may be rigid, semi-rigid, non-rigid or a combination of rigid and non-rigid elements. In one embodiment, a substrate is made up of at least one softer, flexible substrate element and at least one harder, more rigid substrate element, one of which includes the channels and chambers manufactured into its surface. Upon mating the two substrates, the inclusion of the soft element allows formation of an effective fluid seal for the channels and chambers, obviating the need and problems associated with gluing or melting more rigid plastic components together.

### 6.2.1.3 Fluorocarbon Oils

Fluorocarbon oil continuous phases are well-suited as the continuous phase for use in forming hydrogel particles as provided herein. Fluorous oils are both hydrophobic and lipophobic, and thus, have low solubility for components of the aqueous phase. In addition, in contrast to hydrocarbon or silicone oils, fluorous oils do not swell PDMS materials, which is a convenient material for constructing microfluidic channels.

In some embodiments, the fluorocarbon oil is immiscible with the aqueous phase. In some embodiments, the fluorocarbon oil stabilizes hydrogel particles upon formation and subsequent hardening/polymerization. In some embodiments, the fluorocarbon oil maintains chemical and biological inertness with the hydrogel particle, and does not adversely affect the viability of the cells within the particle. In some embodiments, the oil solution does not swell, dissolve, or degrade the materials used to construct the microfluidic device. Preferably, the physical properties of the oil (*e*.*g*., viscosity) should be suitable for the flow and operating conditions of the encapsulation process.

Exemplary fluorocarbon oils for use in the methods provided herein include hydrofluoroethers, which are fluorinated alkyl chains coupled with hydrocarbon chemistry through and ether bond (*i.e.,* Novec Engineered Fluids or HFE-series oils). Useful HFE-series oils include but are not limited to, HFE-7500, HFE-7100, HFE -7200, and HFE -7600. In a particular embodiment, the fluorocarbon oil is 3M Novec HFE-7500, at 1% by weight. HFE-series oils can be used as stand-alone oils or components of oil mixtures to optimize emulsion properties and performance. Other useful fluorocarbon oils include perfluoroalkylamines (*i.e.,* Fluorinert Electronic Liquids (FC-oils)), which are perfluorinated oils based on perfluoroalkyl amine structures. Useful FC-oils include Fluorinert FC-3283 and Fluorinert FC-40. FC-oils can also be used as stand-alone oils or components of oil mixtures to optimize emulsion properties and performance.

In some embodiments, the fluorocarbon oil comprises a fluorosurfactant. Surfactants that may be added to the continuous phase fluid include, but are not limited to, surfactants such as sorbitan-based carboxylic acid esters (*e.g.,* the "Span" surfactants, Fluka Chemika), including sorbitan monolaurate (Span 20), sorbitan monopalmitate (Span 40), sorbitan monostearate (Span 60) and sorbitan monooleate (Span 80), and perfluorinated polyethers (*e.g.,* DuPont Krytox 157 FSL, FSM, and/or ESH). Other non-limiting examples of non-ionic surfactants which may be used include polyoxyethylenated alkylphenols (for example, nonyl-, p-dodecyl-, and dinonylphenols), polyoxyethylenated straight chain alcohols, polyoxyethylenated polyoxypropylene glycols, polyoxyethylenated mercaptans, long chain carboxylic acid esters (for example, glyceryl and polyglycerl esters of natural fatty acids, propylene glycol, sorbitol, polyoxyethylenated sorbitol esters, polyoxyethylene glycol esters, etc.) and alkanolamines (*e.g.,* diethanolamine-fatty acid condensates and isopropanolamine-fatty acid condensates). In a particular embodiment, the fluorosurfactant is the ammonium carboxylate salt of Krytox 157 FSH.

An additional exemplary fluorosurfactant, that may be added to the continuous phase fluid, *e.g.* fluorocarbon oil, can be synthesized as follows:

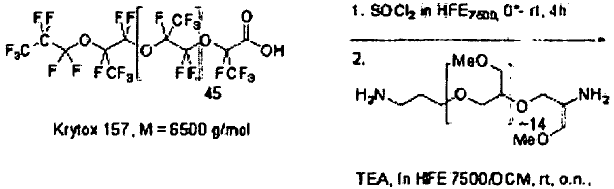

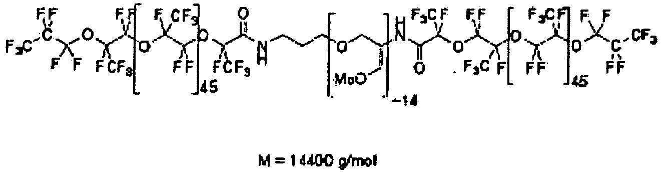

### 6.2.2 Methods of Detection

### 6.2.2.1 Cell Culture

Following hydrogel particle formation, the gel particle suspension is separated from the fluorocarbon oil, transferred to growth medium and grown under suitable conditions, for example, in a medium comprising a carbon source under conditions suitable for heterologous water-immiscible compound production. In some embodiments, the conditions comprise growing the cells in a tube in a 30 °C shaking incubator to allow the cells to divide and produce heterologous water-immiscible compound. The product stays within the agarose particles, and therefore remains associated with the cells from which it was produced.

Suitable conditions and suitable media for culturing microbial cells are well known in the art. In some embodiments, the carbon source is a monosaccharide (simple sugar), a disaccharide, a polysaccharide, a non-fermentable carbon source, or one or more combinations thereof. Non-limiting examples of suitable monosaccharides include glucose, galactose, mannose, fructose, ribose, and combinations thereof. Non-limiting examples of suitable disaccharides include sucrose, lactose, maltose, trehalose, cellobiose, and combinations thereof. Non-limiting examples of suitable polysaccharides include starch, glycogen, cellulose, chitin, and combinations thereof. Non-limited examples of suitable non-fermentable carbon sources include acetate and glycerol. In some embodiments, the suitable medium is supplemented with one or more additional agents, such as, for example, an inducer (*e.g.,* when one or more nucleotide sequences encoding a gene product is under the control of an inducible promoter), a repressor (*e*.*g*., when one or more nucleotide sequences encoding a gene product are under the control of a repressible promoter), or a selection agent (*e.g.,* an antibiotic to select for microbial cells comprising the genetic modifications).

In some embodiments, the hydrogel particles comprising the cells are cultured under conditions suitable for heterologous water-immiscible compound production for a period of at least 12 hours. In some embodiments, the hydrogel particles comprising the cells are cultured under conditions suitable for heterologous water-immiscible compound production for a period of 12 to 24 hours. In some embodiments, the hydrogel particles comprising the cells are cultured under conditions suitable for heterologous water-immiscible compound production for a period of at least 24 hours. In some embodiments, the hydrogel particles comprising the cells are cultured under conditions suitable for heterologous water-immiscible compound production for a period of about 12, 24, 36, 48, 60, 72 or more than 72 hours.

### 6.2.2.2 Detection

Recombinantly produced water-immiscible compound encapsulated within a hydrogel particle, *i.e.,* produced from a cell or clonal population of cells encapsulated therein, can be detected using any standard cell detection technique known in the art, such as flow cytometry, cell sorting, fluorescence activated cell sorting (FACS), magnetic activated cell sorting (MACS), by examination of the particles and/or cells encapsulated therein using light or confocal microscopy, and/or isolating the encapsulated cells.

In particular embodiments, particles comprising water-immiscible compound producing cells may be sorted using a fluorescence activated cell sorter (FACS). Fluorescence activated cell sorting (FACS) is a well-known method for separating particles, including cells, based on the fluorescent properties of the particles (Kamarch, 1987, Methods Enzymol, 151:150-165). Laser excitation of fluorescent moieties in the individual particles results in a small electrical charge allowing electromagnetic separation of positive and negative particles from a mixture. In one embodiment, the particles are stained with a lipophilic fluorescent dye that binds, *e.g.,* directly binds to the heterologous water-immiscible compound produced by the cells encapsulated in the particle. Particles are processed through the cell sorter, allowing separation of particles based on their ability to bind to the fluorescent label used. In some embodiments, forward or side scatter can be used to distinguish cell-occupied from unoccupied hydrogel particles. The occupied particles can then be further gated to detect a sub-population of cells having the desired fluorescence profile. FACS sorted particles may be directly deposited into individual wells of 96-well or 384-well plates to facilitate separation, isolation and cloning of the cells encapsulated therein.

In some embodiments of the methods of detecting, screening and/or enriching provided herein, the method comprises contacting the hydrogel particle with a fluorescent dye that directly binds to the recombinantly produced water-immiscible compound and detecting the fluorescent dye within the hydrogel particle. In some embodiments, the fluorescent dye is a solvatochromic dye. Fluorescent solvatochromic dyes are dyes that change color depending on the polarity of the solvent surrounding the molecules and are used, for example, as probes in high sensitivity real time observations of dynamics of biological molecules, particularly of lipid molecules. The color changing mechanism thereof is achieved through direct binding and does not require contact with specific chemical species. Such fluorescent solvatochromic dyes include NBD, Dansyl, DASPMI, Prodan, Dapoxyl, 4-DMAP, 4-amino-1,8-naphthalimide derivatives, Reichardt's dye, and Nile Red.

In some embodiments, the fluorescent solvatochromic dye is Nile Red. Nile Red is a lipid-soluble fluorescent dye that has frequently been used for the detection of intracellular lipid droplets by fluorescence microscopy and flow cytofluorometry, for example, to evaluate the lipid content of animal cells and microorganisms, including mammalian cells, bacteria, yeasts and microalgae. Nile Red has several unique properties that make it ideal for the high throughput detection of recombinantly produced water-immiscible compounds described herein. For example, Nile Red is highly fluorescent in a hydrophobic environment, is quenched in a hydrophilic environment, and like other solvatochromic dyes, its excitation and emission spectra vary in spectral position, shape, and intensity with the nature of its environment. The solvatochromic property of Nile Red allows for the partial differentiation of Nile Red bound to phospho- and polar lipids and that bound to neutral lipids. In a polar lipid, such as the phospholipid cell membrane, Nile Red has a fluorescence emission maximum of ∼ 590 nm. By contrast, in the presence of a neutral lipid, for example, a hydrocarbon product (*e.g.,* famesene), the spectrum is blue-shifted with an emission maximum of 550 nm. Thus, in certain embodiments of the methods described herein, optical filters in the green (525 +/- 20 nm) and red (670 +/- 20 nm) regions of the spectrum are used during detection in order to maximize the ratio of green to red fluorescence between the ideal producing cell *(e.g.,* pure farnesene) and a complete non-producing cell. Fluorescence data can be captured in both the green and red spectrums, and the ratio of green to red fluorescence can be used to determine the amount of water-immiscible compound within the microcolony normalized to the amount of cell biomass in the microcolony. Thus, the methods provided herein advantageously utilize solvatochromic dyes such as Nile Red to simultaneously determine: (a) the amount of water-immiscible compound produced by an encapsulated microcolony; and (b) the cell biomass within the encapsulated microcolony. By obviating the requirement for separate determinations of cell biomass, for example, by counterstaining the microcolony with a cell wall or nuclear specific stain, or measuring the optical density of the microcolony, higher throughput and efficiency can be achieved compared to other screening methods.

The ratio of green to red fluorescence (G/R) of a microcolony can be advantageously used to determine the relative product:biomass ratios within an encapsulated colony of cells, and the population can be ranked accordingly. For example, a picoscreened colony can be ranked as having: (a) a relatively high G/R ratio, which may indicate a relatively slow growing/high producing population; or (b) a relatively low G/R ratio, which may indicate a relatively fast growing/low producing population, a relatively fast growing/high producing population, or a relatively slow growing/low producing strain. The G/R ratio of the microcolony can further be used in combination with its green fluorescence value alone (G), which is indicative of the amount of compound produced by the population, to further characterize the population. For example, a picoscreened colony having a low G/R ratio but high G value may indicate a relatively fast growing/high producing population, and a picoscreened colony having a low G/R ratio but low G value may indicate a relatively slow growing/low producing population or fast growing/low producing population.

Thus, in some embodiments of the methods of detecting, screening and/or enriching provided herein, the method comprises normalizing the amount of water-immiscible compound within the hydrogel particle to the amount of cell biomass within the hydrogel particle. In some embodiments, said normalizing comprises determining: (a) the level of fluorescence of the water immiscible compound within the hydrogel particle, and (b) the level of fluorescence of cell biomass within the hydrogel particle; and determining the ratio of fluorescence determined in (a) to that determined in (b). In some embodiments, the fluorescent dye is Nile Red, and said normalizing comprises determining the level of fluorescence within the green spectrum (*e.g.,* 525 +/- 20 nm), corresponding to the level water-immiscible compound within the hydrogel particle, and determining the level of fluorescence within the red spectrum (670 +/- 20 nm), corresponding to the level of cell biomass within the hydrogel particle, and determining the ratio of green to red fluorescence (G/R). In some embodiments, the methods further comprise selecting a hydrogel particle having a high G/R ratio. In some embodiments, the methods further comprise selecting a hydrogel particle having a high level of green fluorescence. In some embodiments, the methods further comprise selecting a hydrogel particle having a high G/R ratio and a high level of green fluorescence.

### 6.2.2.3 Selecting Spectral Conditions for Detection

The determination of spectral conditions suitable for the selective detection of fluorescent dye bound to WIC produced from a plurality of encapsulated cells can be carried out in several embodiments. In one embodiment, for any combination of: (1) WIC recombinantly produced by a plurality of cells; (2) a fluorescent dye that directly binds the WIC; and (3) a host cell, the spectral conditions can be determined by a method comprising the step of identifying an excitation wavelength that enables the specific detection of the dye bound to the WIC. In some embodiments, the method comprises the step of identifying an emission wavelength that enables the specific detection of the dye bound to the WIC. In some embodiments, the method comprises the step of identifying an excitation and emission wavelength pairing that enables the specific detection of the dye bound to the WIC. In preferred embodiments, the method comprises identifying an excitation and emission wavelength pairing that is sufficiently selective for the detection of fluorescent dye bound to the WIC, such that fluorescence from the host cell biomass is not detected.

In some embodiments, the method of determining spectral conditions selective for detecting fluorescent dye bound to WIC comprises determining a compatible excitation wavelength. In one embodiment, a compatible excitation wavelength is determined by:
(a) contacting the fluorescent dye with a first plurality of cell populations and a second plurality of cell populations, wherein cells of the first and second plurality are of the same cell type as the WIC-producing cells to be screened, wherein each plurality comprises a cell population having a cell density of x and a cell population having a cell density of 5x, wherein each of the cell populations of the first plurality comprise WIC, and the cell populations of the second plurality do not comprise WIC;
(b) determining an excitation spectrum for the first plurality and the second plurality, respectively; and
(c) selecting an excitation wavelength wherein:
   (i) the difference in fluorescence between a cell population from the first plurality and a cell population from the second plurality having the same cell density is at least 80%; and
   (ii) the difference in fluorescence between cell populations having cell density x and cell density 5x from the second plurality is no greater than 250%.

In some embodiments, the method of determining spectral conditions sufficient to selectively detect fluorescent dye bound to WIC comprises determining a compatible emission wavelength. In one embodiment, a compatible emission wavelength is determined by:
(a) contacting the fluorescent dye with a first plurality of cell populations and a second plurality of cell populations, wherein cells of the first and second plurality are of the same cell type as the WIC-producing cells to be screened, wherein each plurality comprises a cell population having a cell density of x and a cell population having a cell density of 5x, wherein each of the cell populations of the first plurality comprise WIC, and the cell populations of the second plurality do not comprise WIC;
(b) determining an emission spectrum for the first plurality and the second plurality, respectively; and
(c) selecting an emission wavelength wherein:
   (i) the difference in fluorescence between a cell population from the first plurality and a cell population from the second plurality having the same cell density is at least 80%; and
   (ii) the difference in fluorescence between cell populations having cell density x and cell density 5x from the second plurality is no greater than 250%.

In particular embodiments, the method of determining spectral conditions sufficient to selectively detect fluorescent dye bound to WIC comprises selecting both an excitation and emission wavelength, *i.e.,* a compatible emission and excitation wavelength pairing, wherein (i) the difference in fluorescence between a cell population from the first plurality and a cell population from the second plurality having the same optical density is at least 80%; and (ii) the difference in fluorescence between cell populations from the second plurality of OD5 and OD25 is no greater than 250%.

Where the method comprises determining an excitation spectrum for the first and second plurality of cells, the emission wavelength is held constant, and an excitation spectrum is obtained, for example, from 250 nm to 500, or a subset of wavelengths thereof. In some embodiments, the emission wavelength is held constant at a wavelength just outside the range of excitation wavelengths of the excitation spectrum being obtained. In particular embodiments, the emission wavelength is held constant at 550 nm. Similarly, where the method comprises determining an emission spectrum for the first and second plurality of cells, the excitation wavelength is held constant, and an emission spectrum is obtained, for example, from 260 nm to 720, or a subset of wavelengths thereof. In particular embodiments, the excitation wavelength is held constant at 290 nm. Any fluorometer known in the art capable of obtaining fluorescence spectra may be used in the methods described herein.

The first and second pluralities of cell populations useful in the methods described above are preferably contained within a liquid medium that does not contribute an appreciable amount of background fluorescence to the assay. For example, the cells may be added to a well of a microtiter plate in an aqueous solution commonly used in cell culture or cell-based assays, for example, biological buffers, e.g., phosphate buffered saline, or any medium that can support the growth of cells.

In some embodiments, the cell density x of a cell population is the optical density of the cell population at 600 nm (OD₆₀₀). For example, where a first cell population having a cell density x has an OD₆₀₀ of 1, a cell population having a cell density 5x has an OD₆₀₀ of 5. In some embodiments, the first and second pluralities of cells each comprise at least two cell populations of increasing cell density, for example, cell populations of x and 5x (*e.g.,* OD₆₀₀ of 1 and 5), x and 10x (*e.g.,* OD₆₀₀ of 1 and 10), or x and 20x (*e.g.,* OD₆₀₀ of 1 and 20). In some embodiments, the first and second pluralities comprise populations of lower or higher optical densities. For example, the first and second pluralities may further comprise cell populations of OD 1, 2, 3, 4, 6, 7, 8, 9, 10, 11, 12, 13, 14, 15, 16, 17, 18, 19, 20, 21, 22, 23, 24, 30, 35, 40, 45, or higher than 50. In some embodiments, the first and second pluralities comprise at least 3, 4, 5, 6, 7, 8, 9, 10, 11, 12, or more than 12 populations of cells of increasing cell density from which fluorescence spectra are obtained, wherein the pluralities comprise populations of OD₆₀₀ of 5 and OD₆₀₀ of 25. In particular embodiments, the first and second pluralities comprise cell populations of OD₆₀₀ of 5, 10, 15, 20 and 25. In other embodiments, the first and second pluralities of cells comprise populations of OD₆₀₀ of 1 and 10, 1 and 15, 5 and 20, 10 and 20, or 10 and 25. Preferably, cell density x and cell density 5x is within a dynamic range for spectrophotometric detection at 600 nm for a given cell type.

With regard to the water immiscible compound (WIC) for which selective spectral conditions are being sought, for purposes of determining the spectral conditions, the WIC may be added, for example, as a purified compound, to aqueous medium comprising cells of the first plurality. Alternatively, the cells of the first plurality may be recombinant cells modified to produce the WIC. In some embodiments utilizing recombinant cells producing WIC, the amount of WIC produced by the cell is previously established, for example, as a yield (grams of compound per gram of substrate, *e.g.,* sucrose), a level of production (grams per liter) and/or a level of productivity (grams per liter per hour). In some embodiments where the first plurality comprises recombinant cells producing the WIC, the cells are cultured for a period of time sufficient for production of the WIC prior to determining spectral conditions specific for the WIC.

In some embodiments, each of the cell populations of the first plurality comprises the WIC in an equal amount. In other embodiments, the cell populations of the first plurality comprise WIC in differing amounts. Preferably, the amount of WIC is not in excess of the amount of fluorescent dye available to bind the WIC during said contacting. In some embodiments, each of the cell populations of the first plurality comprises WIC in an amount of at least 0.1 g/L. In other embodiments, each of the cell populations of the first plurality comprises WIC in an amount of 0.1 g/L to 10 g/l. In some embodiments, each of the populations of the first plurality comprise WIC in an amount of about 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0 or more than 15.0 g/L. In particular embodiments, the WIC is added to each of the populations of the first plurality as purified WIC, for example, in a solvent that does not contribute an appreciable amount of background fluorescence to the assay. In particular embodiments, WIC is exogenously added to each population of cells of the first plurality at a concentration of at least 2 g/L.

Preferably, the cells of the first and second pluralities are of the same cell type, so as to minimize any differences in the quantity or quality of endogenous cellular targets that may be bound by the fluorescent dye. Preferably, the cells of the second plurality do not comprise WIC, *e.g.,* exogenously added or recombinantly produced WIC. However, where the WIC may be present in the cells of the second plurality as an endogenous molecule, the WIC will also be present in the cells of the first plurality as an endogenous molecule.

In some embodiments, at the excitation and/or emission wavelengths selected for the specific detection of WIC, the difference in fluorescence between a cell population from the first plurality (comprising WIC) and a cell population from the second plurality (not comprising WIC) having the same cell density is at least 80%. In some embodiments, the difference in fluorescence between these cell populations will be at least about 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500 or more than 500%.

In some embodiments, at the excitation and/or emission wavelengths selected for the specific detection of WIC, the difference in fluorescence between cell populations having cell density x and cell density 5x from the second plurality is no greater than 250%. In some embodiments, this difference is no greater than about 240, 230, 220, 210, 200, 190, 180, 170, 160, 150, 140, 130, 120, 110, 100, 90, 80, 70, 60, 50, 40, 20 or 10%.

Further provided herein are methods for determining spectral conditions that are selective for detecting autofluorescence from cells without influence from Nile-Red fluorescence, e.g. fluorescence from Nile Red bound to WIC. Autofluorescence can be used as a proxy for cell biomass, and thus, once spectral conditions that are selective for autofluorescence have been determined, WIC:cell biomass ratios for a given WIC-producing cell population can be obtained using two selective excitation/emission wavelength pairs.

In some embodiments, the method of determining spectral conditions selective for cell autofluorescence comprises:
(a) contacting the fluorescent dye with a first plurality of cell populations and a second plurality of cell populations, wherein cells of the first and second plurality are of the same cell type, wherein each plurality comprises a cell population having a cell density of x and a cell population having a cell density of 5x, wherein each of the cell populations of the first plurality comprise WIC, and the cell populations of the second plurality do not comprise WIC;
(b) determining an excitation spectrum for the first plurality and the second plurality, respectively; and
(c) selecting an excitation wavelength wherein:
   (i) the difference in fluorescence between a cell population from the first plurality and a cell population from the second plurality having the same cell density is no greater than 80%; and
   (ii) the difference in fluorescence between cell populations having cell density x and cell density 5x from the second plurality is at least 250%.

In some embodiments, the method of determining spectral conditions selective for cell autofluorescence comprises:
(a) contacting the fluorescent dye with a first plurality of cell populations and a second plurality of cell populations, wherein cells of the first and second plurality are of the same cell type, wherein each plurality comprises a cell population having a cell density of x and a cell population having a cell density of 5x, wherein each of the cell populations of the first plurality comprise WIC, and the cell populations of the second plurality do not comprise WIC;
(b) determining an emission spectrum for the first plurality and the second plurality, respectively; and
(c) selecting an emission wavelength wherein:
   (i) the difference in fluorescence between a cell population from the first plurality and a cell population from the second plurality having the same cell density is no greater than 80%; and
   (ii) the difference in fluorescence between cell populations having cell density x and cell density 5x from the second plurality is at least 250%.

In particular embodiments, the method of determining spectral conditions selective for cell autofluorescence comprises selecting both an excitation and emission wavelength, *i.e.,* a compatible emission and excitation wavelength pairing, wherein (i) the difference in fluorescence between a cell population from the first plurality and a cell population from the second plurality having the same cell density is no greater than 80%; and (ii) the difference in fluorescence between cell populations having cell density x and cell density 5x from the second plurality is at least 250%.

In some embodiments, at the excitation and/or emission wavelengths selected for the specific detection of cell autofluorescence, the difference in fluorescence between a cell population from the first plurality (comprising WIC) and a cell population from the second plurality (not comprising WIC) having the same cell density is no greater than 80%. In some embodiments, the difference in fluorescence between these cell populations will be no greater than 75, 70, 65, 60, 55, 50, 45, 40, 35, 30, 25, 20, 15 or 10%.

In some embodiments, at the excitation and/or emission wavelengths selected for the specific detection of cell autofluorescence, the difference in fluorescence between cell populations having cell density x and cell density 5x from the second plurality is at least 250%. In some embodiments, this difference is at least 260, 270, 280, 290, 300, 310, 320, 330, 340, 350, 360, 370, 380, 390, 400, 410, 420, 430, 440, 450, 460, 470, 480, 490, 500 or more than 500%.

### 6.2.3 Methods of Screening and Enrichment

In another aspect, provided herein is a method of screening a library of cells for a cell recombinantly producing a water-immiscible compound, comprising encapsulating each cell of the library in a hydrogel particle; detecting the recombinantly produced water-immiscible compound within each hydrogel particle, and selecting a cell producing said recombinantly produced water-immiscible compound. In some embodiments, the method further comprises isolating the cell from the selected hydrogel particle and repeating said steps of encapsulating, detecting and selecting, so that a water-immiscible compound producing cell or clonal population of cells is enriched over successive rounds of selection.

In some embodiments, the methods and compositions provided herein are useful for detecting the production of industrially useful compounds in a microbial cell genetically modified to produce one or more such compounds at greater yield, production, productivity, and/or with increased persistence compared to a parent microbial cell that is not genetically modified, for example, a naïve parental cell producing no amount of the water-immiscible compound natively, or a naïve parental cell that produces native amounts of the water-immiscible compound but no amount heterologously. In other embodiments, the methods are useful for identifying a microbial cell genetically modified to produce one or more such compounds at greater yield, production, productivity, and/or with increased persistence compared to a parental microbial cell that has also been genetically modified. For example, the methods can be used to identify higher producing cells from a population of cells that have all been genetically modified to produce the water-immiscible compound. In some embodiments, all the cells in the population have been genetically modified in the same manner. In other embodiments, the cell population comprises cells that have been genetically modified through different strategies to increase production of the water-immiscible compound. For example, the cell population to be screened may comprise cells that have been modified to comprise varying copy numbers or varying modes of regulation (e.g., varying promoter usage) of one or more components of a metabolic pathway for the compound. The methods provided herein are particularly useful for identifying higher producers from a population of cells that have been genetically modified in an identical fashion to produce the water-immiscible compound, then subjected to mutagenesis. In some such embodiments, the screening methods are used to identify cells harboring one or more mutations that increase the yield, product or productivity of the water-immiscible compound relative to cells not harboring the one or more mutations. Any methods known in the art for producing mutagenized cell populations can be used, such as the use of physicochemical mutagens including, but not limited to, UV irradiation, gamma irradiation, x-rays, restriction enzyme-induced mutagenesis, a mutagenic or teratogenic chemical, a DNA repair mutagen, a DNA repair inhibitor, an error-prone DNA replication protein, N-ethyl-N-nitrosourea (ENU), ethylmethanesulphonate (EMS) and ICR191; or the use of insertional mutagens including, but not limited to, one or more multiple cloning sites, one or more transcription termination sites, one or more transcriptional regulatory sequences, one or more translational signal sequences, one or more open reading frames (ORFs), one or more sequences mutating ORFs, one or more stop codons, one or more sequences mutating or eliminating stop codons, one or more mRNA destabilizing elements, one or more hairpin sequences, one or more sequences mutating or eliminating hairpins, one or more reporter genes, one or more splice acceptor sequences, one or more splice donor sequences, one or more internal ribosome entry sites (IRES), one or more transposon sequences, one or more site-specific recombination site sequences, one or more restriction enzyme sequences, one or more nucleotide sequences encoding a fusion partner protein or peptide, one or more selectable markers or selection modules, one or more bacterial sequences useful for propagating said vector in a host cell, one or more 3' gene traps, one or more 5' gene traps, one or more nucleotide sequences encoding localization signals, one or more origins of replication, one or more protease cleavage sites, one or more desired proteins or peptides encoded by a gene or a portion of a gene, and one or more sequences encoding one or more 5' or 3' polynucleotide tails.

In some embodiments, the methods provide for at least a 2-fold, 3-fold, 4-fold, 5-fold, or greater than 5-fold enrichment in the population for cells producing the water-immiscible compound, for example, cells producing at a higher level relative to other cells in the population, per round of encapsulating, detecting and selecting. In some embodiments, the methods provide for enrichment of a cell or population of cells having a ratio 1:10, 1:100 or 1:1000 in a first population to greater than 1:2, or between 1:2 and 1:1 in an enriched population. In some embodiments, the enrichment occurs over one, two or three rounds of encapsulating, detecting and selecting. In some embodiments, the enrichment occurs within three, four or five rounds of encapsulating, detecting and selecting.

In some embodiments, the method of screening is sufficient to identify a cell or clonal population of cells recombinantly producing one or more water-immiscible compounds expressed as a ratio of WIC to cell biomass. In some embodiments, the cell biomass is determined by a method comprising detecting the autofluorescence of said plurality of cells under spectral conditions wherein fluorescence from the fluorescent dye bound to the WIC is not detected. In some embodiments, the WIC:biomass ratio can be calculated based on the relative fluorescence units (RFU) of the separate yet specific measurements of WIC and biomass, respectively, utilizing select spectral conditions as described herein. In some embodiments, the method of screening is sufficient to identify a cell or clonal population of cells recombinantly producing one or more water-immiscible compounds in a WIC:biomass ratio of about 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95 or 1:100. In some embodiments, the method of screening is sufficient to identify a cell or clonal population of cells recombinantly producing one or more water-immiscible compounds in a WIC:biomass ratio of greater than 100:1 or less than 1:100.

In some embodiments, the method of screening is sufficient to identify a cell or clonal population of cells recombinantly producing one or more water-immiscible compounds in an amount greater than about 10 grams per liter of fermentation medium. In some embodiments, the recombinantly produced water-immiscible compound is produced in an amount from about 10 to about 50 grams, more than about 15 grams, more than about 20 grams, more than about 25 grams, or more than about 30 grams per liter of cell culture.

In some embodiments, the method of screening is sufficient to identify a cell or clonal population of cells recombinantly producing one or more water-immiscible compounds in an amount greater than about 50 milligrams per gram of dry cell weight. In some embodiments, the recombinantly produced water-immiscible compound is produced in an amount from about 50 to about 1500 milligrams, more than about 100 milligrams, more than about 150 milligrams, more than about 200 milligrams, more than about 250 milligrams, more than about 500 milligrams, more than about 750 milligrams, or more than about 1000 milligrams per gram of dry cell weight.

In some embodiments, the method of screening is sufficient to identify a cell or clonal population of cells recombinantly producing one or more water-immiscible compounds in an amount that is at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, at least about 2-fold, at least about 2.5-fold, at least about 5-fold, at least about 10-fold, at least about 20-fold, at least about 30-fold, at least about 40-fold, at least about 50-fold, at least about 75-fold, at least about 100-fold, at least about 200-fold, at least about 300-fold, at least about 400-fold, at least about 500-fold, or at least about 1,000-fold, or more, higher than the amount of the water-immiscible compound produced by a microbial cell that is not genetically modified as described herein, on a per unit volume of cell culture basis. In some embodiments, the method of screening is sufficient to identify a cell or clonal population of cells recombinantly producing one or more water-immiscible compounds in an amount that is at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, at least about 2-fold, at least about 2.5-fold, at least about 5-fold, at least about 10-fold, at least about 20-fold, at least about 30-fold, at least about 40-fold, at least about 50-fold, at least about 75-fold, at least about 100-fold, at least about 200-fold, at least about 300-fold, at least about 400-fold, at least about 500-fold, or at least about 1,000-fold, or more, higher than the amount of the water-immiscible compound produced by a microbial cell that is also genetically modified as described herein, on a per unit volume of cell culture basis.

In some embodiments, the method of screening is sufficient to identify a cell or clonal population of cells recombinantly producing one or more water-immiscible compounds in an amount that is at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, at least about 2-fold, at least about 2.5-fold, at least about 5-fold, at least about 10-fold, at least about 20-fold, at least about 30-fold, at least about 40-fold, at least about 50-fold, at least about 75-fold, at least about 100-fold, at least about 200-fold, at least about 300-fold, at least about 400-fold, at least about 500-fold, or at least about 1,000-fold, or more, higher than the amount of the water-immiscible compound produced by a microbial cell that is not genetically modified according to the methods provided herein, on a per unit dry cell weight basis. In some embodiments, the method of screening is sufficient to identify a cell or clonal population of cells recombinantly producing one or more water-immiscible compounds in an amount that is at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, at least about 2-fold, at least about 2.5-fold, at least about 5-fold, at least about 10-fold, at least about 20-fold, at least about 30-fold, at least about 40-fold, at least about 50-fold, at least about 75-fold, at least about 100-fold, at least about 200-fold, at least about 300-fold, at least about 400-fold, at least about 500-fold, or at least about 1,000-fold, or more, higher than the amount of the water-immiscible compound produced by a microbial cell that is also genetically modified according to the methods provided herein, on a per unit dry cell weight basis.

In some embodiments, the method of screening is sufficient to identify a cell or clonal population of cells recombinantly producing one or more water-immiscible compounds in an amount that is at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, at least about 2-fold, at least about 2.5-fold, at least about 5-fold, at least about 10-fold, at least about 20-fold, at least about 30-fold, at least about 40-fold, at least about 50-fold, at least about 75-fold, at least about 100-fold, at least about 200-fold, at least about 300-fold, at least about 400-fold, at least about 500-fold, or at least about 1,000-fold, or more, higher than the amount of the water-immiscible compound produced by a microbial cell that is not genetically modified according to the methods provided herein, on a per unit volume of cell culture per unit time basis. In some embodiments, the method of screening is sufficient to identify a cell or clonal population of cells recombinantly producing one or more water-immiscible compounds in an amount that is at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, at least about 2-fold, at least about 2.5-fold, at least about 5-fold, at least about 10-fold, at least about 20-fold, at least about 30-fold, at least about 40-fold, at least about 50-fold, at least about 75-fold, at least about 100-fold, at least about 200-fold, at least about 300-fold, at least about 400-fold, at least about 500-fold, or at least about 1,000-fold, or more, higher than the amount of the water-immiscible compound produced by a microbial cell that is also genetically modified according to the methods provided herein, on a per unit volume of cell culture per unit time basis.

In some embodiments, the method of screening is sufficient to identify a cell or clonal population of cells recombinantly producing one or more water-immiscible compounds in an amount that is at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, at least about 2-fold, at least about 2.5-fold, at least about 5-fold, at least about 10-fold, at least about 20-fold, at least about 30-fold, at least about 40-fold, at least about 50-fold, at least about 75-fold, at least about 100-fold, at least about 200-fold, at least about 300-fold, at least about 400-fold, at least about 500-fold, or at least about 1,000-fold, or more, higher than the amount of the water-immiscible compound produced by a microbial cell that is not genetically modified according to the methods provided herein, on a per unit dry cell weight per unit time basis. In some embodiments, the method of screening is sufficient to identify a cell or clonal population of cells recombinantly producing one or more water-immiscible compounds in an amount that is at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, at least about 2-fold, at least about 2.5-fold, at least about 5-fold, at least about 10-fold, at least about 20-fold, at least about 30-fold, at least about 40-fold, at least about 50-fold, at least about 75-fold, at least about 100-fold, at least about 200-fold, at least about 300-fold, at least about 400-fold, at least about 500-fold, or at least about 1,000-fold, or more, higher than the amount of the water-immiscible compound produced by a microbial cell that is also genetically modified according to the methods provided herein, on a per unit dry cell weight per unit time basis.

### 6.2.4 Hydrogel-Encapsulated Cell Compositions

In another aspect, provided herein is a hydrogel-encapsulated cell or clonal cell population comprising one or more recombinantly produced water-immiscible compounds. In another aspect, provided herein is a hydrogel particle comprising a cell or clonal cell population, and further comprising one or more recombinantly produced water-immiscible compounds. Cells useful in the methods and compositions provided herein include any cell capable of naturally or recombinantly producing a water-immiscible compound, *e.g.,* an isoprenoid, a polyketide, a fatty acid, and the like. In some embodiments, the cell is a prokaryotic cell. In some embodiments, the cell is a bacterial cell. In some embodiments, the cell is an *Escherichia coli* cell. In some embodiments, the cell is a eukaryotic cell. In some embodiments, the cell is a mammalian cell. In some embodiments, the cell is a Chinese hamster ovary (CHO) cell, a COS-7 cell, a mouse fibroblast cell, a mouse embryonal carcinoma cell, or a mouse embryonic stem cell. In some embodiments, the cell is an insect cell. In some embodiments, the cell is a S2 cell, a Schneider cell, a S12 cell, a 5B1-4 cell, a Tn5 cell, or a Sf9 cell. In some embodiments, the cell is a unicellular eukaryotic organism cell.

In some embodiments, the cell is a mycelial bacterial cell. In some embodiments, the mycelial bacterial cell is of the class actinomycetes. In particular embodiments, the mycelial bacterial cell is of the genera *Streptomyces,* for example, *Streptomyces ambofaciens, Streptomyces avermitilis, Streptomyces azureus, Streptomyces cinnamonensis*, *Streptomyces coelicolor*, *Streptomyces curacoi, Streptomyces erythraeus*, *Streptomyces fradiae, Streptomyces galilaeus, Streptomyces glaucescens, Streptomyces hygroscopicus, Streptomyces lividans, Streptomyces parvulus, Streptomyces peucetius, Streptomyces rimosus, Streptomyces roseofulvus, Streptomyces thermotolerans, Streptomyces violaceoruber*.

In another embodiment, the cell is a fungal cell. In a more particular embodiment, the cell is a yeast cell. Yeasts useful in the methods and compositions provided herein include yeasts that have been deposited with microorganism depositories (e.g. IFO, ATCC, *etc.)* and belong to the genera *Aciculoconidium, Ambrosiozyma, Arthroascus, Arxiozyma, Ashbya, Babjevia, Bensingtonia, Botryoascus, Botryozyma*, *Brettanomyces*, *Bullera, Bulleromyces, Candida, Citeromyces, Clavispora, Cryptococcus, Cystofilobasidium, Debaryomyces, Dekkara, Dipodascopsis, Dipodascus, Eeniella, Endomycopsella, Eremascus, Eremothecium, Erythrobasidium, Fellomyces, Filobasidium, Galactomyces, Geotrichum, Guilliermondella, Hanseniaspora, Hansenula, Hasegawaea, Holtermannia, Hormoascus, Hyphopichia, Issatchenkia, Kloeckera, Kloeckeraspora, Kluyveromyces, Kondoa, Kuraishia, Kurtzmanomyces, Leucosporidium, Lipomyces, Lodderomyces, Malassezia, Metschnikowia, Mrakia, Myxoryma, Nadsonia, Nakazawaea, Nematospora, Ogataea, Oosporidium, Pachysolen, Phachytichospora, Phaffia, Pichia, Rhodosporidium, Rhodotorula, Saccharomyces, Saccharomycodes, Saccharomycopsis, Saitoella, Sakaguchia, Saturnospora, Schizoblastosporion, Schizosaccharomyces, Schwanniomyces, Sporidiobolus, Sporobolomyces, Sporopachydermia, Stephanoascus, Sterigmatomyces, Sterigmatosporidium, Symbiotaphrina, Sympodiomyces, Sympodiomycopsis, Torulaspora, Trichosporiella, Trichosporon, Trigonopsis, Tsuchiyaea, Udeniomyces, Waltomyces, Wickerhamia, Wickerhamiella, Williopsis, Yamadazyma, Yarrowia, Zygoascus, Zygosaccharomyces, Zygowilliopsis,* and *Zygozyma,* among others.

In particular embodiments, useful yeasts in the methods and compositions provided herein include *Saccharomyces cerevisiae, Pichia pastoris, Schizosaccharomyces pombe, Dekkera bruxellensis, Kluyveromyces lactis* (previously called *Saccharomyces lactis*), *Kluveromyces marxianus, Arxula adeninivorans,* or *Hansenula polymorpha* (now known as *Pichia angusta).* In some embodiments, the microbe is a strain of the genus *Candida*, such as *Candida lipolytica, Candida guilliermondii, Candida krusei, Candida pseudotropicalis,* or *Candida utilis.*

In a particular embodiment, the cell is a *Saccharomyces cerevisiae* cell. In some embodiments, the strain of the *Saccharomyces cerevisiae* cell is selected from the group consisting of Baker's yeast, CBS 7959, CBS 7960, CBS 7961, CBS 7962, CBS 7963, CBS 7964, IZ-1904, TA, BG-1, CR-1, SA-1, M-26, Y-904, PE-2, PE-5, VR-1, BR-1, BR-2, ME-2, VR-2, MA-3, MA-4, CAT-1, CB-1, NR-1, BT-1, and AL-1. In some embodiments, the strain of *Saccharomyces cerevisiae* is selected from the group consisting of PE-2, CAT-1, VR-1, BG-1, CR-1, and SA-1. In a particular embodiment, the strain of *Saccharomyces cerevisiae* is PE-2. In another particular embodiment, the strain of *Saccharomyces cerevisiae* is CAT-1. In another particular embodiment, the strain of *Saccharomyces cerevisiae* is BG-1.

In some embodiments, the cell is a haploid microbial cell. In other embodiments, the cell is a diploid microbial cell. In some embodiments, the cell is heterozygous. In other embodiments, the cell is homozygous other than for its mating type allele (*i.e.,* if the cell should sporulate, the resulting four haploid microbial cells would be genetically identical except for their mating type allele, which in two of the haploid cells would be mating type a and in the other two haploid cells would be mating type alpha).

In some embodiments, the cell is a cell that is suitable for industrial fermentation, e.g., bioethanol fermentation. In particular embodiments, the cell is conditioned to subsist under high solvent concentration, high temperature, expanded substrate utilization, nutrient limitation, osmotic stress due, acidity, sulfite and bacterial contamination, or combinations thereof, which are recognized stress conditions of the industrial fermentation environment.

Exemplary water-immiscible compound producing cells, *e.g.,* cells recombinantly producing isoprenoids, polyketides, and fatty acids, and methods for generating such cells, are provided below.

### 6.2.4.1 Recombinant Cells Producing Isoprenoids

In one aspect, provided herein are methods of detecting isoprenoid production in a cell or a clonal population of cells, *e.g.,* genetically modified to recombinantly produce one or more isoprenoid compounds. Isoprenoids are derived from isopentenyl pyrophosphate (IPP), which can be biosynthesized by enzymes of the mevalonate-dependent ("MEV") pathway or the 1-deoxy-D-xylulose 5-diphosphate ("DXP") pathway. A schematic representation of the MEV pathway is described in Figure 1A, and a schematic representation of the DXP pathway is described in Figure 1B.

### 6.2.4.1.1 MEV Pathway

In some embodiments of the methods of detecting an isoprenoid producing cell provided herein, the isoprenoid producing cell comprises one or more heterologous nucleotide sequences encoding one or more enzymes of the MEV pathway, which effects increased production of one or more isoprenoid compounds as compared to a genetically unmodified parent cell.

In some embodiments, the isoprenoid producing cell comprises a heterologous nucleotide sequence encoding an enzyme that can condense two molecules of acetyl-coenzyme A to form acetoacetyl-CoA, *e.g.,* an acetyl-CoA thiolase. Illustrative examples of nucleotide sequences encoding such an enzyme include, but are not limited to: (NC_000913 REGION: 2324131.2325315; *Escherichia coli*), (D49362; *Paracoccus denitrificans*), and (L20428; *Saccharomyces cerevisiae*).

In some embodiments, the isoprenoid producing cell comprises a heterologous nucleotide sequence encoding an enzyme that can condense acetoacetyl-CoA with another molecule of acetyl-CoA to form 3-hydroxy-3-methylglutaryl-CoA (HMG-CoA), *e*.*g*., a HMG-CoA synthase. Illustrative examples of nucleotide sequences encoding such an enzyme include, but are not limited to: (NC_001145. complement 19061.20536; *Saccharomyces cerevisiae),* (X96617; *Saccharomyces cerevisiae*), (X83882; *Arabidopsis thaliana),* (AB037907; *Kitasatospora griseola),* (BT007302; *Homo sapiens),* and (NC_002758, Locus tag SAV2546, GeneID 1122571; *Staphylococcus aureus*).

In some embodiments, the isoprenoid producing cell comprises a heterologous nucleotide sequence encoding an enzyme that can convert HMG-CoA into mevalonate, *e.g*., a HMG-CoA reductase. Illustrative examples of nucleotide sequences encoding such an enzyme include, but are not limited to: (NM_206548; *Drosophila melanogaster*), (NC_002758, Locus tag SAV2545, GeneID 1122570; *Staphylococcus aureus*), (NM_204485; *Gallus gallus*), (AB015627; *Streptomyces sp.* KO 3988), (AF542543; *Nicotiana attenuata*), (AB037907; *Kitasatospora griseola*), (AX128213, providing the sequence encoding a truncated HMGR; *Saccharomyces cerevisiae),* and (NC_001145: complement (115734.118898; *Saccharomyces cerevisiae*).

In some embodiments, the isoprenoid producing cell comprises a heterologous nucleotide sequence encoding an enzyme that can convert mevalonate into mevalonate 5-phosphate, e.g., a mevalonate kinase. Illustrative examples of nucleotide sequences encoding such an enzyme include, but are not limited to: (L77688; *Arabidopsis thaliana*), and (X55875; *Saccharomyces cerevisiae*).

In some embodiments, the isoprenoid producing cell comprises a heterologous nucleotide sequence encoding an enzyme that can convert mevalonate 5-phosphate into mevalonate 5-pyrophosphate, *e.g*., a phosphomevalonate kinase. Illustrative examples of nucleotide sequences encoding such an enzyme include, but are not limited to: (AF429385; *Hevea brasiliensis*), (NM_006556; *Homo sapiens*), and (NC_001145. complement 712315.713670; *Saccharomyces cerevisiae*).

In some embodiments, the isoprenoid producing cell comprises a heterologous nucleotide sequence encoding an enzyme that can convert mevalonate 5-pyrophosphate into IPP, *e.g.,* a mevalonate pyrophosphate decarboxylase. Illustrative examples of nucleotide sequences encoding such an enzyme include, but are not limited to: (X97557; *Saccharomyces cerevisiae*), (AF290095; *Enterococcus faecium*), and (U49260; *Homo sapiens*).

In some embodiments, the isoprenoid producing cell comprises one or more heterologous nucleotide sequences encoding more than one enzyme of the MEV pathway. In some embodiments, the isoprenoid producing cell comprises one or more heterologous nucleotide sequences encoding two enzymes of the MEV pathway. In some embodiments, the isoprenoid producing cell comprises one or more heterologous nucleotide sequences encoding an enzyme that can convert HMG-CoA into mevalonate and an enzyme that can convert mevalonate into mevalonate 5-phosphate. In some embodiments, the isoprenoid producing cell comprises one or more heterologous nucleotide sequences encoding three enzymes of the MEV pathway. In some embodiments, the isoprenoid producing cell comprises one or more heterologous nucleotide sequences encoding four enzymes of the MEV pathway. In some embodiments, the isoprenoid producing cell comprises one or more heterologous nucleotide sequences encoding five enzymes of the MEV pathway. In some embodiments, the isoprenoid producing cell comprises one or more heterologous nucleotide sequences encoding six enzymes of the MEV pathway.

In some embodiments, the isoprenoid producing cell further comprises a heterologous nucleotide sequence encoding an enzyme that can convert IPP generated via the MEV pathway into its isomer, dimethylallyl pyrophosphate ("DMAPP"). DMAPP can be condensed and modified through the action of various additional enzymes to form simple and more complex isoprenoids (Figure 2).

### 6.2.4.1.2 DXP Pathway

In some embodiments of the methods of detecting an isoprenoid producing cell provided herein, the isoprenoid producing cell comprises one or more heterologous nucleotide sequences encoding one or more enzymes of the DXP pathway, which effects increased production of one or more isoprenoid compounds as compared to a genetically unmodified parent cell.

In some embodiments, the isoprenoid producing cell comprises a heterologous nucleotide sequence encoding an enzyme that can condense two molecules of acetyl-coenzyme A to form acetoacetyl-CoA, *e.g.,* an acetyl-CoA thiolase. Illustrative examples of nucleotide sequences encoding such an enzyme include, but are not limited to: (NC_000913 REGION: 2324131.2325315; *Escherichia coli*), (D49362; *Paracoccus denitrificans*), and (L20428; *Saccharomyces cerevisiae*).

In some embodiments, the isoprenoid producing cell comprises a heterologous nucleotide sequence encoding an enzyme, *e.g.,* 1-deoxy-D-xylulose-5-phosphate synthase, which can condense pyruvate with D-glyceraldehyde 3-phosphate to make 1-deoxy-D-xylulose-5-phosphate. Illustrative examples of nucleotide sequences encoding such an enzyme include but are not limited to: (AF035440; *Escherichia coli*), (NC_002947, locus tag PP0527; *Pseudomonas putida* KT2440), (CP000026, locus tag SPA2301; *Salmonella enterica Paratyphi,* see ATCC 9150), (NC_007493, locus tag RSP_0254; *Rhodobacter sphaeroides* 2.4.1), (NC_005296, locus tag RPA0952; *Rhodopseudomonas palustris* CGA009), (NC_004556, locus tag PD1293; *Xylella fastidiosa Temecula1),* and (NC_003076, locus tag AT5G11380; *Arabidopsis thaliana).*

In some embodiments, the isoprenoid producing cell comprises a heterologous nucleotide sequence encoding an enzyme, *e.g.,* 1-deoxy-D-xylulose-5-phosphate reductoisomerase, which can convert 1-deoxy-D-xylulose-5-phosphate to 2C-methyl-D-erythritol-4-phosphate. Illustrative examples of nucleotide sequences include but are not limited to: (AB013300; *Escherichia coli),* (AF148852; *Arabidopsis thaliana),* (NC_002947, locus tag PP1597; *Pseudomonas putida* KT2440), (AL939124, locus tag SCO5694; *Streptomyces coelicolor* A3(2)), (NC_007493, locus tag RSP_2709; *Rhodobacter sphaeroides* 2.4.1), and (NC_007492, locus tag Pfl_1107; *Pseudomonas fluorescens* PfO-1).

In some embodiments, the isoprenoid producing cell comprises a heterologous nucleotide sequence encoding an enzyme, *e.g.,* 4-diphosphocytidyl-2C-methyl-D-erythritol synthase, which can convert 2C-methyl-D-erythritol-4-phosphate to 4-diphosphocytidyl-2C-methyl-D-erythritol. Illustrative examples of nucleotide sequences include but are not limited to: (AF230736; *Escherichia coli),* (NC_007493, locus tag RSP_2835; *Rhodobacter sphaeroides* 2.4.1), (NC_003071, locus tag AT2G02500; *Arabidopsis thaliana),* and (NC_002947, locus tag PP1614; *Pseudomonas putida* KT2440).

In some embodiments, the isoprenoid producing cell comprises a heterologous nucleotide sequence encoding an enzyme, *e.g*., 4-diphosphocytidyl-2C-methyl-D-erythritol kinase, which can convert 4-diphosphocytidyl-2C-methyl-D-erythritol to 4-diphosphocytidyl-2C-methyl-D-erythritol-2-phosphate. Illustrative examples of nucleotide sequences include but are not limited to: (AF216300; *Escherichia coli)* and (NC_007493, locus tag RSP_1779; Rhodobacter sphaeroides 2.4.1).

In some embodiments, the isoprenoid producing cell comprises a heterologous nucleotide sequence encoding an enzyme, 2C-methyl-D-erythritol 2,4-cyclodiphosphate synthase, which can convert 4-diphosphocytidyl-2C-methyl-D-erythritol-2-phosphate to 2C-methyl-D-erythritol 2,4-cyclodiphosphate. Illustrative examples of nucleotide sequences include but are not limited to: (AF230738; *Escherichia coli*), (NC_007493, locus tag RSP_6071; *Rhodobacter sphaeroides* 2.4.1), and (NC_002947, locus tag PP1618; *Pseudomonas putida* KT2440).

In some embodiments, the isoprenoid producing cell comprises a heterologous nucleotide sequence encoding an enzyme, *e.g*., 1-hydroxy-2-methyl-2-(E)-butenyl-4-diphosphate synthase, which can convert 2C-methyl-D-erythritol 2,4-cyclodiphosphate to 1-hydroxy-2-methyl-2-(E)-butenyl-4-diphosphate. Illustrative examples of nucleotide sequences include but are not limited to: (AY033515; *Escherichia coli*), (NC_002947, locus tag PP0853; *Pseudomonas putida* KT2440), and (NC_007493, locus tag RSP_2982; *Rhodobacter sphaeroides* 2.4.1).

In some embodiments, the isoprenoid producing cell comprises a heterologous nucleotide sequence encoding an enzyme, *e.g*., isopentyl/dimethylallyl diphosphate synthase, which can convert 1-hydroxy-2-methyl-2-(E)-butenyl-4-diphosphate into either IPP or its isomer, DMAPP. Illustrative examples of nucleotide sequences include but are not limited to: (AY062212; *Escherichia coli)* and (NC_002947, locus tag PP0606; *Pseudomonas putida* KT2440).

In some embodiments, the isoprenoid producing cell comprises one or more heterologous nucleotide sequences encoding more than one enzyme of the DXP pathway. In some embodiments, the isoprenoid producing cell comprises one or more heterologous nucleotide sequences encoding two enzymes of the DXP pathway. In some embodiments, the isoprenoid producing cell comprises one or more heterologous nucleotide sequences encoding three enzymes of the DXP pathway. In some embodiments, the isoprenoid producing cell comprises one or more heterologous nucleotide sequences encoding four enzymes of the DXP pathway. In some embodiments, the isoprenoid producing cell comprises one or more heterologous nucleotide sequences encoding five enzymes of the DXP pathway. In some embodiments, the isoprenoid producing cell comprises one or more heterologous nucleotide sequences encoding six enzymes of the DXP pathway. In some embodiments, the isoprenoid producing cell comprises one or more heterologous nucleotide sequences encoding five enzymes of the DXP pathway. In some embodiments, the isoprenoid producing cell comprises one or more heterologous nucleotide sequences encoding seven enzymes of the DXP pathway.

In some embodiments, "cross talk" (or interference) between the host cell's own metabolic processes and those processes involved with the production of IPP are minimized or eliminated entirely. For example, cross talk is minimized or eliminated entirely when the host microorganism relies exclusively on the DXP pathway for synthesizing IPP, and a MEV pathway is introduced to provide additional IPP. Such a host organism would not be equipped to alter the expression of the MEV pathway enzymes or process the intermediates associated with the MEV pathway. Organisms that rely exclusively or predominately on the DXP pathway include, for example, *Escherichia coli.*

In some embodiments, the host cell produces IPP via the MEV pathway, either exclusively or in combination with the DXP pathway. In other embodiments, a host's DXP pathway is functionally disabled so that the host cell produces IPP exclusively through a heterologously introduced MEV pathway. The DXP pathway can be functionally disabled by disabling gene expression or inactivating the function of one or more of the DXP pathway enzymes.

In some embodiments, the isoprenoid produced by the cell is a C₅ isoprenoid. These compounds are derived from one isoprene unit and are also called hemiterpenes. An illustrative example of a hemiterpene is isoprene. In other embodiments, the isoprenoid is a C₁₀ isoprenoid. These compounds are derived from two isoprene units and are also called monoterpenes. Illustrative examples of monoterpenes are limonene, citranellol, geraniol, menthol, perillyl alcohol, linalool, thujone, and myrcene. In other embodiments, the isoprenoid is a C₁₅ isoprenoid. These compounds are derived from three isoprene units and are also called sesquiterpenes. Illustrative examples of sesquiterpenes are periplanone B, gingkolide B, amorphadiene, artemisinin, artemisinic acid, valencene, nootkatone, epi-cedrol, epi-aristolochene, farnesol, gossypol, sanonin, periplanone, forskolin, and patchoulol (which is also known as patchouli alcohol). In other embodiments, the isoprenoid is a C₂₀ isoprenoid. These compounds are derived from four isoprene units and also called diterpenes. Illustrative examples of diterpenes are casbene, eleutherobin, paclitaxel, prostratin, pseudopterosin, and taxadiene. In yet other examples, the isoprenoid is a C₂₀₊ isoprenoid. These compounds are derived from more than four isoprene units and include: triterpenes (C₃₀ isoprenoid compounds derived from 6 isoprene units) such as arbrusideE, bruceantin, testosterone, progesterone, cortisone, digitoxin, and squalene; tetraterpenes (C₄₀ isoprenoid compounds derived from 8 isoprenoids) such as β-carotene; and polyterpenes (C₄₀₊ isoprenoid compounds derived from more than 8 isoprene units) such as polyisoprene. In some embodiments, the isoprenoid is selected from the group consisting of abietadiene, amorphadiene, carene, α-farnesene, β-farnesene, farnesol, geraniol, geranylgeraniol, isoprene, linalool, limonene, myrcene, nerolidol, ocimene, patchoulol, β-pinene, sabinene, γ-terpinene, terpinolene and valencene. Isoprenoid compounds also include, but are not limited to, carotenoids (such as lycopene, α- and β-carotene, α- and β-cryptoxanthin, bixin, zeaxanthin, astaxanthin, and lutein), steroid compounds, and compounds that are composed of isoprenoids modified by other chemical groups, such as mixed terpene-alkaloids, and coenzyme Q-10.

In some embodiments, the isoprenoid producing cell further comprises a heterologous nucleotide sequence encoding an enzyme that can convert IPP generated via the MEV pathway into DMAPP, *e.g.,* an IPP isomerase. Illustrative examples of nucleotide sequences encoding such an enzyme include, but are not limited to: (NC_000913, 3031087.3031635; *Escherichia coli),* and (AF082326; *Haematococcus pluvialis*)*.*

In some embodiments, the isoprenoid producing cell further comprises a heterologous nucleotide sequence encoding a polyprenyl synthase that can condense IPP and/or DMAPP molecules to form polyprenyl compounds containing more than five carbons.

In some embodiments, the isoprenoid producing cell comprises a heterologous nucleotide sequence encoding an enzyme that can condense one molecule of IPP with one molecule of DMAPP to form one molecule of geranyl pyrophosphate ("GPP"), *e.g.,* a GPP synthase. Illustrative examples of nucleotide sequences encoding such an enzyme include, but are not limited to: (AF513111; *Abies grandis),* (AF513112; *Abies grandis),* (AF513113; *Abies grandis),* (AY534686; *Antirrhinum majus),* (AY534687; *Antirrhinum majus),* (Y17376; *Arabidopsis thaliana),* (AE016877, Locus AP11092; *Bacillus cereus*; ATCC 14579), (AJ243739; *Citrus sinensis),* (AY534745; *Clarkia breweri),* (AY953508; *Ips pini*), (DQ286930; *Lycopersicon esculentum*), (AF182828; *Mentha x piperita*), (AF182827; *Mentha x piperita*), (MPI249453; Mentha x piperita), (PZE431697, Locus CAD24425; *Paracoccus zeaxanthinifaciens*), (AY866498; *Picrorhiza kurrooa*), (AY351862; *Vitis vinifera*), and (AF203881, Locus AAF12843; *Zymomonas mobilis*).

In some embodiments, the isoprenoid producing cell comprises a heterologous nucleotide sequence encoding an enzyme that can condense two molecules of IPP with one molecule of DMAPP, or add a molecule of IPP to a molecule of GPP, to form a molecule of farnesyl pyrophosphate ("FPP"), *e.g.,* a FPP synthase. Illustrative examples of nucleotide sequences that encode such an enzyme include, but are not limited to: (ATU80605; *Arabidopsis thaliana),* (ATHFPS2R; *Arabidopsis thaliana),* (AAU36376; *Artemisia annua*), (AF461050; *Bos taurus*), (D00694; *Escherichia coli* K-12), (AE009951, Locus AAL95523; *Fusobacterium nucleatum subsp. nucleatum* ATCC 25586), (GFFPPSGEN; *Gibberella fujikuroi*), (CP000009, Locus AAW60034; *Gluconobacter oxydans* 621 H), (AF019892; *Helianthus annuus*), (HUMFAPS; *Homo sapiens*), (KLPFPSQCR; *Kluyveromyces lactis*), (LAU15777; *Lupinus albus*), (LAU20771; *Lupinus albus*), (AF309508; *Mus musculus*), (NCFPPSGEN; *Neurospora crassa*), (PAFPS1; *Parthenium argentatum*), (PAFPS2; *Parthenium argentatum*), (RATFAPS; *Rattus norvegicus*), (YSCFPP; *Saccharomyces cerevisiae*), (D89104; *Schizosaccharomyces pombe*), (CP000003, Locus AAT87386; *Streptococcus pyogenes*), (CP000017, Locus AAZ51849; *Streptococcus pyogenes*), (NC_008022, Locus YP_598856; *Streptococcus pyogenes* MGAS10270), (NC_008023, Locus YP_600845; *Streptococcus pyogenes* MGAS2096), (NC_008024, Locus YP_602832; *Streptococcus pyogenes* MGAS10750), (MZEFPS; *Zea mays),* (AE000657, Locus AAC06913; *Aquifex aeolicus* VF5), (NM_202836; *Arabidopsis thaliana),* (D84432, Locus BAA12575; *Bacillus subtilis*), (U12678, Locus AAC28894; *Bradyrhizobium japonicum* USDA 110), (BACFDPS; *Geobacillus stearothermophilus*), (NC_002940, Locus NP_873754; *Haemophilus ducreyi* 35000HP), (L42023, Locus AAC23087; *Haemophilus influenzae* Rd KW20), (J05262; *Homo sapiens*), (YP_395294; *Lactobacillus sakei subsp. sakei* 23K), (NC_005823, Locus YP_000273; *Leptospira interrogans serovar Copenhageni str. Fiocruz* L1-130), (AB003187; *Micrococcus luteus*), (NC_002946, Locus YP_208768; *Neisseria gonorrhoeae* FA 1090), (U00090, Locus AAB91752; *Rhizobium sp.* NGR234), (J05091; *Saccharomyces cerevisae*), (CP000031, Locus AAV93568; *Silicibacter pomeroyi* DSS-3), (AE008481, Locus AAK99890; *Streptococcus pneumoniae* R6), and (NC_004556, Locus NP 779706; *Xylella fastidiosa* Temecula1).

In some embodiments, the isoprenoid producing cell further comprises a heterologous nucleotide sequence encoding an enzyme that can combine IPP and DMAPP or IPP and FPP to form geranylgeranyl pyrophosphate ("GGPP"). Illustrative examples of nucleotide sequences that encode such an enzyme include, but are not limited to: (ATHGERPYRS; *Arabidopsis thaliana),* (BT005328; *Arabidopsis thaliana),* (NM_119845; *Arabidopsis thaliana*), (NZ_AAJM01000380, Locus ZP_00743052; *Bacillus thuringiensis serovar israelensis,* ATCC 35646 sq1563), (CRGGPPS; *Catharanthus roseus*), (NZ_AABF02000074, Locus ZP_00144509; *Fusobacterium nucleatum subsp. vincentii,* ATCC 49256), (GFGGPPSGN; *Gibberella fujikuroi*)*,* (AY371321; *Ginkgo biloba*), (AB055496; *Hevea brasiliensis*), (AB017971; *Homo sapiens*), (MCI276129; *Mucor circinelloides f. lusitanicus*), (AB016044; *Mus musculus*), (AABX01000298, Locus NCU01427; *Neurospora crassa*), (NCU20940; *Neurospora crassa*), (NZ_AAKL01000008, Locus ZP_00943566; *Ralstonia solanacearum* UV551), (AB118238; *Rattus norvegicus*), (SCU31632; *Saccharomyces cerevisiae*), (AB016095; Synechococcus elongates), (SAGGPS; *Sinapis alba*), (SSOGDS; *Sulfolobus acidocaldarius*), (NC_007759, Locus YP_461832; *Syntrophus aciditrophicus* SB), (NC_006840, Locus YP_204095; *Vibrio fischeri* ES114), (NM_112315; *Arabidopsis thaliana),* (ERWCRTE; *Pantoea agglomerans*), (D90087, Locus BAA14124; *Pantoea ananatis*), (X52291, Locus CAA36538; *Rhodobacter capsulatus*), (AF195122, Locus AAF24294; *Rhodobacter sphaeroides*), and (NC_004350, Locus NP_721015; *Streptococcus mutans* UA159).

In some embodiments, the isoprenoid producing cell further comprises a heterologous nucleotide sequence encoding an enzyme that can modify a polyprenyl to form a hemiterpene, a monoterpene, a sesquiterpene, a diterpene, a triterpene, a tetraterpene, a polyterpene, a steroid compound, a carotenoid, or a modified isoprenoid compound.

In some embodiments, the heterologous nucleotide encodes a carene synthase. Illustrative examples of suitable nucleotide sequences include, but are not limited to: (AF461460, REGION 43.1926; *Picea abies*) and (AF527416, REGION: 78.1871; *Salvia stenophylla*).

In some embodiments, the heterologous nucleotide encodes a geraniol synthase. Illustrative examples of suitable nucleotide sequences include, but are not limited to: (AJ457070; *Cinnamomum tenuipilum*), (AY362553; *Ocimum basilicum*), (DQ234300; *Perilla frutescens* strain 1864), (DQ234299; *Perilla citriodora* strain 1861), (DQ234298; *Perilla citriodora* strain 4935), and (DQ088667; *Perilla citriodora*).

In some embodiments, the heterologous nucleotide encodes a linalool synthase. Illustrative examples of a suitable nucleotide sequence include, but are not limited to: (AF497485; *Arabidopsis thaliana),* (AC002294, Locus AAB71482; *Arabidopsis thaliana*), (AY059757; *Arabidopsis thaliana*), (NM_104793 *Arabidopsis thaliana*), (AF154124; *Artemisia annua*), (AF067603; *Clarkia breweri),* (AF067602; *Clarkia concinna*), (AF067601; *Clarkia breweri),* (U58314; *Clarkia breweri),* (AY840091; *Lycopersicon esculentum*), (DQ263741; *Lavandula angustifolia*), (AY083653; *Mentha citrate*), (AY693647; *Ocimum basilicum*), (XM_463918; *Oryza sativa*), (AP004078, Locus BAD07605; *Oryza sativa*), (XM_463918, Locus XP_463918; *Oryza sativa*), (AY917193; *Perilla citriodora*), (AF271259; *Perilla frutescens*), (AY473623; *Picea abies),* (DQ195274; *Picea sitchensis*), and (AF444798; *Perilla frutescens* var. crispa cultivar No. 79).

In some embodiments, the heterologous nucleotide encodes a limonene synthase. Illustrative examples of suitable nucleotide sequences include, but are not limited to: (+)-limonene synthases (AF514287, REGION: 47.1867; *Citrus limon*) and (AY055214, REGION: 48.1889; *Agastache rugosa*) and (-)-limonene synthases (DQ195275, REGION: 1.1905; *Picea sitchensis*), (AF006193, REGION: 73.1986; *Abies grandis),* and (MHC4SLSP, REGION: 29:1828; *Mentha spicata*).

In some embodiments, the heterologous nucleotide encodes a myrcene synthase. Illustrative examples of suitable nucleotide sequences include, but are not limited to: (U87908; *Abies grandis),* (AY 195609; *Antirrhinum majus),* (AY 195608; *Antirrhinum majus*), (NM_127982; *Arabidopsis thaliana* TPS10), (NM_113485; *Arabidopsis thaliana* ATTPS-CIN), (NM_113483; *Arabidopsis thaliana* ATTPS-CIN), (AF271259; *Perilla frutescens*), (AY473626; *Picea abies),* (AF369919; *Picea abies),* and (AJ304839; *Quercus ilex*).

In some embodiments, the heterologous nucleotide encodes an ocimene synthase. Illustrative examples of suitable nucleotide sequences include, but are not limited to: (AY195607; *Antirrhinum majus),* (AY195609; *Antirrhinum majus),* (AY195608; *Antirrhinum majus),* (AK221024; *Arabidopsis thaliana),* (NM_113485; *Arabidopsis thaliana* ATTPS-CIN), (NM_13483; *Arabidopsis thaliana* ATTPS-CIN), (NM_117775; *Arabidopsis thaliana* ATTPS03), (NM_001036574; *Arabidopsis thaliana* ATTPS03), (NM_127982; *Arabidopsis thaliana* TPS10), (AB110642; *Citrus unshiu* CitMTSL4), and (AY575970; *Lotus corniculatus var. japonicus*).

In some embodiments, the heterologous nucleotide encodes an α-pinene synthase. Illustrative examples of suitable nucleotide sequences include, but are not limited to: (+) α-pinene synthase (AF543530, REGION: 1.1887; *Pinus taeda*), (-)α-pinene synthase (AF543527, REGION: 32.1921; *Pinus taeda*), and (+)/(-)α-pinene synthase (AGU87909, REGION: 6111892; *Abies grandis*).

In some embodiments, the heterologous nucleotide encodes a β-pinene synthase. Illustrative examples of suitable nucleotide sequences include, but are not limited to: (-) β-pinene synthases (AF276072, REGION: 1.1749; *Artemisia annua*) and (AF514288, REGION: 26.1834; *Citrus limon*).

In some embodiments, the heterologous nucleotide encodes a sabinene synthase. An illustrative example of a suitable nucleotide sequence includes but is not limited to AF051901, REGION: 26.1798 from *Salvia officinalis.*

In some embodiments, the heterologous nucleotide encodes a γ-terpinene synthase. Illustrative examples of suitable nucleotide sequences include, but are not limited to: (AF514286, REGION: 30.1832 from *Citrus limon*) and (AB110640, REGION 1.1803 from *Citrus unshiu*).

In some embodiments, the heterologous nucleotide encodes a terpinolene synthase. Illustrative examples of a suitable nucleotide sequence include but are not limited to: (AY693650 from *Oscimum basilicum*) and (AY906866, REGION: 10.1887 from *Pseudotsuga menziesii*).

In some embodiments, the heterologous nucleotide encodes an amorphadiene synthase. An illustrative example of a suitable nucleotide sequence is SEQ ID NO. 37 of U.S. Patent Publication No. 2004/0005678.

In some embodiments, the heterologous nucleotide encodes a α-farnesene synthase. Illustrative examples of suitable nucleotide sequences include, but are not limited to DQ309034 from *Pyrus communis cultivar d'Anjou* (pear; gene name AFS1) and AY 182241 from *Malus domestica* (apple; gene AFS1). Pechouus et al., Planta 219(1):84-94 (2004).

In some embodiments, the heterologous nucleotide encodes a β-farnesene synthase. Illustrative examples of suitable nucleotide sequences include, but are not limited to GenBank accession number AF024615 from *Mentha x piperita* (peppermint; gene Tspa11), and AY835398 from *Artemisia annua.* Picaud et al., Phytochemistry 66(9): 961-967 (2005).

In some embodiments, the heterologous nucleotide encodes a farnesol synthase. Illustrative examples of suitable nucleotide sequences include, but are not limited to GenBank accession number AF529266 from *Zea mays* and YDR481C from *Saccharomyces cerevisiae* (gene Pho8). Song, L., Applied Biochemistry and Biotechnology 128:149-158 (2006).

In some embodiments, the heterologous nucleotide encodes a nerolidol synthase. An illustrative example of a suitable nucleotide sequence includes, but is not limited to AF529266 from *Zea mays* (maize; gene tps1).

In some embodiments, the heterologous nucleotide encodes a patchouliol synthase. Illustrative examples of suitable nucleotide sequences include, but are not limited to AY508730 REGION: 1.1659 from *Pogostemon cablin.*

In some embodiments, the heterologous nucleotide encodes a nootkatone synthase. Illustrative examples of a suitable nucleotide sequence include, but are not limited to AF441124 REGION: 1.1647 from *Citrus sinensis* and AY917195 REGION: 1.1653 from *Perilla frutescens.*

In some embodiments, the heterologous nucleotide encodes an abietadiene synthase. Illustrative examples of suitable nucleotide sequences include, but are not limited to: (U50768; *Abies grandis)* and (AY473621; *Picea abies).*

### 6.2.4.2 Recombinant Cells Producing Polyketides

In another aspect, provided herein are methods of detecting polyketide production in a cell or a clonal population of cells, *e.g*., genetically modified to recombinantly produce one or more polyketide compounds. Polyketide synthesis is mediated by polyketide synthases (PKSs), which are multifunctional enzymes related to fatty acid synthases (FASs). PKSs catalyze the biosynthesis of polyketides through repeated (decarboxylative) Claisen condensations between acylthioesters, usually acetyl, propionyl, malonyl or methylmalonyl. Following each condensation, PKSs introduce structural variability into the polyketide product by catalyzing all, part, or none of a reductive cycle comprising a ketoreduction, dehydration, and enoylreduction on the β-keto group of the growing polyketide chain.

In some embodiments of the methods of detecting a polyketide producing cell provided herein, the polyketide producing cell comprises one or more heterologous nucleotide sequences encoding a PKS system, *i.e*., one or more PKSs capable of catalyzing the synthesis of a polyketide, to effect increased production of one or more polyketide compounds as compared to a genetically unmodified parent cell.

There are two major classes of polyketide synthases (PKSs): the aromatic PKS and the modular PKS, respectively, which differ in the manner in which the catalytic sites are used. For the aromatic PKS, a minimal system, *i.e.,* the minimal components needed to catalyze the production of a polyketide, comprises a ketosynthase/acyl transferase (KS/AT) catalytic region, a chain length factor (CLF) catalytic region and an acyl carrier protein (ACP) activity. For the modular PKS system, a minimal system comprises a KS catalytic region, an AT catalytic region, and an ACP activity, provided that intermediates in the synthesis are provided as substrates. Where *de novo* polyketide synthesis is to be required, a minimal modular PKS system further comprises a loading acyl transferase, which includes additional AT and ACP regions.

Thus, in some embodiments, the polyketide producing cell comprises one or more heterologous nucleotide sequences encoding an enzyme comprising a KS catalytic region. In some embodiments, the polyketide producing cell comprises one or more heterologous nucleotide sequences encoding an enzyme comprising an AT catalytic region. In some embodiments, the polyketide producing cell comprises more than one heterologous nucleotide sequence encoding an enzyme comprising an AT catalytic region. In some embodiments, the polyketide producing cell comprises one or more heterologous nucleotide sequences encoding an enzyme comprising a CLF catalytic region. In some embodiments, the polyketide producing cell comprises one or more heterologous nucleotide sequences encoding an enzyme comprising an ACP activity. In some embodiments, the polyketide producing cell comprises more than one heterologous nucleotide sequence encoding an enzyme comprising an ACP activity.

In a particular embodiment, the polyketide producing cell comprises a minimal aromatic PKS system, *e.g*., heterologous nucleotide sequences encoding an enzyme comprising a KS catalytic region, an enzyme comprising an AT catalytic region, an enzyme comprising a CLF catalytic region, and an enzyme comprising an ACP activity, respectively. In a particular embodiment, the polyketide producing cell comprises a minimal modular PKS system, *e.g.,* heterologous nucleotide sequences encoding an enzyme comprising a KS catalytic region, an enzyme comprising an AT catalytic region, and an enzyme comprising an ACP activity, respectively. In yet another particular embodiment, the polyketide producing cell comprises a modular aromatic PKS system for *de novo* polyketide synthesis, *e.g.,* heterologous nucleotide sequences encoding an enzyme comprising a KS catalytic region, one or more enzymes comprising an AT catalytic region, and one or more enzymes comprising an ACP activity, respectively.

In some embodiments, the polyketide producing cell comprising a minimal PKS system, *e.g*., a minimal aromatic PKS system or minimal modular PKS system, as described above, further comprises additional catalytic activities which can contribute to production of the end-product polyketide. In some embodiments, the polyketide producing cell comprises one or more heterologous nucleotide sequences encoding an enzyme comprising a cyclase (CYC) catalytic region, which facilitates the cyclization of the nascent polyketide backbone. In some embodiments, the polyketide producing cell comprises one or more heterologous nucleotide sequences encoding an enzyme comprising a ketoreductase (KR) catalytic region. In some embodiments, the polyketide producing cell comprises one or more heterologous nucleotide sequences encoding an enzyme comprising an aromatase (ARO) catalytic region. In some embodiments, the polyketide producing cell comprises one or more heterologous nucleotide sequences encoding an enzyme comprising an enoylreductase (ER) catalytic region. In some embodiments, the polyketide producing cell comprises one or more heterologous nucleotide sequences encoding an enzyme comprising a thioesterase (TE) catalytic region. In some embodiments, the polyketide producing cell further comprises one or more heterologous nucleotide sequences encoding an enzyme comprising a holo ACP synthase activity, which effects pantetheinylation of the ACP.

In some embodiments, the polyketide producing cell further comprises one or more heterologous nucleotide sequences conferring a postsynthesis polyketide modifying activity. In some embodiments, the polyketide producing cell further comprises one or more heterologous nucleotide sequences encoding an enzyme comprising a glycosylase activity, which effects postsynthesis modifications of polyketides, for example, where polyketides having antibiotic activity are desired. In some embodiments, the polyketide producing cell further comprises one or more heterologous nucleotide sequences encoding an enzyme comprising a hydroxylase activity. In some embodiments, the polyketide producing cell further comprises one or more heterologous nucleotide sequences encoding an enzyme comprising an epoxidase activity. In some embodiments, the polyketide producing cell further comprises one or more heterologous nucleotide sequences encoding an enzyme comprising a methylase activity.

In some embodiments, the polyketide producing cell comprises heterologous nucleotide sequences, for example sequences encoding PKS enzymes and polyketide modification enzymes, capable of producing a polyketide selected from, but not limited to, the following polyketides: Avermectin (*see, e.g.,* U.S. Pat. No. 5,252,474; U.S. Pat. No. 4,703,009; EP Pub. No. 118,367; MacNeil et al., 1993, "Industrial Microorganisms: Basic and Applied Molecular Genetics"; Baltz, Hegeman, & Skatrud, eds. (ASM), pp. 245-256, "A Comparison of the Genes Encoding the Polyketide Synthases for Avermectin, Erythromycin, and Nemadectin"; MacNeil et al., 1992, Gene 115:-119-125; and Ikeda and Omura, 1997, Chem. Res. 97: 2599-2609); Candicidin (FR008) (*see*; *e.g.,* Hu et al., 1994, Mol. Microbiol. 14: 163-172); Carbomycin, Curamycin (*see, e.g.,* Bergh et al., Biotechnol Appl Biochem. 1992 Feb;15(1):80-9); Daunorubicin (*see, e.g.,* J Bacteriol. 1994 Oct;176(20):6270-80); Epothilone *(see, e.g.,* PCT Pub. No. 99/66028; and PCT Pub. No. 00/031247); Erythromycin (*see, e.g.,* PCT Pub. No. 93/13663; U.S. Pat. No. 6,004,787; U.S. Pat. No. 5,824,513; Donadio et al., 1991, Science 252:675-9; and Cortes et al., Nov. 8, 1990, Nature 348:176-8); FK-506 (*see, e.g.,* Motamedi et al., 1998; Eur. J Biochem. 256: 528-534; and Motamedi et al., 1997, Eur. JBiochem. 244: 74-80); FK-520 *(see, e.g.,* PCT Pub. No. 00/020601; and Nielsen et al., 1991, Biochem. 30:5789-96); Griseusin (*see, e.g.,* Yu et al., J Bacteriol. 1994 May;176(9):2627-34); Lovastatin *(see, e.g.,* U.S. Pat. No. 5,744,350); Frenolycin (*see, e.g.,* Khosla et al., Bacteriol. 1993 Apr;175(8):2197-204; and Bibb et al., Gene 1994 May 3;142(1):31-9); Granaticin *(see, e.g.,* Sherman et al., EMBO J. 1989 Sep;8(9):2717-25; and Bechtold et al., Mol Gen Genet. 1995 Sep 20;248(5):610-20); Medermycin (*see, e.g.,* Ichinose et al., Microbiology 2003 Jul;149(Pt 7):1633-45); Monensin *(see, e.g.,* Arrowsmith et al., Mol Gen Genet. 1992 Aug;234(2):254-64); Nonactin (*see, e.g.,* FEMS Microbiol Lett. 2000 Feb 1;183(1):171-5); Nanaomycin *(see, e.g.,* Kitao et al., J Antibiot (Tokyo). 1980 Jul;33(7):711-6); Nemadectin *(see, e.g.,* MacNeil *et al.,* 1993, supra); Niddamycin *(see, e.g.,* PCT Pub. No. 98/51695; and Kakavas et al., 1997, J. Bacteriol. 179: 7515-7522); Oleandomycin (*see e.g.,* Swan et al., 1994, Mol. Gen. Genet. 242: 358-362; PCT Pub. No. 00/026349; Olano et al., 1998, Mol. Gen. Genet. 259(3): 299-308; and PCT Pat. App. Pub. No. WO 99/05283); Oxytetracycline *(see, e.g.,* Kim et al., Gene. 1994 Apr 8;141(1):141-2); Picromycin *(see, e.g.,* PCT Pub. No. 99/61599; PCT Pub. No. 00/00620; Xue et al., 1998, Chemistry & Biology 5(11): 661-667; Xue et al., October 1998, Proc. Natl. Acad. Sci. USA 95: 1211 12116); Platenolide *(see, e.g.,* EP Pub. No. 791,656; and U.S. Pat. No. 5,945,320); Rapamycin *(see, e.g.,* Schwecke et al., August 1995, Proc. Natl. Acad. Sci. USA 92:7839-7843; and Aparicio et al., 1996, Gene 169: 9-16); Rifamycin *(see, e.g.,* PCT Pub. No. WO 98/07868; and August et al., Feb. 13, 1998, Chemistry & Biology, 5(2): 69-79); Sorangium *(see, e.g.,* U.S. Pat. No. 6,090,601); Soraphen *(see, e.g.,* U.S. Pat. No. 5,716,849; Schupp et al., 1995, J. Bacteriology 177: 3673-3679); Spinocyn *(see, e.g.,* PCT Pub. No. 99/46387); Spiramycin *(see, e.g.,* U.S. Pat. No. 5,098,837); Tetracenomycin *(see, e.g.,* Summers et al., J Bacteriol. 1992 Mar;174(6):1810-20; and Shen et al., J Bacteriol. 1992 Jun;174(11):3818-21); Tetracycline (*see, e.g.,* JAm Chem Soc. 2009 Dec 9;131(48):17677-89); Tylosin *(see, e.g.,* U.S. Pat. No. 5,876,991; U.S. Pat. No. 5,672,497; U.S. Pat. No. 5,149,638; EP Pub. No. 791,655; EP Pub. No. 238,323; Kuhstoss et al., 1996, Gene 183:231-6; and Merson-Davies and Cundliffe, 1994, Mol. Microbiol. 13: 349-355); and 6-methylsalicyclic acid (*see, e.g.,* Richardson et al., Metab Eng. 1999 Apr; 1(2):180-7; and Shao et al., Biochem Biophys Res Commun. 2006 Jun 23;345(1):133-9).

### 6.2.4.3 Recombinant Cells Producing Fatty Acids

In another aspect, provided herein are methods of detecting fatty acid production in a cell or a clonal population of cells, *e.g.,* genetically modified to recombinantly produce one or more fatty acids. Fatty acid synthesis is mediated by fatty acid synthases (FAS), which catalyze the initiation and elongation of acyl chains. The acyl carrier protein (ACP) along with the enzymes in the FAS pathway control the length, degree of saturation, and branching of the fatty acid produced. The fatty acid biosynthetic pathway involves the precursors acetyl-CoA and malonyl-CoA. The steps in this pathway are catalyzed by enzymes of the fatty acid biosynthesis (*fab*) and acetyl-CoA carboxylase (*ace*) gene.

In some embodiments of the methods of detecting a fatty acid producing cell provided herein, the fatty acid producing cell comprises one or more heterologous nucleotide sequences encoding acetyl-CoA synthase and/or malonyl-CoA synthase, to effect increased production of one or more fatty acids as compared to a genetically unmodified parent cell.

For example, to increase acetyl-CoA production, one or more of the following genes can be expressed in the cell: *pdh, panK, aceEF* (encoding the EIp dehydrogenase component and the E2p dihydrolipoamide acyltransferase component of the pyruvate and 2-oxoglutarate dehydrogenase complexes), *fabH, fabD,fabG, acpP,* and *fabF.* Illustrative examples of nucleotide sequences encoding such enzymes include, but are not limited to: *pdh* (BAB34380, AAC73227, AAC73226), *panK* (also known as coaA, AAC76952), *aceEF* (AAC73227, AAC73226), *fabH* (AAC74175), fabD (AAC74176), *fabG* (AAC74177), *acpP* (AAC74178), *fabF* (AAC74179).

In some embodiments, increased fatty acid levels can be effected in the cell by attenuating or knocking out genes encoding proteins involved in fatty acid degradation. For example, the expression levels of *fadE, gpsA, idhA, pflb, adhE, pta, poxB, ackA,* and/or *ackB* can be attenuated or knocked-out in an engineered host cell using techniques known in the art. Illustrative examples of nucleotide sequences encoding such proteins include, but are not limited to: *fadE* (AAC73325), *gspA* (AAC76632), *IdhA* (AAC74462), *pflb* (AAC73989), *adhE* (AAC74323), *pta* (AAC75357), *poxB* (AAC73958), *ackA* (AAC75356), and *ackB* (BAB81430). The resulting host cells will have increased acetyl-CoA production levels when grown in an appropriate environment.

In some embodiments, the fatty acid producing cell comprises a heterologous nucleotide sequence encoding an enzyme that can convert acetyl-CoA into malonyl-CoA, *e.g.,* the multisubunit AccABCD protein. An illustrative example of a suitable nucleotide sequence encoding AccABCD includes but is not limited to accession number AAC73296, EC 6.4.1.2.

In some embodiments, the fatty acid producing cell comprises a heterologous nucleotide sequence encoding a lipase. Illustrative examples of suitable nucleotide sequences encoding a lipase include, but are not limited to accession numbers CAA89087 and CAA98876.

In some embodiments, increased fatty acid levels can be effected in the cell by inhibiting PlsB, which can lead to an increase in the levels of long chain acyl-ACP, which will inhibit early steps in the fatty acid biosynthesis pathway (*e.g., accABCD, fabH, and fabl*)*.* The expression level of PlsB can be attenuated or knocked-out in an engineered host cell using techniques known in the art. An illustrative example of a suitable nucleotide sequence encoding PlsB includes but is not limited to accession number AAC77011. In particular embodiments, the *plsB* D31 IE mutation can be used to increase the amount of available acyl-CoA in the cell.

In some embodiments, increased production of monounsaturated fatty acids can be effected in the cell by overexpressing an *sfa* gene, which would result in suppression of *fabA.* An illustrative example of a suitable nucleotide sequence encoding *sfa* includes but is not limited to accession number AAN79592.

In some embodiments, increased fatty acid levels can be effected in the cell by modulating the expression of an enzyme which controls the chain length of a fatty acid substrate, *e.g.,* a thioesterase. In some embodiments, the fatty acid producing cell has been modified to overexpress a *tes* or *fat* gene. Illustrative examples of suitable *tes* nucleotide sequences include but are not limited to accession numbers: (*tesA:* AAC73596, from *E. Coli,* capable of producing C_{18:1} fatty acids) and (*tesB:* AAC73555 from *E. Coli*). Illustrative examples of suitable *fat* nucleotide sequences include but are not limited to: (*fatB:* Q41635 and AAA34215, from *Umbellularia california,* capable of producing C_{12:0} fatty acids), (*fatB2:* Q39513 and AAC49269, from *Cuphea hookeriana,* capable of producing C_{8:0} - C_{10:0} fatty acids), (*fatB3:* AAC49269 and AAC72881, from *Cuphea hookeriana,* capable of producing C_{14:0}-C_{16:0} fatty acids), (*fatB:* Q39473 and AAC49151, from *Cinnamonum camphorum,* capable of producing C_{14:0} fatty acids), (*fatB [M141T]:* CAA85388, from m*Arabidopsis thaliana,* capable of producing C_{16:1} fatty acids), (*fatA*: NP 189147 and NP 193041, from *Arabidopsis thaliana,* capable of producing C_{18:1} fatty acids), (*fatA:* CAC39106, from *Bradvrhiizobium japonicum,* capable of preferentially producing C_{18:1} fatty acids ), (*fatA:* AAC72883, from *Cuphea hookeriana,* capable of producing C_{18:1} fatty acids ), and (*fatA1,* AAL79361 from *Helianthus annus*)*.*

In some embodiments, increased levels of C₁₀ fatty acids can be effected in the cell by attenuating the expression or activity of thioesterase C₁₈ using techniques known in the art. Illustrative examples of suitable nucleotide sequences encoding thioesterase C₁₈ include, but are not limited to accession numbers AAC73596 and P0ADA1. In other embodiments, increased levels of C₁₀ fatty acids can be effected in the cell by increasing the expression or activity of thioesterase C₁₀ using techniques known in the art. An illustrative example of a suitable nucleotide sequence encoding thioesterase C₁₀ includes, but is not limited to accession number Q39513.

In some embodiments, increased levels of C₁₄ fatty acids can be effected in the cell by attenuating the expression or activity of endogenous thioesterases that produce non-C₁₄ fatty acids, using techniques known in the art. In other embodiments, increased levels of C₁₄ fatty acids can be effected in the cell by increasing the expression or activity of thioesterases that use the substrate C₁₄-ACP, using techniques known in the art. An illustrative example of a suitable nucleotide sequence encoding such a thioesterase includes, but is not limited to accession number Q39473.

In some embodiments, increased levels of C₁₂ fatty acids can be effected in the cell by attenuating the expression or activity of endogenous thioesterases that produce non-C₁₂ fatty acids, using techniques known in the art. In other embodiments, increased levels of C₁₂ fatty acids can be effected in the cell by increasing the expression or activity of thioesterases that use the substrate C₁₂-ACP, using techniques known in the art. An illustrative example of a suitable nucleotide sequence encoding such a thioesterase includes, but is not limited to accession number Q41635.

### 5.2.4.4 Additional Genetic Modifications

In some embodiments of the methods and compositions provided herein, the genetically modified cell engineered to produce one or more water-immiscible compounds further comprises one or more genetic modifications which confer to the cell useful properties in the context of industrial fermentation.

In some embodiments, the cell further comprises one or more heterologous nucleotide sequences encoding one or more proteins that increase flocculation. Flocculation is the asexual, reversible, and calcium-dependent aggregation of microbial cells to form flocs containing large numbers of cells that rapidly sediment to the bottom of the liquid growth substrate. Flocculation is of significance in industrial fermentations of yeast, *e.g*., for the production of bioethanol, wine, beer, and other products, because it greatly simplifies the processes for separating the suspended yeast cells from the fermentation products produced therefrom in the industrial fermentation. The separation may be achieved by centrifugation or filtration, but separation by these methods is time-consuming and expensive. Clarification can be alternatively achieved by natural settling of the microbial cells. Although single microbial cells tend to settle over time, natural settling becomes a viable option in industrial processes only when cells aggregate (*i.e.,* flocculate). Recent studies demonstrate that the flocculation behavior of yeast cells can be tightly controlled and fine-tuned to satisfy specific industrial requirements (*see, e.g.,* Governder et al., Appl Environ Microbiol. 74(19):6041-52 (2008)).
Flocculation behavior of yeast cells is dependent on the function of specific flocculation proteins, including, but not limited to, products of the *FLO1, FLO5, FLO8, FLO9, FLO10,* and *FLO11* genes. Thus, in some embodiments, the genetically modified cell engineered to produce one or more water-immiscible compounds described herein comprises one or more heterologous nucleotide sequences encoding one or more flocculation proteins selected from the group consisting of Flolp, Flo5p, Flo8p, Flo9p, Flo10p, and Flo11p.

In some embodiments, the cell is sporulation impaired and/or endogenous mating impaired. A sporulation and/or endogenous mating impaired genetically modified microbial cell poses reduced risk of: (1) dissemination in nature; and (2) exchange of genetic material between the genetically modified microbial cell and a wild-type microbe that is not compromised in its ability to disseminate in nature. In yeast, the ability of diploid microbial cells to sporulate, and of haploid microbial cells to mate, is dependent on the function of specific gene products. Among these in yeast are products of sporulation genes, such as of the *IME1, IME2, NDT80, SPO11, SPO20, AMA1, HOP2,* and *SPO21* genes, and products of pheromone response genes, such as of the *STE5, STE4, STE18, STE12, STE7* and *STE11* genes.

In some embodiments, the cell is a haploid yeast cell in which one or more of the following pheromone response genes are functionally disrupted: *STE5, STE4, STE18, STE12, STE7,* and *STE11.* In some embodiments, the cell is a haploid yeast cell in which one or more of the following sporulation genes are functionally disrupted: *IME1, IME2, NDT80, SPO11, SPO20, AMA1, HOP2,* and *SPO21.* In some embodiments, the cell is a haploid yeast cell in which one or more of the following pheromone response genes: *STE5, STE4, STE18, STE12, STE7,* and *STE11,* and one or more of the following sporulation genes: *IME1, IME2, NDT80, SPO11, SPO20, AMA1, HOP2,* and *SPO21,* are functionally disrupted. In some embodiments, the cell is a haploid yeast cell in which the *IMPE1* gene and the *STE5* gene are functionally disrupted. In some embodiments, the cell is a haploid yeast cell in which the *IME1* gene and the *STE5* gene are functionally disrupted and that comprises a heterologous nucleotide sequence encoding an enzyme that can convert HMG-CoA into mevalonate. In some embodiments, the cell is a haploid yeast cell in which the *IME1* gene and the *STE5* gene are functionally disrupted, and that comprises a heterologous nucleotide sequence encoding an enzyme that can convert mevalonate into mevalonate 5-phosphate.

In some embodiments, the cell is a diploid yeast cell in which both copies of one or more of the following pheromone response genes are functionally disrupted: *STE5, STE4, STE18, STE12, STE7,* and *STE11.* In some embodiments, the cell is a diploid yeast cell in which both copies of one or more of the following sporulation genes are functionally disrupted: *IME1, IME2, NDT80, SPO11, SPO20, AMA1, HOP2,* and *SPO21.* In some embodiments, the cell is a diploid yeast cell in which both copies of one or more of the following pheromone response genes: *STE5, STE4, STE18, STE12, STE7,* and *STE11,* and both copies of one or more of the following sporulation genes: *IME1, IME2, NDT80, SPO11, SPO20, AMA1, HOP2,* and *SPO21,* are functionally disrupted. In some embodiments, the cell is a diploid yeast cell in which both copies of the *IME1* gene and both copies of the *STE5* gene are functionally disrupted. In some embodiments, the cell is a diploid yeast cell in which both copies of the *IME1* gene and both copies of the *STE5* gene are functionally disrupted, and that comprises a heterologous nucleotide sequence encoding an enzyme that can convert HMG-CoA into mevalonate. In some embodiments, the cell is a diploid yeast cell in which both copies of the *IME1* gene and both copies of the *STE5* gene are functionally disrupted, and that comprises a heterologous nucleotide sequence encoding an enzyme that can convert mevalonate into mevalonate 5-phosphate.

Methods and compositions useful for the introduction of heterologous sequences encoding flocculation proteins, and for the functional disruption of one or more sporulation genes and/or pheromone response genes, are described in U.S. Patent Application Publication No. 2010/0304490 and U.S. Patent Application Publication No. 2010/0311065.

In some embodiments, the cell comprises a functional disruption in one or more biosynthesis genes, wherein the cell is auxotrophic as a result of the disruption. In certain embodiments, the cell does not comprise a heterolgous nucleotide sequence that confers resistance to an antibiotic compound. In other embodiments, the cell comprises one or more selectable marker genes. In some embodiments, the selectable marker is an antibiotic resistance marker. Illustrative examples of antibiotic resistance markers include, but are not limited to the *BLA, NAT1, PAT, AUR1 -C, PDR4, SMR1, CAT,* mouse dhfr, *HPH, DSDA, KAN^{R},* and *SH BLE* gene products. The *BLA* gene product from *E. coli* confers resistance to beta-lactam antibiotics (e.g., narrow-spectrum cephalosporins, cephamycins, and carbapenems (ertapenem), cefamandole, and cefoperazone) and to all the anti-gram-negative-bacterium penicillins except temocillin; the *NAT1* gene product from *S. noursei* confers resistance to nourseothricin; the *PAT* gene product from *S. viridochromogenes* Tu94 confers resistance to bialophos; the *AUR1-C* gene product from *Saccharomyces cerevisiae* confers resistance to Auerobasidin A (AbA); the PDR4 gene product confers resistance to cerulenin; the SMR1 gene product confers resistance to sulfometuron methyl; the CAT gene product from Tn9 transposon confers resistance to chloramphenicol; the mouse dhfr gene product confers resistance to methotrexate; the *HPH* gene product of *Klebsiella pneumonia* confers resistance to Hygromycin B; the *DSDA* gene product of *E. coli* allows cells to grow on plates with D-serine as the sole nitrogen source; the *KAN^{R}* gene of the Tn903 transposon confers resistance to G418; and the *SH BLE* gene product from *Streptoalloteichus hindustanus* confers resistance to Zeocin (bleomycin). In some embodiments, the antibiotic resistance marker is excised, *e.g.,* from the host cell genome after the cell has been genetically modified to effect increased water-immiscible compound production. Methods and compositions useful for the precise excision of nucleotide sequences, *e.g*., sequences encoding such antibiotic resistance markers from the genome of a genetically modified host cell, are described in U.S. Patent Application No. 12/978,061, filed on December 23, 2010.

In some embodiments, the selectable marker rescues an auxotroph (e.g., a nutritional auxotroph) in the genetically modified microbial cell. In such embodiments, a parent microbial cell comprises a functional disruption in one or more gene products that function in an amino acid or nucleotide biosynthetic pathway, such as, for example, the *HIS3, LEU2, LYS1, LYS2, MET15, TRP1, ADE2,* and *URA3* gene products in yeast, which renders the parent microbial cell incapable of growing in media without supplementation with one or more nutrients (auxotrophic phenotype). The auxotrophic phenotype can then be rescued by transforming the parent microbial cell with an expression vector or chromosomal integration encoding a functional copy of the disrupted gene product, and the genetically modified microbial cell generated can be selected for based on the loss of the auxotrophic phenotype of the parent microbial cell. Utilization of the *URA3, TRP1,* and *LYS2* genes as selectable markers has a marked advantage because both positive and negative selections are possible. Positive selection is carried out by auxotrophic complementation of the *URA3, TRP1,* and *LYS2* mutations, whereas negative selection is based on specific inhibitors, *i.e*., 5-fluoro-orotic acid (FOA), 5-fluoroanthranilic acid, and α-aminoadipic acid (αAA), respectively, that prevent growth of the prototrophic strains but allows growth of the *URA3, TRP1,* and *LYS2* mutants, respectively.

In other embodiments, the selectable marker rescues other non-lethal deficiencies or phenotypes that can be identified by a known selection method.

Methods for genetically modifying microbes using expression vectors or chromosomal integration constructs, *e.g.,* to effect increased production of one or more water-immiscible compounds in a host cell, or to confer useful properties to such cells as described above, are well known in the art. *See,* for example, Sherman, F., et al., Methods Yeast Genetics, Cold Spring Harbor Laboratory, N.Y. (1978); Guthrie, C., et al. (Eds.) Guide To Yeast Genetics and Molecular Biology Vol. 194, Academic Press, San Diego (1991); Sambrook et al., 2001, Molecular Cloning -- A Laboratory Manual, 3rd edition, Cold Spring Harbor Laboratory, Cold Spring Harbor, NY; and Ausubel et al., eds., Current Edition, Current Protocols in Molecular Biology, Greene Publishing Associates and Wiley Interscience, NY. In addition, inhibition of gene expression, *e.g.,* which results in increased production of one or more water-immiscible compounds in the cell, may be accomplished by deletion, mutation, and/or gene rearrangement. It can also be carried out with the use of antisense RNA, siRNA, miRNA, ribozymes, triple stranded DNA, and transcription and/or translation inhibitors. In addition, transposons can be employed to disrupt gene expression, for example, by inserting it between the promoter and the coding region, or between two adjacent genes to inactivate one or both genes.

In some embodiments, increased production of water-immiscible compound in the cell is effected by the use of expression vectors to express a particular protein, *e.g.,* a protein involved in a biosynthetic pathway as described above. Generally, expression vectors are recombinant polynucleotide molecules comprising replication signals and expression control sequences, *e.g*., promoters and terminators, operatively linked to a nucleotide sequence encoding a polypeptide. Expression vectors useful for expressing polypeptide-encoding nucleotide sequences include viral vectors (*e.g*., retroviruses, adenoviruses and adeno-associated viruses), plasmid vectors, and cosmids. Illustrative examples of expression vectors sutibale for use in yeast cells include, but are not limited to CEN/ARS and 2µ plasmids. Illustrative examples of promoters suitable for use in yeast cells include, but are not limited to the promoter of the TEF1 gene of *K. lactis,* the promoter of the PGK1 gene of *Saccharomyces cerevisiae,* the promoter of the TDH3 gene of *Saccharomyces cerevisiae,* repressible promoters, *e.g.,* the promoter of the CTR3 gene of *Saccharomyces cerevisiae,* and inducible promoters, *e.g.,* galactose inducible promoters of *Saccharomyces cerevisiae* (*e.g.,* promoters of the GAL1, GAL7, and GAL10 genes).

Expression vectors and chromosomal integration constructs can be introduced into microbial cells by any method known to one of skill in the art without limitation. *See,* for example, Hinnen et al., Proc. Natl. Acad. Sci. USA 75:1292-3 (1978); Cregg et al., Mol. Cell. Biol. 5:3376-3385 (1985); U.S. Patent No. 5,272,065; Goeddel et al., eds, 1990, Methods in Enzymology, vol. 185, Academic Press, Inc., CA; Krieger, 1990, Gene Transfer and Expression -- A Laboratory Manual, Stockton Press, NY; Sambrook et al., 1989, Molecular Cloning -- A Laboratory Manual, Cold Spring Harbor Laboratory, NY; and Ausubel et al., eds., Current Edition, Current Protocols in Molecular Biology, Greene Publishing Associates and Wiley Interscience, NY. Exemplary techniques include, but are not limited to, spheroplasting, electroporation, PEG 1000 mediated transformation, and lithium acetate or lithium chloride mediated transformation.

### 7. EXAMPLES

### 7.1 Example 1: Generation of Genetically Modified Cells Producing a Water-immiscible Compound

This example describes an exemplary method for generating genetically modified haploid *S. cerevisiae* cells engineered to produce the isoprenoid farnesene.

The Phase I integration construct comprises as an integrating sequence nucleotide sequences that encode a selectable marker (hygA, which confers resistance to hygromycin B); two enzymes of the *S. cerevisiae* MEV pathway (the truncated HMG1 coding sequence, which encodes a truncated HMG-CoA reductase, and the ERG 13 coding sequence, which encodes HMG-CoA synthase), and another enzyme of *S. cerevisiae* (the ERG10 coding sequence, which encodes acetoacetyl-CoA thiolase), under control of galactose-inducible promoters (promoters of the *S. cerevisiae* genes GAL1 and GAL10); flanked by homologous sequences consisting of upstream and downstream nucleotide sequences of the *S. cerevisiae* GAL80 locus. Upon introduction into a *S. cerevisiae* host cell, the Phase I integration construct can integrate by homologous recombination into the GAL80 locus of the *S. cerevisiae* host cell genome, and functionally disrupt the GAL80 locus by replacing the GAL80 coding sequence with its integrating sequence. The Phase I integration construct was cloned into the TOPO Zero Blunt II cloning vector (Invitrogen, Carlsbad, CA), yielding plasmid TOPO-Phase I integration construct. The construct was propagated in TOP10 cells grown on LB agar containing 50 µg/ml kanamycin.

The Phase II integration construct comprises as an integrating sequence nucleotide sequences encoding a selectable marker (natA, which confers resistance to nourseothricin) and several enzymes of the *S. cerevisiae* MEV pathway (the ERG12 coding sequence, which encodes mevalonate kinase, and the ERG8 coding sequence, which encodes phosphomevalonate kinase), under control of galactose-inducible promoters (promoters of the *S. cerevisiae* genes GAL1 and GAL10); as well as the coding sequence of the *S. cerevisiae* GAL4 gene under control of the GAL4oc promoter (GAL4 promoter comprising a mutation that removes the MIG1 binding site thus making the promoter less sensitive to the repression by glucose); flanked by homologous sequences consisting of upstream and downstream nucleotide sequences of the *S. cerevisiae* LEU2 locus. Upon introduction into a *S. cerevisiae* host cell, the Phase II integration construct can integrate by homologous recombination into the LEU2 locus of the *S. cerevisiae* host cell genome, and functionally disrupt the LEU2 locus by replacing the LEU2 coding sequence with its integrating sequence. The Phase II integration construct was cloned into the TOPO Zero Blunt II cloning vector, yielding plasmid TOPO-Phase II integration construct. The construct was propagated in TOP10 cells (Invitrogen, Carlsbad, CA) grown on LB agar containing 50 µg/ml kanamycin.

The Phase III integration construct comprises as an integrating sequence nucleotide sequences encoding a selectable marker (kanA, which confers resistance to G418); an enzyme of the *S. cerevisiae* MEV pathway (the ERG19 coding sequence, which encodes diphosphomevalonate decarboxylase), and two enzymes of *S. cerevisiae* involved in converting the product of the MEV pathway, IPP, into FPP (the ERG20 coding sequence, which encodes farnesyl pyrophosphate synthase, and the IDI1 coding sequence, which encodes isopentenyl pyrophosphate decarboxylase), under control of galactose-inducible promoters (promoters of the *S. cerevisiae* genes GAL1, GAL10, and GAL7); as well as the promoter of the *S. cerevisiae* CTR3 gene; flanked by upstream and coding nucleotide sequences of the *S. cerevisiae* ERG9 locus. Upon introduction into a *S. cerevisiae* host cell, the Phase II integration construct can integrate by homologous recombination upstream of the ERG9 locus of the *S. cerevisiae* host cell genome, replacing the native ERG9 promoter with the CTR3 promoter in such a way that the expression of ERG9 (squalene synthase) can be modulated by copper. The Phase III integration construct was cloned into the TOPO Zero Blunt II cloning vector, yielding plasmid TOPO-Phase III integration construct. The construct was propagated in TOP10 cells grown on LB agar containing 50 µg/ml kanamycin.

The Phase I marker recycling construct comprises nucleotide sequences encoding a selectable marker (URA3, which confers the ability to grow on media lacking uracil); and an enzyme of *A. annua* (the FS coding sequence, which encodes farnesene synthase), under regulatory control of the promoter of the *S. cerevisiae* GAL7 gene; flanked by upstream nucleotide sequences of the *S. cerevisiae* GAL80 locus and coding sequences of the S. cerevisiae HMG1 gene. Upon introduction into a *S. cerevisiae* host cell, the Phase I marker recycling construct can integrate by homologous recombination into the already integrated Phase I integrating sequence such that the selective marker hphA is replaced with URA3.

The Phase II marker recycling construct comprises nucleotide sequences encoding a selectable marker (URA3, which confers ability to grow on media lacking uracil) and an enzyme of A *annua* (the FS coding sequence, which encodes farnesene synthase), under regulatory control of the promoter of the *S. cerevisiae* GAL7 gene; flanked by upstream nucleotide sequences of the *S. cerevisiae* LEU2 locus and coding sequences of the S. cerevisiae ERG 12 gene. Upon introduction into a *S. cerevisiae* host cell, the Phase II marker recycling construct can integrate by homologous recombination into the already integrated Phase II integrating sequence such that the selective marker natA is replaced with URA3.

The Phase III marker recycling construct comprises nucleotide sequences encoding a selectable marker (URA3, which confers the ability to grow on media lacking uracil) and an enzyme of *A annua* the FS coding sequence encodes farnesene synthase), under regulatory control of the promoter of the *S. cerevisiae* GAL7 gene; flanked by upstream nucleotide sequences of the *S. cerevisiae* ERG9 locus and coding sequences of the *S. cerevisiae* ERG 19 gene. Upon introduction into a *S. cerevisiae* host cell, the Phase II marker recycling construct can integrate by homologous recombination into the already integrated Phase III integrating sequence such that the selective marker kanA is replaced with URA3.

Expression plasmid pAM404 encodes a β-farnesene synthase. The nucleotide sequence insert was generated synthetically, using as a template the coding sequence of the β-farnesene synthase gene of *Artemisia annua* (GenBank accession number AY835398) codon-optimized for expression in *Saccharomyces cerevisiae.*

Starter host strain Y1198 was generated by resuspending active dry PE-2 yeast (isolated in 1994; gift from Santelisa Vale, Sertãozinho, Brazil) in 5 mL of YPD medium containing 100 ug/mL carbamicillin and 50 ug/mL kanamycin. The culture was incubated overnight at 30°C on a rotary shaker at 200 rpm. An aliquot of 10 uL of the culture was then plated on a YPD plate and allowed to dry. The cells were serially streaked for single colonies, and incubated for 2 days at 30°C. Twelve single colonies were picked, patched out on a new YPD plate, and allowed to grow overnight at 30°C. The strain identities of the colonies were verified by analyzing their chromosomal sizes on a Bio-Rad CHEF DR II system (Bio-Rad, Hercules, CA) using the Bio-Rad CHEF Genomic DNA Plug Kit (Bio-Rad, Hercules, CA) according to the manufacturer's specifications. One colony was picked and stocked as strain Y 1198.

Strains Y1661, Y1662, Y1663, and Y1664 were generated from strain Y1198 by rendering the strain haploid to permit genetic engineering. Strain Y1198 was grown overnight in 5 mL of YPD medium at 30°C in a glass tube in a roller drum. The OD600 was measured, and the cells were diluted to an OD600 of 0.2 in 5 mL of YP medium containing 2% potassium acetate. The culture was grown overnight at 30°C in a glass tube in a roller drum. The OD600 was measured again, and 4 OD600*mL of cells was collected by centrifugation at 5,000 x g for 2 minutes. The cell pellet was washed once with sterile water, and then resuspended in 3 mL of 2% potassium acetate containing 0.02% raffinose. The cells were grown for 3 days at 30°C in a glass tube in a roller drum. Sporulation was confirmed by microscopy. An aliquot of 33 µL of the culture was transferred to a 1.5 mL microfuge tube and was centrifuged at 14,000rpm for 2 minutes. The cell pellet was resuspended in 50 µL of sterile water containing 2 µL of 10 mg/mL Zymolyase 100T (MP Biomedicals, Solon, OH), and the cells were incubated for 10 minutes in a 30°C waterbath. The tube was transferred to ice, and 150 µL of ice cold water was added. An aliquot of 10 µL of this mixture was added to a 12 mL YPD plate, and tetrads were dissected on a Singer MSM 300 dissection microscope (Singer, Somerset, UK). The YPD plate was incubated at 30°C for 3 days, after which spores were patched out onto a fresh YPD plate and grown overnight at 30°C. The mating types of each spore from 8 four-spore tetrads were analyzed by colony PCR. A single 4 spore tetrad with 2 *MATα* and 2 *MATa* spores was picked and stocked as strains Y1661 (MATa), Y1662 (MATa), Y1663 (MATα), and Y1664 (MATα).

For yeast cell transformations, 25 ml of Yeast Extract Peptone Dextrose (YPD) medium was inoculated with a.single colony of a starting host strain. The culture was grown overnight at 30°C on a rotary shaker at 200rpm. The OD600 of the culture was measured, and the culture was then used to inoculate 50 ml of YPD medium to an OD600 of 0.15. The newly inoculated culture was grown at 30°C on a rotary shaker at 200rpm up to an OD600 of 0.7 to 0.9, at which point the cells were transformed with 1 µg of DNA. The cells were allowed to recover in YPD medium for 4 hours before they were plated on agar containing a selective agent to identify the host cell transformants.

Host strain Y1515 was generated by transforming strain Y1664 with plasmid TOPO-Phase I integration construct digested to completion using *PmeI* restriction endonuclease. Host cell transformants were selected on YPD medium containing 300 ug/mL hygromycin B, and positive transformants comprising the Phase I integrating sequence integrated at the GAL80 locus were verified by the PCR amplification.

Host strain Y1762 was generated by transforming strain Y1515 with plasmid TOPO-Phase II integration construct digested to completion using *PmeI* restriction endonuclease. Host cell transformants were selected on YPD medium containing 100 ug/mL nourseothricin, and positive transformants comprising the Phase II integrating sequence integrated at the LEU2 locus were verified by the PCR amplification.

Host strain Y1770 was generated by transforming strain Y1762 in two steps with expression plasmid pAM404 and plasmid TOPO-Phase III integration construct digested to completion using *PmeI* restriction endonuclease. Host cell transformants with pAM404 were selected on Complete Synthetic Medium (CSM) lacking methionine and leucine. Host cell transformants with pAM404 and Phase III integration construct were selected on CSM lacking methionine and leucine and containing 200 ug/mL G418 (Geneticin®), and positive transformants comprising the Phase III integrating sequence integrated at the ERG9 locus were verified by the PCR amplification.

Host strain Y1793 was generated by transforming strain Y1770 with a URA3 knockout construct (SEQ ID NO: 154). The URA3 knockout construct comprises upstream and downstream sequences of the URA3 locus (generated from *Saccharomyces cerevisiae* strain CEN.PK2 genomic DNA). Host cell transformants were selected on YPD medium containing 5-FOA.

Host strain YAAA was generated by transforming strain Y1793 with the Phase I marker recycling construct. Host cell transformants were selected on CSM lacking methionine and uracil. The URA3 marker was excised by growing the cells overnight in YPD medium at 30°C on a rotary shaker at 200rpm, and then plating the cells onto agar containing 5-FOA. Marker excision was confirmed by colony PCR.

Host strain YBBB was generated by transforming strain YAAA with the Phase II marker recycling construct. Host cell transformants were selected on CSM lacking methionine and uracil. The URA3 marker was excised by growing the cells overnight in YPD medium at 30°C on a rotary shaker at 200rpm, and then plating the cells onto agar containing 5-FOA. Marker excision was confirmed by colony PCR.

Host strain Y1912 was generated by transforming strain YBBB with the Phase III marker recycling construct. Host cell transformants were selected on CSM lacking methionine and uracil. The URA3 marker was excised by growing the cells overnight in YPD medium at 30°C on a rotary shaker at 200rpm, and then plating the cells onto agar containing 5-FOA. Marker excision was confirmed by colony PCR.

### 7.2 Example 2: Encapsulation of Cells

This example describes an exemplary method for encapsulating a cell in a hydrogel and screening the encapsulated cell for the recombinant production of one or more water-immiscible compounds.

### 7.2.1 Preparation of a Microfluidic System

A microfluidic device, composed of the elastomeric polymer poly(dimethysiloxane) (PDMS), and comprising at least two channels interconnected at a T-junction, is fabricated using an etched wafer substrate, *e.g.*, prepared by photolithography, as a mould.

In brief, the wafer substrate is prepared by rinsing the wafer with acetone and isopropyl alcohol. A photoresist, for example, SU8-3000 photresist (Microchem, Newton, MA) is applied to the wafer by spin-coating. The photoresist coating is then selectively irradiated with UV light through a mask designed to allow for exposure to the photoresist in a selected pattern, *e.g.*, a pattern comprising two channels interconnected at a T-junction. Following UV exposure, the wafer is processed by baking at 65°C for 1 minute, followed by baking at 95°C for 4 minutes. The photoresist is developed by immersing the wafer in glycol monomethyl ether acetate (PGMEA) solution, with shaking at 80 RPM for 4-5 minutes, followed by three rinses with isopropyl alcohol. The wafer is then baked at 200°C for 5-30 minutes, then allowed to cool to room temperature.

The etched wafer is contacted with PDMS (Sylgard®, Dow Coming, Midland, MI) to form the microfluidic device. Briefly, 70 grams of Sylgard® elastomer base is mixed with 7 grams of Sylgard® crosslinker in a container, and mixed. 34 grams of the mixed composition is poured onto the etched wafer, then placed in a vacuum dessicator for approximately 5 minutes (or until no bubbles are apparent on the surface of the wafer), followed by baking at 65°C for 60-90 minutes.

The polymerized PDMS is removed from the wafer, and subjected to plasma treatment (0.3 mbar for 20 secs.). A glass slide, used to enclose the microfluidic device, is also subjected to plasma treatment (0.3 mbar for 20 secs.). Following plasma treatment, the glass slide is placed, plasma-exposed side down,. on the PDMS, and air bubbles are removed with gentle pressure. The sealed microfluidics device is then baked at 65°C for 10 minutes. The channels of the device are then subjected to treatment with a water repellant, for example, a silane/siloxane based repellent such as Aquapel™ (Pittsburgh Glass Works LLC, Pittsburgh, PA). A two-dimensional view of an exemplary microfluidic device comprising two channels interconnected at a T-junction, prepared as described herein, is provided in **FIG. 4** and **FIG. 5A**.

### 7.2.2 Preparation of Cells for Encapsulation

500 ul of a liquid cell culture, or a few colonies from a plate, comprising cells to be screened for heterologous water-immiscible compound production, are added to 1 ml of PBS-MBF (1x PBS with: 10 mM mannose, 0.5% BSA, 0.001% Pluronic F-127), vortexed for approximately 10 seconds, and centrifuged for 30 seconds at 5000 g. The supernatant is removed, 1 ml PBS-MBF is added, and the cells are vortexed for 10 seconds and centrifuged for 1 minute at 5000 g. The supernatant is removed, and the cells are resuspended in 1 ml of PBS-F.

The cell suspension is filtered by loading the suspension onto a black 10 µM Par-tec filter and centrifuging briefly (~500 rcf). The sample is resuspended with brief vortexing, then diluted 1:9 in PBS. The OD₆₀₀ is determined, and the cells are diluted in PBS-F (lx PBS with: 10 mM mannose, 0.5% BSA, 0.001% Pluronic F-127) to 1 ml of culture at the correct OD₆₀₀, that is, 2x the final cell concentration needed for a given drop size.

An agarose solution is prepared for encapsulating the cells. Briefly, 0.5 g LMP agarose (*e.g.*, Omnipur®, EM Science, Gibbstown, NJ) is suspended in 25 ml water in a 100 ml bottle, and microwaved in 10 second intervals until fully melted and dissolved. The agarose is allowed to cool to ~30-35°C, and then mixed 1:1 with the cell suspension that has also been warmed to 30-35°C.

### 7.2.3 Encapsulation of Cells in a Hydrogel Particle

The cell suspension, prepared as described above, is added to the syringe of a 26 gauge needle, and 12 inches of PE/2 tubing is fitted onto the needle tip. Cells are expelled, *e.g.*, through automated means, through the needle tip into the tubing. The tubing is inserted into the entryway of the cell solution flow channel. 12 inches of 1/32" OD PEEK tubing is inserted at the opposite end of the flow channel, to serve as an outlet for the oil/hydrogel emulsion.

An automated oil dispenser is fitted with a syringe and tubing, and the syringe is primed with oil at a flow rate of 5,000 to 10,000 µl/h. The tubing is inserted into the entryway of the oil flow channel on the microfluidic device, and the oil flow rate is set to 850 to 1,000 µl/h. The oil flow is turned on momentarily to wet the junction between the tubing and the device.

Aqueous flow (comprising cells in agarose) is started through the cell solution flow channel, followed shortly by the oil flow through the oil flow channel. Emulsion comprising the hydrogel particles is collected from the PEEK tubing in a 2 ml collection tube stored on ice.

After hydrogel particles have been solidified on ice for 5 min., the lower oil layer is removed with a pipette. 500 ul of 20% PFO (20% V/V perfluorooctanol in HFE-7500), is added to the particles and the suspension is vortexed, then centrifuged for 30 sec. at 6000 g. Residual oil is removed and 5 ml of desired growth medium + 0.001% F127 is added, and the suspension is vortexed thoroughly. An additional 5 mls of growth medium is added, and the suspension is centrifuged for 1 minute at 6000 g. The supernatant is poured off, and the pellet is vortexed briefly before being transferred into a 1.5 ml eppendorf tube. 500 µl of growth media is added and the suspension is vortexed briefly, then centrifuged for 1 minute at 6000 g. The supernatant is removed, the approximate weight of the hydrogel particles is noted, and twice the particle weight of growth medium is added. The particles are resuspended and transferred to a 14 ml falcon tube. The solution is then shaken for 24 hours at 34°C under suitable cell culture conditions to allow for cell proliferation and heterologous water-immiscible compound production.

### 7.3 Example 3: Particle Analysis and Sorting

This example describes an exemplary method for analyzing and sorting hydrogel particles comprising cells producing water-immiscible compound.

A 70 µm BD cellstrainer is placed on a 50 ml conical tube. Culture comprising the encapsulated cells is applied to the center of the filter membrane with a 5 ml pipette. The particles are washed off the membrane with two 1 ml aliquots of PBS, centrifuged for 30 sec. at 100 g, resuspended in 1 ml PBS and centrifuged again for 30 sec. at 100 g. The filtered culture is then added in aliquots to a 10 µm Partec filter and centrifuged for 90 sec. at 100g. 1 ml of PBS is added to the filter cake of each filter and the cake is resuspended by pipetting up and down. Another 1 ml of PBS is added to the suspension and centrifuged for 90 sec. at 100 g.

Nile Red staining solution (2 ml / sample) is prepared by adding 200 µl Nile Red stock (100 µg/ml in EtOH) to every 10 ml of PBS (2 µg/ml final), as needed.

1 ml of staining solution is added to the filter cake of each filter and resuspended by pipetting up and down. Another 1 ml of staining solution is added and centrifuged for 90 sec. at 120 g. The particles are removed from the filter by adding 1 ml of plain PBS, pipetting up and down to re-suspend, and transferring the particles to a FACS tube. The membrane is washed with a second 1 ml of PBS and transferred to the same tube.

The particles are then sorted based on fluorescence intensity by fluorescence activated cell sorting (FACS), corresponding to the level of heterologous water-immiscible compound production, normalized against the biomass of cells contained in the particle. This analysis takes advantage of the solvatochromic properties of Nile Red. In a polar lipid, such as the phospholipid cell membrane, Nile Red has a fluorescence emission maximum of ~ 5.90 nm. By contrast, in the presence of a neutral lipid, the spectrum is blue-shifted with an emission maximum of 550 nm. Thus, optical filters in the green (515 +/- 20 nm) and red (670 +/- 20 nm) regions of the spectrum are used in order to maximize the ratio of green to red fluorescence between the ideal producer (pure farnesene) and a complete non-producer (see **FIG. 6**).

Encapsulated samples are analyzed on a FACSAria II (Becton Dickinson, San Jose, CA). Peak height, area and width are collected from four parameters: forward and side scatter from a solid state 488 nm laser (FSC and SSC respectively), green fluorescence from a 515 nm bandpass filter, and red fluorescence from a 670 nm bandpass filter. Encapsulated colonies are first gated away from any debris, remaining unencapsulated cells and empty particles using a plot of SSC area as a function of the FSC area parameters (see **FIG. 7A**).

To determine the amount of product produced within particles, a first analysis is performed wherein noise caused by the variable number of cells within a particle is accounted for. The fluorescence of Nile Red in a neutral lipid, such as a hydrocarbon product (*e.g.*, famesene) is blue-shifted relative to its fluorescence in a polar lipid such as a phospholipid membrane. By taking the ratio of fluorescence from green (product) to red (cell biomass), most of the noise arising from variation in cell number is eliminated. This normalization reduces the coefficient of variation from 80-100% to 8-12%, depending on the strain (see, *e.g.*, **FIGS. 7C** and **7D**, respectively). Therefore, the second plot for selecting the sorted population is of the ratio of green to red fluorescence (G/R); a population having both high green (G) and high G/R signal is selected (see, *e.g.*, the boxed population in **FIG 7B**).

Events are collected at 500-1000 events per second to collect 10,000 events for setting gates. The "sort gate" of high G+G/R colonies contains between 0.5% and 20% of the total colonies depending on the expected phenotype change of mutants, the round of screening, and the population size. The sorted population is plated directly onto nutrient agar petri dishes; cells grow out of the particles and form colonies on the agar, which are recovered for further processing, for example, a subsequent round of encapsulation, sorting and culturing. A screen comprising between 2 to 6 rounds of this encapsulation, sorting and growth cycle can be performed to enrich for cell populations producing high amounts of water-immiscible compound. After the final round of sorting, individual colonies can be selected for evaluation in traditional bulk-culture screening methods for corroboration of high level production.

### 7.4 Example 4: Identification and Selection of a Farnesene Producing Cell

This example demonstrates the sensitivity and fidelity of detection of heterologous water-immiscible compound in recombinant yeast cells engineered to produce low to high levels of farnesene. A ladder of yeast strains, generated in accordance with the methods described in Example 1, and which represent a broad range of farnesene production, was encapsulated and sorted in accordance with the methods described in Examples 2 and 3, respectively. Nile Red was used as the detection agent. Farnesene levels detected by the picoscreening method were compared to those detected by standard methods of measurement, including 2-liter fermenter yields, Nile Red 96-well shake plates, and farnesene flux.

Nile Red 96-well shake plate assays were performed as follows. For each strain, single colonies were picked from an agar plate into a 1.1 ml 96 well plate containing 360 µl of Bird Seed Medium (BSM) 2% sucrose 0.25N+ crb (pre-culture media). The pre-culture plate was sealed with a breathable membrane seal, and the culture was incubated for 96 hours at 33.5°C, 80% humidity, with shaking at 1000 RPM. 14.4 µl of pre-culture media was transferred into 360 µl (1:25 dilution) of BSM 4% sucrose contained in a 1.1 ml 96 well production plate. The production plate was sealed with a breathable membrane seal, and the culture was incubated for 48 hrs at 33.5°C, 80% humidity, with shaking at 1000 RPM. Following incubation, 98µl of production culture was mixed with 2µL of Nile Red solution (final Nile Red concentration of 2 µg/ml) in a 96-well black polystyrene flat bottom assay plate. The plate was mixed for 30 sec. prior to loading onto a spectrophotometer, and a farnesene-specific read was obtained with excitation at 290 nm and emission at 550 nm. A cell biomass-specific read was also obtained, with excitation at 350 nm and emission at 450 nm, and a farnesene to biomass ratio was determined.

Farnesene flux assays were performed as follows. For each strain, single colonies were picked from an agar plate into a 1.1 ml 96 well plate containing 360 µl of BSM 2% sucrose (pre-culture media). The pre-culture plate was sealed with a breathable membrane seal, and the culture was incubated for 65-72 hours at 33.5°C, 80% humidity, with shaking at 1000 RPM. 30 µl of pre-culture media was transferred into 360 µl of BSM 4% sucrose contained in a 1.1 ml 96 well production plate. The production plate was sealed with a breathable membrane seal, and the culture was incubated for 48 hours at 33.5°C, 80% humidity, with shaking at 1000 RPM. OD₆₀₀ measurements were taken for each culture prior to determination of farnesene titer. 200 µL of each culture was transferred into a 2.2-mL plate, and 400 µL/well of methanol was added. The plate was sealed and shaken for 30-40 minutes. The seal was removed and 600 µL/well of n-heptane containing 0.001% trans-caryophyllene was added, and the plate was sealed and shaken for 90 minutes. Plates were then centrifuged at 2500 g for 5 minutes. For each sample, 200 µl/well of the top heptane layer from the 2.2-mL plate was transferred into a well of a 1.1-mL plate, and 200 µL/well of n-heptane containing 0.001% trans-caryophyllene was added. The plate was sealed, shaken for 2 minutes, then centrifuged at 2500 g for 2 minutes. The heptane extracts were analyzed on an Agilent 7890 Gas Chromatography System (Agilent Technologies, Inc., Palo Alto, CA) with flame ionization detection (FID). Farnesene titers were calculated by comparing generated peak areas against a quantitative calibration curve of purified biologically derived trans-β-farnesene (Amyris Inc., Emeryville, CA) in heptane.

2L fermenter yields were determined as follows. For each strain, a 250-mL unbaffled shake flask containing 50-mL sterile BSM 2% sucrose buffered w/succinate was inoculated with 1.0 mL of thawed working seed stock. The inoculum flask grew for approximately 24 hours at 200 rpm with 2" throw, 34°C to an approximate OD₆₀₀ of 3.5. A Fernbach shake flask containing 800 mL of sterile media was inoculated with 50 mL of inoculum. Seed flasks were allowed to grow for ~24 hours at 200 rpm with 2" throw, 34°C to an approximate OD₆₀₀ of 3.5 and ethanol >2 g/L. The entirety of the post-inoculation volume was used for 2-L fermentations. The fermentor batch medium was prepared by combining 2X batch base with DI water and autoclaving. Sterile trace metals and vitamins were added as post-sterile additions and the media was equilibrated at the target process conditions prior to inoculation. Tergitol L-81 antifoam (0.1 mL/L final volume) was also added to the fermentor after inoculation. The pH was controlled using 28% ammonium hydroxide. The sugar feed was 750 g/L sucrose. Fermentation proceeded for six days at 33.5°C, pH 5, impeller agitation, OUR 80-150 during production phase. Cell densities were measured and farnesene titers were determined by gas chromatography as described above.

**FIG. 8** provides, for each of seven strains engineered to produce varying levels of farnesene, farnesene yield (normalized to biomass and expressed in arbitrary units) as determined by picoscreening (y-axis), plotted against yield determinations made by 2L fermenter yield (x-axis, left plot), nile red shake plate assay (x-axis, center plot), and farnesene flux analysis (x-axis, right plot), respectively. Correlation is good (R²>90%) across a broad range of production values. These correlations validate the picoscreen assay as being able to distinguish farnesene production across a population of strains producing varying amounts of farnesene.

### 7.5 Example 5: Enrichment for a High Farnesene-Producing Population

This example demonstrates that the methods of encapsulation and detection ("picoscreening") provided herein, when performed iteratively, are effective to enrich for high producing cells from a background of lower producers.

To demonstrate the ability of picoscreening methodology to enrich high producers out of a background of lower producers, a digital colony PCR assay was developed to unambiguously distinguish different encapsulated strains co-cultured in a single population before and after sorting, and to determine the relative abundance of one strain over another. A pair of strains differing by the number of integrated farnesene synthase (FS) genes in their genome was utilized as a model system. A greater number of copies of FS in the genome results in higher amounts of farnesene produced by the strain. The first strain, FS2, has two integrated copies of FS, and the second, FS5, has five integrated copies. FS5 was independently confirmed to produce farnesene at a yield of ~ 30% over FS2.

Three primers were designed that could be utilized to unambiguously distinguish between the FS2 and FS5 strains in a single PCR reaction. The first primer targets the TCYC1 terminator sequence, which is common to both strains, and acts as the forward primer. The second primer acts as a reverse primer that targets a region found in the integrated sequence comprising the second copy of FS, which is also common to both strains; amplification from this locus produces an 89 bp amplicon. The third primer also acts as a reverse primer that targets a region found in the integrated sequence comprising the fifth copy of FS, which is unique to strain FS5 and not present in strain FS2; amplification from this locus produces a 304 bp amplicon. When a PCR was performed on each strain with a mixture of the three primers, strain FS2 produced one band (89 bp only), and strain FS5 produced two bands (89 bp and 304 bp) (see FIG. 9). The presence of the 304 bp band is used as an unambiguous marker that a colony is derived from strain FS5.

Strains FS2 and FS5 were mixed into single separate libraries in FS2:FS5 ratios of 10:1, 100:1, and 1000:1, respectively, and picoscreening was performed on each of these model libraries. Three rounds of encapsulation and sorting were performed on each library. Between rounds, the sorted particles were directly plated on large petri trays to grow, and 96 colonies were selected from each round and subjected to colony PCR using the aforementioned three primers to determine the identity of each colony. From these PCR data, the percentage of the plated population represented by colonies derived from strain FS5 were determined after each round of encapsulation and sorting.

The results provided in **FIG. 10** demonstrate the successful enrichment of strain FS5 over 3 rounds of encapsulation and sorting. **FIG. 10A** provides FACS histrogram data of the pure strains, presented in rows 1 and 2. Histograms of the 100:1 mixed population after rounds 1, 2 and 3 of encapsulation and sorting (rows 3-5) show that substantial enrichment of strain FS5 was achieved by round three. **FIG. 10B** provides quantitative results, derived from cPCR data, of enrichment between rounds of sorting for each of the 10:1, 100:1 and 1000:1 libraries. In these studies, for libraries having starting FS2:FS5 ratios of 10:1 and 100:1, respectively, enrichment for FS5 was nearly complete, such that by round 3, nearly 100% of the resulting population consisted of FS5 derived colonies. Even when FS5 was rare in the starting population (~10%), enrichments of approximately 5-fold per round were observed. These results demonstrate that near complete enrichment for a rare, high producing population of cells within a population of lower producing cells can be efficiently achieved by picoscreening.

### 7.6 Example 6: Identification and Selection of Cells Producing Other Water-Immiscible Compounds

This example demonstrates the effectiveness of picoscreening for detecting, sorting and selecting cells recombinantly producing other water-immiscible compounds.

### 7.6.1 Limonene Producing Cells

In addition to recombinant production of the sesquiterpene farnesene, other isoprenoids, including monoterpenes, can be detected in cells by picoscreening. Monoterpenes generally comprise two isoprene units and have the molecular formula C₁₀H₁₆. The monoterpene limonene is naturally found in the rind of citrus fruits and peppermint, and is represented by the structure: 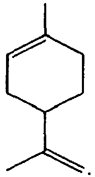 Limonene is made from geranyl pyrophosphate (GPP) by limonene synthase. A series of yeast strains producing varying amounts of limonene were generated in accordance with the methods described in Example 1, with the exception that strains were engineered to express a gene encoding limonene synthase rather than farnesene synthase.

Limonene producing strains were encapsulated and sorted in accordance with the methods described in Examples 2 and 3, respectively. Nile Red was used as the detection agent. Limonene levels detected by the picoscreening method were compared to those detected by 96-well shake plate assays, which were performed as follows. For each strain, single colonies were picked from an agar plate into a 1.1 ml 96 well plate containing 360 µl of Bird Seed Medium (BSM) 2% sucrose 0.25N+ crb (pre-culture media). The pre-culture plate was sealed with a breathable membrane seal, and the culture was incubated for 72 hours at 30°C, 80% humidity, with shaking at 1000 RPM. 6 µl of pre-culture media was transferred into 75 µl of BSM 4% galactose and 75 µl of isopropyl myristate contained in a 1.1 ml 96 well production plate. The production plate was sealed with a non-permeable aluminum foil heat seal with a Velocity 11 plate loc^{™} (Agilent Technologies), and the culture was incubated for 72 hrs at 30°C, 80% humidity, with shaking at 1000 RPM. Following incubation, the plates were flash frozen at -20C for 2 hours to reduce the vapor pressure of the monoterpene product. At this stage, the plate seals were rapidly removed and 300 ul of ethyl acetate containing a hexadecane internal standard were added using a Phoenix liquid handler (Art Robbins Instruments) with a 15 mm dispense height and a pumping speed of 3mm/s directly to the frozen culture broth. The plates were immediately sealed again using non-permeable aluminum seals and shaken at RT for 2 hours with a rotational speed of 90RPM on a microtiter plate agitator. After shaking for 2 hours, the plates were centrifuged at 2000RPM for 2 minutes and directly assayed using GC-FID (gas chromatography with flame ionization detection). GC-FID analysis for monoterpene production was quantified by injecting 2ul of the ethyl acetate with a split ratio of 1:50 onto a methyl silicone stationary phase column. Oven parameters were ramped from 25°C to 250°C over the course of 2.5 minutes followed by rapid cooling for the next sample injection. Standards of limonene and myrcene were injected before each run to quantify the precise ppm integrated area under sample peaks. These values were subsequently converted to absolute titers to determine the values shown in **FIG. 11**.

**FIG. 11A** provides, for each of three strains engineered to produce varying levels of limonene (L1, L2 and L3) and a naïve non-producing strain (Y0): (left panel) G/R fluorescence peaks corresponding to the levels of limonene produced per strain; and (right panel) limonene yield (normalized to biomass and expressed in arbitrary units) as determined by picoscreening (y-axis), plotted against yield determinations made by shake flask fermenter yield (x-axis). A correlation can be seen across a broad range of limonene production values. **FIG. 11B** provides fluorescence peaks of encapsulated producing cells (L1) and non-producing cells (Y0) either co-cultured together (solid peaks) or cultured separately (hollow peaks). The separate fluorescence peaks for Y0 and L1 are maintained under co-culture conditions, indicating that product remains encapsulated in particles containing a producing strain, and does not bleed out or into particles containing a non-producing strain. Differences in the median value can be attributed to tube-to-tube variation.

These results demonstrate that the picoscreen assay can be used to identify and distinguish host strains producing varying levels of limonene.

### 7.6.2 Patchoulol Producing Cells

Patchoulol, a sesquiterpene whose structure is 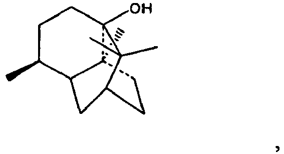 is also known as patchouli alcohol and is a constituent of the essential oil of *Pogostemon patchouli.* Patchoulol is made from FPP by patchoulol synthase. A series of yeast strains producing varying amounts of patchoulol were generated in accordance with the methods described in Example 1, with the exception that strains were engineered to express patchoulol synthase rather than farnesene synthase. Patchoulol producing strains were encapsulated and sorted in accordance with the methods described in Examples 2 and 3, respectively. Nile Red was used as the detection agent. Patchoulol levels detected by the picoscreening method were compared to those detected by 96-well shake plate yields as follows. For each strain, single colonies were incubated in separate wells of a 96-well plate containing 360 uL Bird Seed Medium (BSM) with 2% sucrose per well (preculture). After 2 days of incubation at 30°C with 999 rpm agitation, 6.4 uL of each well was inoculated into a well of a new 96-well plate containing 150 uL of fresh BSM with 4% galactose and 3.33% mineral oil and Brij-56 emulsion (production culture). After another 4 days of incubation at 30°C with 999 rpm agitation, samples were taken and analyzed for patchoulol production by gas chromatography (GC) analysis. For GC analysis, samples were extracted with methanol-butoxy ethanolheptane (100uL:50uL:400uL v/v), and the cell material was allowed to settle by gravity. An aliquot of the heptane extract was further diluted into heptane, and then injected onto a methyl silicone stationary phase using a pulsed split injection.

**FIG. 12** provides results of a picoscreen for the detection of patchoulol recombinantly produced from encapsulated yeast cells. A non-producing strain (Y0) and two different patchoulol producing strains (P1 and P2) were encapsulated and subjected to picoscreen. **FIG. 12A** provides G/R fluorescence peaks corresponding to the levels of patchoulol produced per strain. **FIG. 12B** provides limonene yield (normalized to biomass and expressed in arbitrary units) as determined by picoscreening (y-axis), plotted against yield determinations made by 96-well shake plate yield (x-axis). These results show that the FACS values are proportional to the standard shake plate titers as measured by GC.

These results demonstrate that the picoscreen assay can be used to identify and distinguish host strains producing varying levels of patchoulol.

## Claims

1. A method of detecting a recombinant cell produced water-immiscible compound, the method comprising:
(a) encapsulating the cell in a hydrogel particle;
(b) culturing the cell within the hydrogel particle whereby a cell population is formed within the particle; and
(c) detecting the recombinantly produced water-immiscible compound within the hydrogel particle, wherein said detecting comprises contacting the hydrogel particle with a fluorescent solvatochromic dye and normalizing the amount of water-immiscible compound within the hydrogel particle to the amount of cell biomass within the hydrogel particle.

2. The method of claim 1, wherein said encapsulating comprises contacting the cell with an aqueous hydrogel suspension under conditions sufficient to form a hydrogel particle comprising the cell.

3. The method of claim 2, wherein said conditions comprise contacting the aqueous hydrogel suspension comprising the cell with a fluorocarbon oil comprising a fluorosurfactant.

4. The method of claim 3, wherein said contacting with a fluorocarbon oil comprises loading the aqueous hydrogel suspension comprising the cell onto a microfluidic device comprising the fluorocarbon oil, wherein said hydrogel suspension contacts the fluorocarbon oil at a T-junction of the microfluidic device, wherein said contacting with the fluorocarbon oil results in formation of a non-aqueous hydrogel particle comprising the cell.

5. The method of claim 4, further comprising the step of separating the hydrogel particle from the fluorocarbon oil.

6. The method of claim 1, further comprising the step of culturing the cell within the hydrogel particle prior to said detecting.

7. The method of claim 6, wherein said culturing is for a period of 12 to 24 hours.

8. A method of screening a library of cells for a cell recombinantly producing a water-immiscible compound, the method comprising:
(a) encapsulating each cell of the library in a hydrogel particle;
(b) culturing the cell within the hydrogel particle whereby a cell population is formed within the particle;
(c) detecting recombinantly produced water-immiscible compound within each hydrogel particle, wherein said detecting comprises contacting the hydrogel particle with a fluorescent solvatochromic dye and normalizing the amount of water-immiscible compound within the hydrogel particle to the amount of cell biomass within the hydrogel particle; and
(d) selecting a cell producing said recombinantly produced water-immiscible compound.

9. A method of enriching a population of cells for cells recombinantly producing a water-immiscible compound, the method comprising:
(a) providing a population of hydrogel particles, wherein the population comprises hydrogel particles that encapsulate a cell or a clonal population of cells genetically modified to produce a water-immiscible compound;
(b) culturing the cell or clonal population of cells within each hydrogel particle;
(c) detecting a hydrogel particle comprising recombinantly produced water-immiscible compound, wherein said detecting comprises contacting the hydrogel particle with a fluorescent solvatochromic dye and normalizing the amount of water-immiscible compound within the hydrogel particle to the amount of cell biomass within the hydrogel particle;
(d) recovering the cell or clonal population of cells from the hydrogel particle of step (b);
(e) re-encapsulating the cell or clonal population of cells from step (c); and
(f) repeating steps (a) - (d).

10. A hydrogel-encapsulated cell or clonal cell population comprising recombinantly produced water-immiscible compound and a fluorescent solvatochromic dye that enables normalization of the amount of water-immiscible compound within the hydrogel to the amount of cell biomass within the hydrogel.

11. A hydrogel particle comprising a cell or clonal cell population, recombinantly produced water-immiscible compound and a fluorescent solvatochromic dye that enables normalization of the amount of water-immiscible compound within the hydrogel to the amount of cell biomass within the hydrogel.

12. The method of claim 1, the hydrogel-encapsulated cell or clonal cell population of claim 10, or the hydrogel particle of claim 11, wherein the fluorescent solvatochromic dye is Nile Red.

13. The method of claim 1, the hydrogel-encapsulated cell or clonal cell population of claim 10, or the hydrogel particle of claim 11, wherein the cell is selected from the group consisting of a yeast cell, a bacterial cell, a mammalian cell, a fungal cell, an insect cell, and a plant cell.

14. The method of claim 1, the hydrogel-encapsulated cell or clonal cell population of claim 10, or the hydrogel particle of claim 11, wherein the cell is a yeast cell.

15. The method of claim 1, the hydrogel-encapsulated cell or clonal cell population of claim 10, or the hydrogel particle of claim 11, wherein the recombinantly produced water-immiscible compound is selected from the group consisting of an isoprenoid, a polyketide and a fatty acid.

16. The method of claim 1 or the hydrogel particle of claim 11, wherein the hydrogel particle is capable of retaining a water-immiscible compound.

17. The method of claim 1 or the hydrogel particle of claim 11, wherein the hydrogel particle comprises agarose.

18. The method of claim 1 or the hydrogel particle of claim 11, wherein the hydrogel particle is less than about 1 millimeter in diameter.

19. The method of claim 1 or the hydrogel particle of claim 11, wherein the hydrogel particle is less than about 500 micrometers in diameter.

20. The method of claim 1 or the hydrogel particle of claim 11, wherein the hydrogel particle is less than about 100 micrometers in diameter.

21. The method of claim 1 or the hydrogel particle of claim 11, wherein the hydrogel particle is less than 50 micrometers in diameter.

22. The method of claim 1 or the hydrogel particle of claim 11, wherein the hydrogel particle is about 50, 45, 40, 35, 30 or 25 micrometers in diameter.

23. The method of claim 15, the hydrogel-encapsulated cell or clonal cell population of claim 15, or the hydrogel particle of claim 15, wherein the isoprenoid is a C₅-C₃₀ isoprenoid.

24. The method of claim 15, the hydrogel-encapsulated cell or clonal cell population of claim 15, or the hydrogel particle of claim 15, wherein the isoprenoid is selected from the group consisting of abietadiene, amorphadiene, carene, α-farnesene, β-farnesene, farnesol, geraniol, geranylgeraniol, isoprene, linalool, limonene, myrcene, nerolidol, ocimene, patchoulol, β-pinene, sabinene, γ-terpinene, terpinolene, and valencene.

## Patentansprüche

1. Verfahren zum Detektieren einer von einer rekombinanten Zelle produzierten, nicht mit Wasser mischbaren Verbindung, wobei das Verfahren Folgendes umfasst:
(a) Einkapseln der Zelle in ein Hydrogelpartikel;
(b) Kultivieren der Zelle innerhalb des Hydrogelpartikels, wodurch innerhalb des Partikels eine Zellpopulation gebildet wird; und
(c) Detektieren der rekombinant hergestellten, nicht mit Wasser mischbaren Verbindung innerhalb des Hydrogelpartikels, worin das Detektieren das Kontaktieren des Hydrogelpartikels mit einem fluoreszierenden solvatochromen Farbstoff und das Normalisieren der Menge der nicht mit Wasser mischbaren Verbindung innerhalb des Hydrogelpartikels auf die Menge der Zellbiomasse innerhalb des Hydrogelpartikels umfasst.

2. Verfahren nach Anspruch 1, worin das Einkapseln das Kontaktieren der Zelle mit einer wässrigen Hydrogel-Suspension unter Bedingungen umfasst, die ausreichen, um ein Hydrogelpartikel zu bilden, das die Zelle umfasst.

3. Verfahren nach Anspruch 2, worin die Bedingungen das Kontaktieren der wässrigen Hydrogel-Suspension, die die Zelle umfasst, mit einem Fluorkohlenstofföl, das ein Fluortensid umfasst, umfassen.

4. Verfahren nach Anspruch 3, worin das Kontaktieren mit einem Fluorkohlenstofföl das Laden der wässrigen Hydrogel-Suspension, die die Zelle umfasst, auf eine Mikrofluidvorrichtung umfasst, die das Fluorkohlenstofföl umfasst, worin die Hydrogel-Suspension das Fluorkohlenstofföl an einer T-Übergangsstelle der Mikrofluidvorrichtung kontaktiert, worin das Kontaktieren mit dem Fluorkohlenstofföl zu einer Bildung eines nichtwässrigen Hydrogel-Partikels führt, das die Zelle umfasst.

5. Verfahren nach Anspruch 4, ferner umfassend den Schritt des Abtrennens des Hydrogelpartikels von dem Fluorkohlenstofföl.

6. Verfahren nach Anspruch 1, ferner umfassend den Schritt des Kultivierens der Zelle innerhalb des Hydrogelpartikels vor dem Detektieren.

7. Verfahren nach Anspruch 6, worin das Kultivieren über einen Zeitraum von 12 bis 24 Stunden hinweg durchgeführt wird.

8. Verfahren zum Screenen einer Bibliothek von Zellen auf eine Zelle, die eine nicht mit Wasser mischbare Verbindung rekombinant produziert, wobei das Verfahren Folgendes umfasst:
(a) Einkapseln jeder Zelle der Bibliothek in ein Hydrogelpartikel;
(b) Kultivieren der Zelle innerhalb des Hydrogelpartikels, wodurch innerhalb des Partikels eine Zellpopulation gebildet wird;
(c) Detektieren der rekombinant hergestellten, nicht mit Wasser mischbaren Verbindung innerhalb jedes Hydrogelpartikels, worin das Detektieren das Kontaktieren des Hydrogelpartikels mit einem fluoreszierenden solvatochromen Farbstoff und das Normalisieren der Menge der nicht mit Wasser mischbaren Verbindung innerhalb des Hydrogelpartikels auf die Menge der Zellbiomasse innerhalb des Hydrogelpartikels umfasst; und
(d) Selektieren einer Zelle, die die rekombinant hergestellte, nicht mit Wasser mischbare Verbindung produziert.

9. Verfahren zum Anreichern einer Population von Zellen mit Zellen, die eine nicht mit Wasser mischbare Verbindung rekombinant produzieren, wobei das Verfahren Folgendes umfasst:
(a) Bereitstellen einer Population von Hydrogelpartikeln, worin die Population Hydrogelpartikel umfasst, die eine Zelle oder eine Klonpopulation von Zellen, die genetisch modifiziert wurden, um eine nicht mit Wasser mischbare Verbindung zu produzieren, einkapseln;
(b) Kultivieren der Zelle oder der Klonpopulation von Zellen innerhalb jedes Hydrogelpartikels;
(c) Detektieren eines Hydrogelpartikels, das eine rekombinant hergestellte, nicht mit Wasser mischbare Verbindung umfasst, worin das Detektieren das Kontaktieren des Hydrogelpartikels mit einem fluoreszierenden solvatochromen Farbstoff und das Normalisieren der Menge der nicht mit Wasser mischbaren Verbindung innerhalb eines Hydrogelpartikels auf die Menge der Zellbiomasse innerhalb des Hydrogelpartikels umfasst;
(d) Gewinnen der Zelle oder der Klonpopulation von Zellen aus dem Hydrogelpartikel von Schritt (b);
(e) erneutes Einkapseln der Zelle oder der Klonpopulation von Zellen aus Schritt (c); und
(f) Wiederholen der Schritte (a) - (d).

10. In Hydrogel eingekapselte Zelle oder Klonzellpopulation, die eine rekombinant hergestellte, nicht mit Wasser mischbare Verbindung und einen fluoreszierenden solvatochromen Farbstoff umfasst, der eine Normalisierung der Menge der nicht mit Wasser mischbaren Verbindung innerhalb des Hydrogels auf die Menge der Zellbiomasse innerhalb des Hydrogels ermöglicht.

11. Hydrogelpartikel, das eine Zelle oder eine Klonzellpopulation, eine rekombinant hergestellte, nicht mit Wasser mischbare Verbindung und einen fluoreszierenden solvatochromen Farbstoff umfasst, der eine Normalisierung der Menge der nicht mit Wasser mischbaren Verbindung innerhalb des Hydrogels auf die Menge der Zellbiomasse innerhalb des Hydrogels ermöglicht.

12. Verfahren nach Anspruch 1, in Hydrogel eingekapselte Zelle oder Klonzellpopulation nach Anspruch 10 oder Hydrogelpartikel nach Anspruch 11, worin der fluoreszierende solvatochrome Farbstoff Nilrot ist.

13. Verfahren nach Anspruch 1, in Hydrogel eingekapselte Zelle oder Klonzellpopulation nach Anspruch 10 oder Hydrogelpartikel nach Anspruch 11, worin die Zelle aus der Gruppe bestehend aus einer Hefezelle, einer Bakterienzelle, einer Säugetierzelle, einer Pilzzelle, einer Insektenzelle und einer Pflanzenzelle ausgewählt ist.

14. Verfahren nach Anspruch 1, in Hydrogel eingekapselte Zelle oder Klonzellpopulation nach Anspruch 10 oder Hydrogelpartikel nach Anspruch 11, worin die Zelle eine Hefezelle ist.

15. Verfahren nach Anspruch 1, in Hydrogel eingekapselte Zelle oder Klonzellpopulation nach Anspruch 10 oder Hydrogelpartikel nach Anspruch 11, worin die rekombinant hergestellte, nicht mit Wasser mischbare Verbindung aus der Gruppe bestehend aus einem Isoprenoid, einem Polyketid und einer Fettsäure ausgewählt ist.

16. Verfahren nach Anspruch 1 oder Hydrogelpartikel nach Anspruch 11, worin das Hydrogelpartikel dazu in der Lage ist, eine nicht mit Wasser mischbare Verbindung zurückzuhalten.

17. Verfahren nach Anspruch 1 oder Hydrogelpartikel nach Anspruch 11, worin das Hydrogenpartikel Agarose umfasst.

18. Verfahren nach Anspruch 1 oder Hydrogelpartikel nach Anspruch 11, worin der Durchmesser des Hydrogelpartikels weniger als etwa 1 mm beträgt.

19. Verfahren nach Anspruch 1 oder Hydrogelpartikel nach Anspruch 11, worin der Durchmesser des Hydrogelpartikels weniger als etwa 500 µm beträgt.

20. Verfahren nach Anspruch 1 oder Hydrogelpartikel nach Anspruch 11, worin der Durchmesser des Hydrogelpartikels weniger als etwa 100 µm beträgt.

21. Verfahren nach Anspruch 1 oder Hydrogelpartikel nach Anspruch 11, worin der Durchmesser des Hydrogelpartikels weniger als etwa 50 µm beträgt.

22. Verfahren nach Anspruch 1 oder Hydrogelpartikel nach Anspruch 11, worin der Durchmesser des Hydrogelpartikels etwa 50, 45, 40, 35, 30 oder 25 µm beträgt.

23. Verfahren nach Anspruch 15, in Hydrogel eingekapselte Zelle oder Klonzellpopulation nach Anspruch 15 oder Hydrogelpartikel nach Anspruch 15, worin das Isoprenoid ein C₅-C₃₀-Isoprenoid ist.

24. Verfahren nach Anspruch 15, in Hydrogel eingekapselte Zelle oder Klonzellpopulation nach Anspruch 15 oder Hydrogelpartikel nach Anspruch 15, worin das Isoprenoid aus der Gruppe bestehend aus Abietadien, Amorphadien, Caren, α-Farnesen, β-Farnesen, Farnesol, Geraniol, Gernaylgeraniol, Isopren, Linalool, Limonen, Myrcen, Nerolidol, Ocimen, Patschulol, β-Pinen, Sabinen, γ-Terpinen, Terpinolen und Valencen ausgewählt ist.

## Revendications

1. Méthode de détection d'un composé non miscible à l'eau produit de cellules recombinantes, la méthode comprenant :
(a) encapsuler la cellule dans une particule d'hydrogel ;
(b) cultiver la cellule dans la particule d'hydrogel moyennant quoi une population de cellules est formée dans la particule ; et
(c) détecter le composé non miscible à l'eau produit de manière recombinante dans la particule d'hydrogel, où ladite détection comprend la mise en contact de la particule d'hydrogel avec un colorant fluorescent solvatochrome et la normalisation de la quantité de composé non miscible à l'eau dans la particule d'hydrogel à la quantité de la biomasse cellulaire dans la particule d'hydrogel.

2. Méthode selon la revendication 1, dans laquelle l'encapsulation comprend la mise en contact de la cellule avec une suspension d'hydrogel aqueuse sous des conditions suffisantes pour former une particule d'hydrogel comprenant la cellule.

3. Méthode selon la revendication 2, dans laquelle lesdites conditions comprennent la mise en contact de la suspension d'hydrogel aqueuse comprenant la cellule avec une huile de fluoro-carbone comprenant un agent fluoro-surfacique.

4. Méthode selon la revendication 3, dans laquelle ladite mise en contact avec une huile de fluoro-carbone comprend le chargement de la suspension d'hydrogel aqueuse comprenant la cellule sur un dispositif micro-fluidique comprenant l'huile de fluoro-carbone, où ladite suspension d'hydrogel vient en contact avec l'huile de fluoro-carbone à une jonction T du dispositif micro-fluidique, où ladite mise en contact avec l'huile de fluoro-carbone se traduit par la formation d'une particule d'hydrogel non aqueuse comprenant la cellule.

5. Méthode selon la revendication 4, comprenant en outre l'étape consistant à séparer la particule d'hydrogel de l'huile de fluoro-carbone.

6. Méthode selon la revendication 1, comprenant en outre l'étape consistant à cultiver la cellule dans la particule d'hydrogel avant ladite détection.

7. Méthode selon la revendication 6, dans laquelle ladite culture dure pendant une période de 12 à 24 heures.

8. Méthode de criblage d'une bibliothèque de cellules pour une cellule produisant d'une manière recombinante un composé non miscible à l'eau, la méthode comprenant :
(a) encapsuler chaque cellule de la bibliothèque dans une particule d'hydrogel ;
(b) cultiver la cellule dans la particule d'hydrogel moyennant quoi une population de cellules est formée dans la particule ;
(c) détecter un composé non miscible à l'eau produit de manière recombinante dans chaque particule d'hydrogel, où ladite détection comprend la mise en contact de la particule d'hydrogel avec un colorant fluorescent solvato-chrome et la normalisation de la quantité de composé non miscible à l'eau dans la particule d'hydrogel à la quantité de la biomasse cellulaire dans la particule d'hydrogel ; et
(d) sélectionner une cellule produisant ledit composé non miscible à l'eau produit de manière recombinante.

9. Méthode d'enrichissement d'une population de cellules pour des cellules produisant d'une manière recombinante un composé non miscible à l'eau, la méthode comprenant :
(a) réaliser une population de particules d'hydrogel, où la population comprend des particules d'hydrogel qui encapsulent une cellule ou une population clonale de cellules génétiquement modifiées pour produire un composé non miscible à l'eau ;
(b) cultiver une cellule ou population de cellules clonales dans chaque particule d'hydrogel ;
(c) détecter une particule d'hydrogel comprenant un composé non miscible à l'eau produit de manière recombinante, où ladite détection comprend la mise en contact de la particule d'hydrogel avec un colorant fluorescent solvato-chrome et la normalisation de la quantité de composé non miscible à l'eau dans la particule d'hydrogel à la quantité de la biomasse de cellule dans la particule d'hydrogel ;
(d) récupérer la cellule ou population clonale de cellules de la particule d'hydrogel de l'étape (b) ;
(e) ré-encapsuler la cellule ou population clonale de cellules de l'étape (c) ; et
(f) répéter les étapes (a) à (d).

10. Cellule ou population de cellules clonales encapsulées dans de l'hydrogel comprenant un composé non miscible à l'eau produit d'une manière recombinante et un colorant solvato-chrome fluorescent qui permet la normalisation de la quantité de composant non miscible à l'eau dans l'hydrogel à la quantité de biomasse cellulaire dans l'hydrogel.

11. Particule d'hydrogel comprenant une cellule ou population de cellules clonales, un composé non miscible à l'eau produit d'une manière recombinante et un colorant fluorescent solvato-chrome qui permet la normalisation de la quantité de composé non miscible à l'eau dans l'hydrogel à la quantité de la biomasse cellulaire dans l'hydrogel.

12. Méthode selon la revendication 1, la cellule ou population de cellules clonales encapsulées dans de l'hydrogel selon la revendication 10, ou la particule d'hydrogel de la revendication 11, où le colorant fluorescent solvato-chrome est le Rouge du Nil.

13. Méthode selon la revendication 1, la cellule ou population de cellules clonales encapsulées dans de l'hydrogel de la revendication 10, ou la particule d'hydrogel de la revendication 11, où la cellule est sélectionnée dans le groupe consistant en une cellule de levure, une cellule bactérienne, une cellule mammalienne, une cellule fongique, une cellule d'insecte et une cellule de plante.

14. Méthode selon la revendication 1, la cellule ou population de cellules clonales encapsulée dans de l'hydrogel de la revendication 10, ou la particule d'hydrogel de la revendication 11, où la cellule est une cellule de levure.

15. Méthode selon la revendication 1, la cellule ou population de cellules clonales encapsulée dans de l'hydrogel de la revendication 10, ou la particule d'hydrogel de la revendication 11, où le composé non miscible à l'eau produit d'une manière recombinante est sélectionné dans le groupe consistant en un isoprénoïde, un polycétide et un acide gras.

16. Méthode selon la revendication 1 ou la particule d'hydrogel de la revendication 11, où la particule d'hydrogel est apte à retenir un composé non miscible à l'eau.

17. Méthode selon la revendication 1 ou la particule d'hydrogel selon la revendication 11, où la particule d'hydrogel comprend de l'agarose.

18. Méthode selon la revendication 1 ou la particule d'hydrogel selon la revendication 11, où la particule d'hydrogel a un diamètre inférieur à environ 1 millimètre.

19. Méthode selon la revendication 1 ou la particule d'hydrogel selon la revendication 11, où la particule d'hydrogel à un diamètre inférieur à environ 500 micromètres.

20. Méthode selon la revendication 1 ou la particule d'hydrogel de la revendication 11, dans laquelle la particule d'hydrogel a un diamètre inférieur à environ 100 micromètres.

21. Méthode selon la revendication 1 ou la particule d'hydrogel de la revendication 11, où la particule d'hydrogel à un diamètre inférieur à 50 micromètres.

22. Méthode selon la revendication 1 ou la particule d'hydrogel selon la revendication 11, où la particule d'hydrogel à un diamètre d'environ 50, 45, 40, 35, 30 ou 25 micromètres.

23. Méthode selon la revendication 15, la cellule ou population de cellules clonales encapsulée dans le l'hydrogel selon la revendication 15, ou la particule d'hydrogel de la revendication 15, où l'isoprénoïde est un isoprénoïde C₅-C₃₀.

24. Méthode selon la revendication 15, la cellule ou population de cellules clonales encapsulée dans de l'hydrogel selon la revendication 15, ou la particule d'hydrogel de la revendication 15, où l'isoprénoïde est sélectionné dans le groupe consistant en abietadiène, amorphadiène, carène, a-farnesène, ß-farnesène, farnesol, géraniol, geranylgeraniol, isoprène, linalool, limonene, myrcène, nérolidod, ocimène, patchoulol, ß-pinène, sabinène, γ-terpinène, terpinolène et valencène.
